# FASCICULE DE BREVET EUROPEEN

(11) **EP 3 119 799 B1**
(45) Date de publication et mention de la délivrance du brevet: **14.03.2018**
(21) Numéro de dépôt: 15718519.0
(22) Date de dépôt: 23.03.2015
(51) Int. Cl.: C07K 5/06, C07K 5/08, C07K 5/10, A61K 38/00

(54) **COMPOSES ANTIMICROBIENS**
ANTIMIKROBIELLE VERBINDUNGEN
ANTIMICROBIAL COMPOUNDS

(30) Priorité: 21.03.2014 FR 1452424
(43) Date de publication de la demande: 25.01.2017
(73) Titulaire: bioMérieux, 69280 Marcy-l'Étoile (FR)
(72) Inventeur: ANDERSON, Rosaleen Joy, Sunderland Tyne and Wear SR6 9LU (GB); BEDERNJAK, Alexandre F., F-18570 Morthomiers (FR); CELLIER, Marie, F-38390 Montalieu Vercieu (FR); NG, Keng Tiong, 52200 Kuala Lumpur (MY); ORENGA, Sylvain, F-01160 Neuville-sur-Ain (FR); PERRY, John D., Newcastle upon Tyne Tyne and Wear NE7 7BH (GB); VÁRADI, Linda, H-3712 Sajovamos (HU)
(74) Mandataire: Murgitroyd & Company
(86) Numéro de dépôt international: PCT/FR2015/050727
(87) Numéro de publication internationale: WO 2015/140481

(56) Documents cités:
- ATHERTON F R ET AL: "synthesis and structure-activity relationships of antibacterial phosphonopeptides", JOURNAL OF MEDICINAL CHEMISTRY, AMERICAN CHEMICAL SOCIETY, US, vol. 29, no. 1, 1 janvier 1986 (1986-01-01), pages 29-40, XP002486143, ISSN: 0022-2623, DOI: 10.1021/JM00151A005
- F. E. HAHN: "Alafosfalin, a new synthetic antibacterial compound", NATURWISSENSCHAFTEN, vol. 68, no. 2, 1 février 1981 (1981-02-01), pages 90-90, XP055152383, ISSN: 0028-1042, DOI: 10.1007/BF01047228
- K S Cheung ET AL: "Chloroalanyl antibiotic peptides: antagonism of their antimicrobial effects by L-alanine and L-alanyl peptides in gram-negative bacteria", Journal of medicinal chemistry, 1 octobre 1986 (1986-10-01), pages 2060-2068, XP055152366, UNITED STATES DOI: 10.1021/jm00160a045 Extrait de l'Internet: URL:http://www.ncbi.nlm.nih.gov/pubmed/309 3682 cité dans la demande

## Description

### Domaine technique

La présente invention concerne de nouveaux composés antimicrobiens, de nouveaux milieux réactionnels les comprenant, ainsi que leurs utilisations. En particulier, les nouveaux composés selon l'invention s'avèrent efficaces à l'encontre des micro-organismes multirésistants aux antimicrobiens et notamment à l'encontre des bactéries multirésistantes aux antibactériens conventionnels.

### Etat de la technique

L'augmentation des micro-organismes présentant des résistances multiples aux agents antimicrobiens classiques (par exemple aux antibactériens) nécessite régulièrement la mise au point de nouveaux agents antimicrobiens dotés de modes d'action d'un nouveau type. Un manque préoccupant de nouveaux antimicrobiens, notamment concernant le traitement des bactéries à gram-négatif multirésistantes, comme celles produisant des carbapénémases, s'avère particulièrement préoccupant. Qui plus est, ces micro-organismes multirésistants sont susceptibles de donner lieu à des diagnostics erronés, générant des résultats faussement positifs lorsque lesdits micro-organismes multirésistants sont présents au sein de milieux réactionnels dits « spécifiques » d'autres micro-organismes, induisant ainsi des problèmes majeurs en termes de spécificité de détection.

Les techniques de diagnostic microbiologique reposent encore largement sur des étapes de culture pour isoler les micro-organismes d'intérêt. Lorsqu'elles sont utilisées pour rechercher des pathogènes potentiellement présents en faible quantité au sein d'une flore microbienne commensale abondante, il est nécessaire d'utiliser des milieux de culture sélectifs. Ces milieux reposent notamment sur l'utilisation d'inhibiteurs spécifiques agissant sur les principales espèces de la flore commensale sans inhiber la croissance des pathogènes recherchés.

À la fin des années 1970, des « peptides mimétiques » ont été développés pour leurs propriétés antibactériennes. Ceux-ci étaient typiquement constitués d'une partie « active » antibactérienne liée par liaison covalente à un ou plusieurs acide(s) aminé(s) destiné(s) à faciliter l'absorption de ces « peptides mimétiques » par l'intermédiaire du système de transport de peptides microbiens. Un clivage hydrolytique intracellulaire (dû à une activité aminopeptidase) libérait alors la partie active antibactérienne à l'intérieur des bactéries d'intérêt pour une interaction avec sa cible. A titre de « peptide mimétique », l'on peut citer l'alafosfaline (acide L-alanyl-L-1-aminoéthylphosphonique ; identifiant CAS : 60668-24-8), développée par la société Roche à la fin des années 1970. L'absorption bactérienne de l'alafosfaline est décrite dans la littérature comme étant accomplie par l'intermédiaire de LL-dipeptide perméases. Une fois à l'intérieur des bactéries cibles, la partie active antibactérienne, en l'espèce la fosfaline (acide L-1-aminoéthylphosphonique), est hydrolysée (plus précisément « libérée » du résidu d'alanine par hydrolyse de la liaison peptidique l'unissant audit résidu d'alanine), puis se lie à l'alanine racémase, empêchant ainsi la synthèse de D-alanine, ingrédient essentiel pour la biosynthèse du peptidoglycane **[1].** L'alafosfaline a été décrite comme possédant un large spectre d'activité antibactérienne **[2],** à la fois *in vitro* et *in vivo,* vis-à-vis de certains micro-organismes aérobies à Gram positif (*Staphylococcus aureus, Enterococcus faecalis,* mais ni les streptocoques du Groupe A ni ceux du Groupe B comme les *S. agalactiae,* ni les *Streptococcus pneumoniae*), des bactéries anaérobies (*Bacteroides* sp. et *Clostridium perfringens,* mais pas *Clostridium difficile*) et de nombreuses espèces de bacilles à Gram négatif, mais pas *Pseudomonas* ou *Acinetobacter.*

L'utilisation de l'alafosfaline a également était décrite dans la demande de brevet WO 02/22785 et dans le brevet EP-B-1325024 en tant qu'agent sélectif pour la mise au point d'un milieu de culture sélectif permettant l'isolement de micro-organismes appartenant à la famille *Salmonella spp.*

Toutefois, dès 1984, Gibson et al. **[3]** mettent en évidence le fait que l'alafosfaline s'avérait inactive contre de nombreuses souches bactériennes en raison de la fréquence élevée des souches résistantes.

D'autres antibactériens synthétiques mis au point dans les années 1980 exploitaient une voie d'administration et/ou un mode d'action voisin(s). Par exemple, Cheung *et al.* **[4]** ont étudié l'activité antibactérienne de plusieurs dipeptides halogénés, parmi lesquels le L-β-chloroalanyl-L-β-chloroalanine. La β-chloroalanine, libérée par hydrolyse, apparaît interagir avec un certain nombre de systèmes enzymatiques, dont l'alanine racémase, et apparaît également présenter un large spectre d'activité antibactérienne **[5].** Dans le cadre d'une tentative d'optimisation des substrats de Cheung et al. **[4],** le résidu β-Cl-L-Alanine (β-Cl-L-Ala) a été incorporé dans divers di- et tripeptides **[5].** Cependant, non seulement aucun des peptides ainsi obtenus n'a permis d'améliorer, de manière significative, l'activité antibactérienne du composé L-β-chloroalanyl-L-β-chloroalanine, mais les auteurs de cette publication ont conclu que les di- et tri-peptides comprenant un résidu L-alanyl étaient dénués d'activité antibactérienne, notamment en ce qui concerne les micro-organismes à Gram négatif. Les auteurs de cette publication en ont déduit que l'absence d'activité antibactérienne observée pour ce qui concerne les résidus di- et tri-peptides comprenant un résidu L-alanyl pouvait être corrélée avec la propriété de la L-alanine de protéger l'alanine racémase des bactéries à Gram négatif contre les résidus β-Cl-L-Ala. En d'autres termes, une compétition semble s'opérer entre le résidu β-Cl-L-Ala et le résidu L-alanyl par rapport à l'alanine racémase.

Toujours dans un souci d'optimisation, Atherton et al. **[6]** ont tenté de combiner, dans une même molécule, l'alafosfaline d'une part, et la β-chloro-L-alanine (β-Cl-L-ala), de l'autre. Toutefois, cette tentative s'est soldée par un échec, dans la mesure où cette molécule hybride ne présentait pas d'activité antibactérienne, malgré les propriétés antibactériennes connues de la β-chloroalanine **[7].** Atherton et al. avaient conclu que ce résultat était lié à l'incapacité des L,L dipeptide perméases à transporter efficacement ce composé au sein des bactéries.

A l'heure où la résistance aux antimicrobiens classiques/conventionnels augmente, un objectif consiste donc à mettre au point de nouveaux composés antimicrobiens, tant sur le plan de la thérapie humaine ou animale, que sur le plan du diagnostic *in vitro* mettant en oeuvre ces nouveaux agents antimicrobiens en tant qu'agents sélectifs. Cette tâche est d'autant plus ardue que, tel qu'indiqué précédemment (cf. publications **[5]** et **[6]**), les modifications et autres combinaisons de composés antimicrobiens existants sont susceptibles d'entraîner une diminution - voire une perte totale - de leur activité antimicrobienne.

### Exposé de l'invention

La Demanderesse a découvert, de manière surprenante, que l'objectif susvisé était atteint par les composés de formule générale (I) : 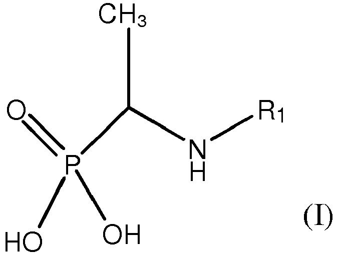 ainsi que leur(s) sel(s), dérivé(s) et analogue(s), de préférence leur(s) sel(s) dans laquelle R₁ représente :
- une partie peptidique P1 consistant en une séquence linéaire d'un à cinq résidus d'acide aminé ; ladite partie peptidique P1 comprenant au moins un résidu de β-chloroalanine, de préférence de β-chloro-L-alanine, ou
- une partie peptidique P2, ladite partie peptidique P2 étant représentée par la formule générale (II) suivante : 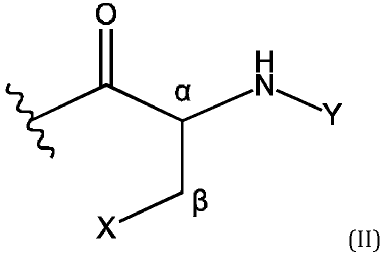
   dans laquelle X représente un atome d'hydrogène ou de chlore, et
   Y représente un atome d'hydrogène ou une séquence linéaire d'un à quatre résidus d'acide aminé, comprenant avantageusement au moins un résidu de β-chloroalanine, de préférence de β-chloro-L-alanine, et/ou au moins un résidu de norvaline, de préférence de L-norvaline, et/ou au moins un résidu de méthionine, de préférence de L-méthionine, et dans laquelle lorsque X représente un atome d'hydrogène, Y est tel que défini précédemment à l'exception d'un atome d'hydrogène ou d'un résidu d'alanine, que ce résidu d'alanine soit en position N-terminale (« en bout de chaîne ») ou qu'il soit lié à un autre résidu d'acide aminé par une liaison peptidique (entre la fonction amine en α dudit résidu d'alanine et la fonction acide carboxylique en α de l'autre résidu d'acide aminé).

Bien évidemment, comme l'indique la terminologie utilisée, et en particulier l'expression «partie peptidique », une séquence linéaire constituée d'une pluralité de résidus d'acide aminé signifie que la pluralité de résidus d'acide aminé sont liés entre eux par des liaisons peptidiques. Comme cela est bien connu en biochimie, une liaison peptidique est une liaison covalente qui s'établit entre la fonction carboxyle porté par le carbone en α d'un acide aminé (« fonction carboxyle en α ») et la fonction amine portée par le carbone en α de l'acide amine suivant dans la chaîne peptidique (« fonction amine en α »). Cette liaison peptidique correspond à une fonction amide.

De préférence, R₁ est représenté par la formule générale (III) : 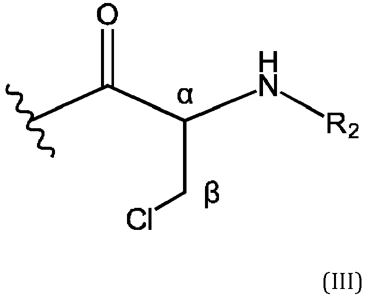 dans laquelle R₂ est un atome d'hydrogène ou une séquence linéaire d'un à quatre résidus d'acide aminé, comprenant avantageusement au moins un résidu de β-chloroalanine, de préférence de β-chloro-L-alanine, et/ou au moins un résidu de norvaline, de préférence de L-norvaline, et/ou au moins un résidu de méthionine, de préférence de L-méthionine.

Par définition, lorsque R₂ est un atome d'hydrogène, R₁ est une partie peptidique comportant un seul résidu d'acide aminé, à savoir un résidu de β-chloroalanine, de préférence de β-chloro-L-alanine.

Tel qu'indiqué précédemment, et selon un mode de réalisation préféré, lorsque R₂ est une séquence linéaire d'un à quatre résidus d'acide aminé, celle-ci comprend au moins un résidu de β-chloroalanine (de préférence β-chloro-L-alanine), et/ou au moins un résidu de norvaline (de préférence de L-norvaline), et/ou au moins un résidu de méthionine (de préférence de L-méthionine). Avantageusement, le susdit résidu de β-chloroalanine (de préférence β-chloro-L-alanine), de norvaline (de préférence de L-norvaline) ou de méthionine (de préférence de L-méthionine) est directement lié par une liaison peptidique au résidu de β-chloro-L-alanine représenté sur la formule générale (III) (ladite liaison peptidique s'établissant entre la fonction acide carboxylique en α dudit résidu de β-chloroalanine, de norvaline ou de méthionine et la fonction amine représentée sur la formule générale (III)).

Selon un mode de réalisation particulier, R₂ consiste en un résidu de norvaline, de préférence de L-norvaline ou en un résidu de méthionine, de préférence de L-méthionine.

Selon un mode de réalisation préféré, R₁ est représenté par la formule générale (IV) : 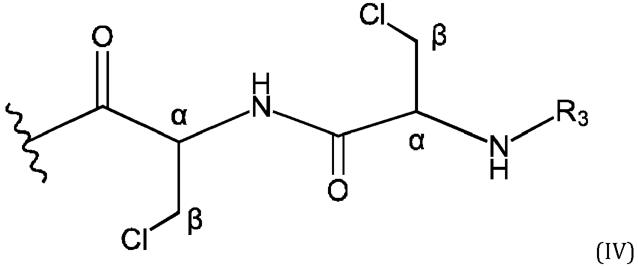 dans laquelle R₃ est un atome d'hydrogène ou une séquence linéaire d'un à trois résidus d'acide aminé, comprenant avantageusement au moins un résidu de β-chloroalanine, de préférence de β-chloro-L-alanine.

Conformément aux conventions d'écritures en vigueur en chimie (notamment en matière de formules topologiques), le symbole dit « en vague » ou « en zigzag » 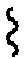 , représenté sur les formules (II), (III) et (IV) supra, schématise la liaison du groupement R₁ au reste du composé de formule générale (I) (via l'établissement d'une liaison peptidique).

Par définition, lorsque R₃ est un atome d'hydrogène, R₁ est une partie peptidique comportant uniquement deux résidus d'acide aminé identiques, à savoir deux résidus de β-chloroalanine, de préférence de β-chloro-L-alanine, reliés l'un à l'autre par une liaison peptidique. Le motif ainsi constitué est dénommé « β-chloroalanyl-β-chloroalanine ».

Selon un mode de réalisation particulier, R₃ comprend un résidu de norvaline, de préférence de L-norvaline, ou un résidu de méthionine, de préférence de L-méthionine. Avantageusement, ledit résidu de norvaline (de préférence de L-norvaline) ou de méthionine (de préférence de L-méthionine) est directement lié par une liaison peptidique (entre la fonction acide carboxylique en α dudit résidu de norvaline ou de méthionine et la fonction amine NH-R₃ du résidu β-chloroalanyl-β-chloroalanine représenté sur la formule générale (IV)) au résidu β-chloroalanyl-β-chloroalanine (de préférence au résidu β-chloro-L-alanyl-β-chloro-L-alanine) représenté sur la formule générale (IV). Comme cela est bien connu de l'homme du métier, la formation de cette liaison peptidique est obtenue par une réaction de condensation entre la fonction amine primaire du motif β-chloroalanyl-β-chloroalanine (de préférence de β-chloro-L-alanyl-β-chloro-L-alanine) et la fonction acide carboxylique de la norvaline (de préférence de L-norvaline) ou de la méthionine (de préférence de L-méthionine), afin de former une liaison peptidique entre ledit motif β-chloroalanyl-β-chloroalanine et ladite norvaline (de préférence de L-norvaline) ou ladite méthionine (de préférence de L-méthionine). Selon un mode de réalisation particulier, R₃ consiste en un résidu de norvaline, de préférence de L-norvaline, ou un résidu de méthionine, de préférence de L-méthionine.

De préférence, ladite partie peptidique R₁ du composé de formule générale (I) consiste en un enchaînement linéaire de deux à trois, de préférence de deux, résidus d'acide aminé ; ces résidus d'acide aminé étant reliés entre eux par des liaisons peptidiques, tel qu'expliqué précédemment.

De manière générale, ledit/lesdits résidu(s) d'acide aminé est/sont sélectionné(s) parmi la glycine, la sarcosine et les formes L et D, de préférence L, des résidus de β-chloroalanine, d'alanine, d'arginine, d'asparagine, d'acide aspartique, de cystéine, d'acide glutamique, d'acide *gamma*-glutamique, de glutamine, d'histidine, d'isoleucine, de leucine, de lysine, de méthionine, de norvaline, de phénylalanine, de proline, d'acide pyroglutamique, de sérine, de thréonine, de tryptophane, de tyrosine et de valine. Avantageusement, ledit/lesdits résidu(s) d'acide aminé est/sont sélectionné(s) parmi les formes L et D, de préférence L, des résidus de β-chloroalanine, d'alanine, de méthionine, de norvaline et de valine.

Selon un autre mode de réalisation, le composé antimicrobien selon l'invention est défini par la formule générale (I) susvisée, dans laquelle R₁ représente ladite partie peptidique P2 et dans laquelle X représente un atome d'hydrogène. Avantageusement, dans cet autre mode de réalisation, Y est un résidu d'acide aminé sélectionné parmi la glycine, la sarcosine, les formes L et D, de préférence L, des résidus de β-chloroalanine, d'arginine, d'asparagine, d'acide aspartique, de cystéine, d'acide glutamique, d'acide gamma-glutamique, de glutamine, d'histidine, d'isoleucine, de leucine, de lysine, de méthionine, de norvaline, de phénylalanine, de proline, d'acide pyroglutamique, de sérine, de thréonine, de tryptophane, de tyrosine et de valine ; avantageusement Y est un résidu d'acide aminé sélectionné parmi les formes L et D, de préférence L, des résidus de méthionine et de norvaline.

Selon un mode de réalisation particulièrement préféré, le composé antimicrobien selon l'invention consiste en :
- l'acide β-chloro-L-alanyl-D/L-1-aminoéthylphosphonique, de préférence l'acide β-chloro-L-alanyl-L-1-aminoéthylphosphonique, et/ou
- l'acide β-chloro-L-alanyl-β-chloro-L-alanyl-D/L-1-aminoéthylphosphonique, de préférence l'acide β-chloro-L-alanyl-β-chloro-L-alanyl-L-1-aminoéthylphosphonique, et/ou
- l'acide L-norvalinyl-β-chloro-L-alanyl-D/L-1-aminoéthylphosphonique, de préférence l'acide L-norvalinyl-β-chloro-L-alanyl-L-1-aminoéthylphosphonique, et/ou
- l'acide L-méthionyl-β-chloro-L-alanyl-D/L-1-aminoéthylphosphonique, de préférence l'acide L-méthionyl-β-chloro-L-alanyl-L-1-aminoéthylphosphonique, et/ou
- l'acide L-norvalinyl- L-alanyl-D/L-1-aminoéthylphosphonique, de préférence l'acide L-norvalinyl-L-alanyl-L-1-aminoéthylphosphonique, et/ou
- l'acide L-méthionyl-L-alanyl-D/L-1-aminoéthylphosphonique, de préférence l'acide L-méthionyl-L-alanyl-L-1-aminoéthylphosphonique ; avantageusement ledit composé antimicrobien consistant en l'acide β-chloro-L-alanyl-D/L-1-aminoéthylphosphonique, de préférence l'acide β-chloro-L-alanyl-L-1-aminoéthylphosphonique, et/ou l'acide β-chloro-L-alanyl-β-chloro-L-alanyl-D/L-1-aminoéthylphosphonique, de préférence l'acide β-chloro-L-alanyl-β-chloro-L-alanyl-L-1-aminoéthylphosphonique.

Avantageusement, le composé antimicrobien selon l'invention consiste en l'acide β-chloro-L-alanyl-D/L-1-aminoéthylphosphonique, de préférence l'acide β-chloro-L-alanyl-L-1-aminoéthylphosphonique, et/ou l'acide β-chloro-L-alanyl-β-chloro-L-alanyl-D/L-1-aminoéthylphosphonique, de préférence l'acide β-chloro-L-alanyl-β-chloro-L-alanyl-L-1-aminoéthylphosphonique, ou un mélange des deux.

Selon un mode de réalisation préféré de l'invention, la fonction amine N-terminale du composé antimicrobien selon l'invention est protégée par un groupement protecteur tel qu'un groupement tertiobutylocarbonyle, 9-fluorenylmethoxycarbonyl ou benzyloxycarbonyl.

La présente invention concerne également un milieu réactionnel comprenant au moins un composé antimicrobien tel que défini précédemment, ledit composé étant présent dans une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L.

Selon un mode de réalisation préféré, ledit milieu réactionnel est un milieu permettant la détection et/ou l'identification et/ou le dénombrement d'au moins un micro-organisme cible, de préférence d'au moins une bactérie cible, dans un échantillon susceptible de le contenir, tel qu'un échantillon d'origine industrielle ou d'origine clinique, dans lequel ledit au moins un composé est au moins un agent sélectif permettant d'inhiber la survie et/ou la croissance de(s) micro-organisme(s) non-cible(s) de façon à privilégier la survie et/ou la croissance dudit au moins un micro-organisme cible.

Avantageusement, ledit milieu réactionnel est un milieu de culture comprenant au moins un nutriment permettant la croissance dudit au moins un micro-organisme cible, dans lequel ledit au moins un agent sélectif permet d'inhiber la croissance de(s) micro-organisme(s) non-cible(s) de façon à privilégier la croissance dudit au moins un micro-organisme cible.

De manière préférentielle, ledit milieu réactionnel comprend, en outre, un substrat enzymatique spécifique d'une activité enzymatique dudit au moins un micro-organisme cible.

De préférence, ledit milieu réactionnel comprend au moins un agent sélectif consistant en :
- l'acide β-chloro-L-alanyl-L-1-aminoéthylphosphonique, de préférence à une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L, ou
- l'acide β-chloro-L-alanyl-β-chloro-L-alanyl-L-1-aminoéthylphosphonique, de préférence à une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L, ou
- un mélange des deux.

Selon un mode de réalisation avantageux, ledit milieu réactionnel permet la détection et/ou l'identification et/ou le dénombrement d'au moins un micro-organisme cible (micro-organisme d'intérêt), ledit au moins un micro-organsime cible étant :
- au moins un micro-organisme cible à Gram négatif appartenant au genre *Salmonella,* par exemple appartenant à l'espèce *Salmonella enterica,* au sérotype *Salmonella Typhimurium* ou au sérotype *Salmonella Enteridis,* au genre *Acinetobacter,* par exemple à l'espèce *Acinetobacter baumannii,* au genre *Burkholderia,* par exemple à l'espèce *Burkholderia cepacia,* au genre *Pseudomonas,* par exemple à l'espèce *Pseudomonas aeruginosa* ; avantageusement ledit au moins un micro-organisme cible à Gram négatif appartenant au genre *Salmonella* ; ou
- au moins un micro-organisme cible à Gram positif, appartenant au genre *Listeria,* par exemple appartenant à l'espèce *Listeria monocytogenes,* ou au genre *Streptococcus,* par exemple *Streptococcus agalactiae* et/ou *Streptococcus pneumoniae* et/ou *Streptococcus pyogenes* ; avantageusement ledit au moins un micro-organisme cible à Gram positif appartenant au genre *Listeria.*

Selon un mode de réalisation avantageux, ledit milieu réactionnel permet la détection et/ou l'identification et/ou le dénombrement d'au moins un micro-organisme cible appartenant au genre *Salmonella.*

Selon un mode de réalisation particulièrement avantageux, ledit milieu réactionnel permet la détection et/ou l'identification et/ou le dénombrement d'au moins un micro-organisme cible appartenant au genre *Salmonella,* ledit milieu comprenant :
- au moins un agent nutritif, tel que des peptones, par exemple d'origine porcine ou bovine, à une concentration comprise entre 0,2 et 30,0 g/L,
- éventuellement un tampon,
- au moins un marqueur chromogène, tel qu'un substrat enzymatique d'estérase et/ou un substrat enzymatique d'alpha-galactosidase, à une concentration comprise entre 0,05 et 15 ,0 g/L,
- de l'agar à une concentration comprise entre 9,0 et 28,0 g/L, et
- au moins un agent sélectif tel que défini dans l'une des revendications 12 à 16, de préférence à une concentration comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L.

Avantageusement, ledit milieu réactionnel comprend un mélange d'au moins un substrat enzymatique d'estérase et/ou d'au moins un substrat enzymatique d'alpha-galactosidase. Il peut en outre comprendre un substrat enzymatique de glucosidase (avantageusement de β-glucosidase) et/ou un substrat enzymatique de galactosidase. Selon un mode de réalisation préféré, ledit milieu réactionnel comprend au moins un substrat enzymatique d'estérase ou au moins un substrat enzymatique d'alpha-galactosidase, en association avec au moins un substrat enzymatique de β-glucosidase. Selon un mode de réalisation particulièrement préféré, ledit milieu réactionnel comprend au moins un substrat enzymatique d'estérase en association avec un substrat enzymatique de β-glucosidase.

Un exemple de milieu réactionnel de ce type est illustré ci-après, au sein du tableau 2 (cf. exemple 3.1 infra).

De préférence, le(s)dit(s) micro-organisme(s) non-cible(s) est/sont compris dans le groupe constitué par :
- des genres de la famille des *Enterobacteriaceae,* tels que le genre *Enterobacter* (par exemple *Enterobacter cloacae*) et/ou le genre *Escherichia* (par exemple *Escherichia Coli*) et/ou le genre *Klebsiella* (par exemple *Klebsiella pneumoniae*) et/ou le genre *Serratia* (par exemple *Serratia marcescens*) et/ou le genre *Yersinia* (par exemple *Yersinia enterocolitica*), et/ou
- le genre *Enterococcus* (par exemple *Enterococcus faecalis* et/ou *Enterococcus faecium*), et/ou
- le genre *Staphylococcus* (par exemple *Staphylococcus epidermidis* et/ou *Staphylococcus aureus*)
de préférence le(s) micro-organisme(s) non-cible(s) est/sont résistant(s) à au moins un antibactérien conventionnel.

Des bactéries de la famille des *Enterobacteriaceae* (entérobactéries), comme par exemple *E*. *coli,* sont des bactéries à Gram négatif fréquemment rencontrées dans les échantillons biologiques, y compris en mélange avec des bactéries cibles à Gram négatif, telles que les bactéries du genre *Salmonella.* Ces entérobactéries croissent facilement sur le milieu de culture et peuvent ainsi masquer lesdites bactéries cibles à Gram négatif (également dénommées bactéries à Gram négatif d'intérêt). Dans la mesure où les composés antimicrobiens selon la présente invention inhibent, de manière sélective et à faible concentration, certaines entérobactéries par rapport aux bactéries cibles à Gram négatif (telles que les bactéries du genre *Salmonella*), lesdits agents antimicrobiens selon l'invention sont particulièrement adaptés à la préparation de milieux réactionnels destinés à la détection et/ou l'identification et/ou le dénombrement de bactéries cibles à Gram négatif telles que les bactéries du genre *Salmonella.* Il résulte de ce qui précède que les composés antimicrobiens selon l'invention sont particulièrement recommandés pour la préparation de milieux réactionnels visant à la détection et/ou l'identification et/ou le dénombrement des bactéries du genre *Salmonella.*

Par ailleurs, les entérocoques et notamment ceux appartenant à l'espèce *Enterococcus faecalis* sont des bactéries à Gram positif fréquemment rencontrées dans les échantillons biologiques, y compris en mélange avec d'autres bactéries à Gram positif. Ces entérocoques croissent facilement sur/dans les milieux de culture et peuvent, en conséquence, masquer des bactéries cibles à Gram positif (bactéries à Gram positif d'intérêt) telles que des bactéries du genre *Listeria* (par exemple appartenant à l'espèce *Listeria monocytogenes*), des bactéries de l'espèce *Staphylococcus aureus,* des bactéries du genre *Streptococcus (*dont *Streptococcus agalactiae, Streptococcus pneumoniae* ou *Streptococcus pyogenes*). Dans la mesure où les composés antimicrobiens selon l'invention inhibent, de manière sélective et à faible concentration, les susdits entérocoques par rapport aux bactéries cibles à Gram positif (telles que des bactéries du genre *Listeria*), lesdits composés antimicrobiens selon l'invention sont particulièrement appropriés à la préparation de milieux réactionnels destinés à la détection et/ou l'identification et/ou le dénombrement de bactéries cibles à Gram positif, telles que celles mentionnées supra.

Un autre objet de l'invention concerne l'utilisation *in vitro* d'un milieu réactionnel pour détecter et/ou identifier et/ou dénombrer au moins un micro-organisme cible, de préférence au moins une bactérie cible, dans un échantillon susceptible de le contenir, tel qu'un échantillon d'origine industrielle ou d'origine clinique.

Un autre objet de l'invention concerne un procédé de détection et/ou d'identification et/ou de dénombrement d'au moins un micro-organisme cible, de préférence d'au moins une bactérie cible, dans un échantillon susceptible de le contenir, tel qu'un échantillon d'origine industrielle ou d'origine clinique, ledit procédé comprenant les étapes suivantes :
a) ensemencer le milieu réactionnel tel que défini précédemment avec ledit échantillon,
b) si nécessaire incuber le tout durant un laps de temps suffisant pour permettre la détection et/ou l'identification et/ou le dénombrement d'au moins un micro-organisme cible,
c) identifier les colonies formées par ledit au moins un micro-organisme cible.

L'invention concerne également le composé antimicrobien, tel que défini ci-dessus, pour son utilisation en tant que médicament à usage humain ou vétérinaire.

Un autre objet de l'invention concerne l'utilisation du composé tel que défini ci-dessus pour son utilisation en tant que médicament dans le traitement des infections microbiennes, de préférence des infections bactériennes, humaines ou vétérinaires.

Selon un mode de réalisation préféré, les composés de formule (I), ainsi que leurs sels, dérivés et analogues (en particulier leurs sels), sont particulièrement efficaces contre les bactéries présentant des résistances multiples (également dénommées multirésistantes) aux antibiotiques conventionnels (notamment aux antibiotiques classiques), à des concentrations variant de 0,002 à 1024,0 mg/L préférentiellement de 0,003 à 32,0 mg/L, préférablement de 0,2 à 8,0 mg/L, avantageusement de 0,2 à 2,0 mg/L.

Ainsi, la présente invention concerne de nouveaux composés antimicrobiens, préférentiellement antibactériens (par exemple antibiotiques bactéricides ou antibiotiques bactériostatiques), permettant de traiter les infections microbiennes, et de préférence bactériennes, difficiles à traiter en utilisant des agents antibactériens conventionnels tels que des antibiotiques classiques, à savoir communément utilisés pour traiter lesdites infections.

Par « résidu d'acide aminé », l'on entend, au sens de la présente invention, le reste d'un acide aminé d'intérêt après que celui-ci a établi au moins une liaison peptidique avec au moins un autre acide aminé. Par exemple, si l'on examine la formule générale (IV) susvisée, il apparaît que la molécule représentée par la formule générale (III) est liée à un résidu de β-chloroalanine, lui-même lié au substituant R₃ ; ledit résidu de β-chloroalanine *per se* ayant donc la formule suivante :
- i) 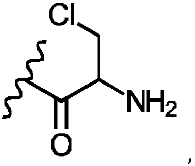 lorsque R₃ est un atome d'hydrogène, ou
- ii) 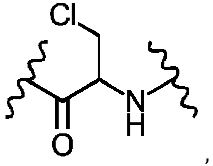 lorsque R₃ est une séquence (chaîne) linéaire d'un à trois résidus d'acide aminé ; auquel cas ledit résidu de β-chloroalanine est lié à un autre résidu d'acide aminé du fait de l'établissement d'une liaison peptidique entre la fonction amine en α du résidu de β-chloroalanine et la fonction acide carboxylique en α de l'autre résidu d'acide aminé.

Bien évidemment, le même raisonnement s'applique par analogie à la définition des résidus d'acide aminé autres qu'un résidu d'alanine.

Par « composé antimicrobien », l'on entend un composé actif contre les micro-organismes, à savoir destiné à lutter contre ces derniers. Selon un mode de réalisation préféré, ce composé antimicrobien est un composé antibactérien, à savoir actif contre les bactéries (et destiné à lutter contre ces dernières). Au sens de la présente invention, le composé antibactérien peut être un antibiotique bactéricide, à savoir qui détruit des bactéries ou un antibiotique bactériostatique, à savoir qui bloque la croissance bactérienne ; en d'autres termes qui empêche la multiplication des bactéries sans nécessairement les tuer. A noter qu'un antibiotique peut être bactériostatique à faible dose et bactéricide à une dose plus élevée.

Selon un autre mode de réalisation, le « composé antimicrobien », peut consister en un composé « antifongique ». Par « composé antifongique », l'on entend tout composé susceptible d'empêcher ou de ralentir la croissance d'une levure ou d'une moisissure. A titre indicatif, l'on peut citer notamment l'amphotéricine B, le fluconazole, l'itraconazole, le voriconazole, la cycloheximide. De préférence, lorsque l'on utilise au moins un agent antifongique, ce dernier est utilisé à des concentrations connues de l'homme du métier pour obtenir l'effet susvisé.

Par « milieu réactionnel », l'on entend un milieu comprenant tous les éléments nécessaires à l'expression d'un métabolisme et/ou la survie et/ou la croissance des micro-organismes. Ce milieu réactionnel peut soit servir uniquement de milieu de révélation, soit servir de milieu de culture et de révélation. Dans le premier cas, la culture des micro-organismes peut être effectuée avant ensemencement, et, dans le second cas, le milieu réactionnel constitue également le milieu de culture. Le milieu réactionnel peut être solide, semi-solide ou liquide. Par « milieu semi-solide », l'on entend, par exemple, un milieu gélifié. L'agar est l'agent gélifiant traditionnel utilisé en microbiologie pour la culture des micro-organismes, mais il est possible d'utiliser d'autres agents gélifiants comme par exemple la gelrite, la gélatine, l'agarose ainsi que d'autres gélifiants naturels ou artificiels. Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media (CRC Press). Le milieu réactionnel selon l'invention peut en outre comprendre d'éventuels additifs comme par exemple des acides aminés, des peptones (dans une concentration appropriée et connue de l'homme du métier pour ne pas risquer d'annuler l'effet inhibiteur des composés antimicrobiens objet de la présente invention), un ou plusieurs facteurs de croissance, des hydrates de carbone, des acides aminés, des nucléotides, des minéraux, des vitamines, un ou plusieurs agents sélectifs, des tampons, un ou plusieurs agents gélifiants, etc. Ledit milieu réactionnel peut également comprendre un colorant. A titre indicatif, l'on peut citer comme colorant le bleu d'Evans, du rouge neutre, du sang de mouton, du sang de cheval, un opacifiant tel que l'oxyde de Titane, de la nitroaniline, du vert malachite, du vert brillant, un ou plusieurs indicateurs métaboliques, un ou plusieurs régulateurs métaboliques, etc. Ce milieu réactionnel peut, par exemple, se présenter sous forme liquide à savoir de gel prêt à l'emploi, c'est-à-dire prêt à l'ensemencement en tube, flacon ou sur boîte de Petri.

L'homme du métier peut également utiliser une bi-boite, permettant de comparer aisément deux milieux, comprenant différents substrats ou différents mélanges sélectifs, sur lesquels un même échantillon biologique aura été déposé. Le milieu réactionnel peut comprendre un ou plusieurs agents sélectifs.

Par « agent sélectif », l'on entend tout composé susceptible d'empêcher ou de ralentir la croissance d'un micro-organisme dit « non-cible », à savoir autre que le ou les micro-organisme(s) cible(s). Sans être limitatif, une concentration comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L, est particulièrement adaptée à la présente invention. D'une manière générale, nous indiquons une concentration en agent sélectif comprise entre 0,01 mg/l et 5,0 g/l.

Par « détection », l'on entend la détection à l'oeil nu ou à l'aide d'un appareil optique de l'existence d'une croissance des micro-organismes cibles (de préférence des bactéries cibles). Lorsque le milieu réactionnel à partir duquel l'on souhaite détecter les micro-organismes cibles comprend un substrat chromogène ou fluorogène, la détection peut être effectuée à l'aide d'un appareil optique pour les substrats fluorogènes, ou à l'oeil nu ou à l'aide d'un appareil optique pour les substrats chromogènes.

Par « dénombrement d'au moins un micro-organisme cible », l'on entend le fait de comptabiliser/quantifier le nombre de micro-organismes cibles, par exemple le nombre de colonies bactériennes lorsque le micro-organisme cible est une bactérie.

Par « échantillon », l'on entend une petite partie ou petite quantité isolée d'une entité pour l'analyse. L'échantillon peut être d'origine industrielle, soit, selon une liste non exhaustive, un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc.). Ces échantillons d'origine alimentaire peuvent aussi provenir de sauces ou de plats élaborés. Un échantillon alimentaire peut enfin être issu d'une alimentation destinée aux animaux, telle que notamment des farines animales ou végétales. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, selle, ...), un prélèvement externe (peau, nez, gorge, périnée, rectum, vagin, ...) ou tissulaire ou des cellules isolées. Ce prélèvement peut être utilisé tel quel ou, préalablement à l'analyse, subir une préparation de type enrichissement, extraction, concentration, purification, selon des méthodes connues de l'homme du métier.

Le contrôle microbiologique correspond à l'analyse d'un échantillon dans le but de détecter et/ou dénombrer des micro-organismes suspectés/susceptibles d'être présents au sein dudit échantillon.

A titre de nutriment permettant la croissance d'au moins un micro-organisme cible sur le milieu réactionnel ou au sein du milieu réactionnel selon l'invention, l'on peut notamment citer les acides aminés, des peptones (dans une concentration appropriée et connue de l'homme du métier pour ne pas risquer d'annuler l'effet inhibiteur des composés antimicrobiens objet de la présente invention), des hydrates de carbone, des nucléotides, des minéraux et des vitamines.

Lorsque, le milieu réactionnel selon l'invention comprend en outre un substrat enzymatique spécifique d'une activité enzymatique d'au moins un micro-organisme cible, l'on utilise de préférence un substrat chromogène et/ou fluorogène.

Par «substrat chromogène et/ou fluorogène », l'on entend un substrat permettant la détection d'une activité enzymatique ou métabolique des micro-organismes cibles/recherchés grâce à un signal détectable directement ou indirectement. Pour une détection directe, ce substrat peut être lié à une partie faisant office de marqueur, fluorescent ou coloré **[8].** Pour une détection indirecte, le milieu réactionnel selon l'invention peut comporter en sus un indicateur de pH, sensible à la variation de pH induite par la consommation du substrat et révélant le métabolisme des micro-organismes cibles. Ledit indicateur de pH peut être un chromophore ou un fluorophore. L'on citera, comme exemple de chromophore, le bromocresol pourpre, le bleu de bromothymol, le rouge neutre, le bleu d'aniline, le bleu de bromocresol.

Les substrats enzymatiques chromogènes utilisables au sens de la présente invention peuvent être de différentes natures.

Premièrement, il convient de mentionner les substrats à base d'indoxyl et ses dérivés qui, après hydrolyse et en présence d'oxygène, produisent un précipité variant du bleu au rose. Ces substrats à base d'indoxyl et ses dérivés sont particulièrement préférés au sens de la présente invention du fait de leur mise en oeuvre relativement aisée et de leur bonne sensibilité dans le cadre de la détection et/ou du dénombrement de bactéries. Leurs applications concernent essentiellement les activités enzymatiques de type osidases, estérases, lipases et phosphatases (la phosphatase étant une activité estérase de l'acide phosphorique). Bien adaptés à une utilisation sur support solide ou semi-solide (filtre, gélose, gel d'électrophorèse, etc.), ils le sont moins à une utilisation en milieu liquide (formation d'un précipité).

Certains dérivés d'indoxyl de type Aldol® représentent des substrats enzymatiques d'intérêt au sens de la présente invention, dans la mesure où l'apparition d'un précipité coloré ne nécessite aucun ajout (oxygène, sels de métaux, etc.). L'utilisation de tels substrats enzymatiques peut donc s'avérer particulièrement avantageuse dans le cadre d'un ensemencement dans la masse des bactéries. Ces dérivés d'indoxyl de type Aldol® sont des dérivés d'indoxyl (1H-indolyl-3-yl) particuliers, à savoir des substrats à base d'indoxyl conjugués sur l'amine cyclique (N-arylés), tels que divulgués dans la demande de brevet PCT publiée sous la référence WO 2010/128120 (au nom de Biosynth® AG [CH]). Ces substrats enzymatiques peuvent être obtenus auprès de la société Biosynth® AG, et en particulier, peuvent être commandés via le site internet de la société Biosynth® AG, à savoir : http://www.biosynth.com.

Deuxièmement, il existe des substrats enzymatiques à base d'hydroxyquinoline, de dihydroxyanthraquinone, de catéchol, de dihydroxyflavone ou d'esculétine et leurs dérivés qui, en présence de sels de fer, produisent un précipité coloré. Là encore, leurs applications concernent essentiellement des activités enzymatiques de type osidases, estérases et phosphatases.

Troisièmement, l'on peut mentionner les substrats enzymatiques à base de nitrophénol et nitroaniline et dérivés, lesquels résultent en la formation d'un composé de couleur jaune. Ils permettent de détecter des activités osidases, estérases et phosphatases dans le cas de substrats à base de nitrophénol et des activités peptidases dans le cas de substrats à base de nitroaniline. Cependant, dans le cas de la détection d'activités peptidases, la nitroaniline libérée est toxique pour les bactéries que l'on souhaite identifier ou caractériser, ce qui peut s'avérer préjudiciable pour des analyses en cours ou ultérieures. D'autre part, ils sont généralement peu adaptés à une utilisation sur support solide et mieux adaptés à une utilisation en milieu liquide. De plus, la couleur (jaune) est peu contrastée dans les milieux biologiques (ce qui influe sur la sensibilité de détection des tests microbiologiques correspondants).

Quatrièmement, il existe des substrats enzymatiques à base de naphthol et naphthylamine et ses dérivés. Dans ce cas, la réaction enzyme-substrat s'effectue en deux temps, le naphthol ou naphthylamine libéré par l'activité enzymatique subit un "azo-coupling" en présence d'un sel de diazonium qui est ajouté au moment de la révélation, conduisant à la formation d'un composé insoluble coloré. Ils permettent de détecter les activités osidases et estérases par l'intermédiaire du naphthol et les activités peptidases par l'intermédiaire de la naphthylamine. La réaction d'"azo-coupling" s'effectue dans un milieu souvent chimiquement agressif, toxique pour les bactéries et rendant l'échantillon inutilisable pour d'autres analyses, de plus les naphtylamines sont cancérigènes.

Parmi les substrats enzymatiques fluorogènes utilisables au sens de la présente invention, l'on peut notamment citer les dérivés de coumarine, de fluorescéine, de rhodamine, de phénoxazine et d'hydroxyflavone, ou encore les substrats enzymatiques ELF®97 ou leurs dérivés.

Selon un mode de réalisation préféré, lorsque le milieu réactionnel selon l'invention dans lequel les composés antimicrobiens selon l'invention sont utilisés en tant qu'agent sélectif afin de cibler spécifiquement une bactérie à Gram négatif (Gram-), telle que celle appartenant au genre *Salmonella,* ledit milieu réactionnel peut également comprendre au moins un système sélectif anti Gram positif (anti Gram+) et/ou antifongique. Les systèmes sélectifs anti Gram+ et/ou antifongique sont bien connus de l'homme du métier. A titre d'exemple, l'on peut citer les tensio-actifs, les glycopeptides, l'amphotéricine, les azolés, le cristal violet (liste non exhaustive) à des concentrations connues de l'homme du métier pour obtenir l'effet escompté, à savoir l'élimination des bactéries à Gram positif. Par analogie, lorsque le milieu réactionnel selon l'invention utilise au moins un nouveau composé antimicrobien en tant qu'agent sélectif d'au moins une bactérie à Gram positif (Gram+), telle qu'une bactérie de l'espèce *Staphylococcus aureus,* l'on peut utiliser au moins un système sélectif anti Gram- et/ou antifongique, bien connu de l'homme du métier. A titre d'exemple, l'on peut citer l'aztréonam, les polymixines, l'acide nalidixique.

Lorsqu'une incubation est nécessaire afin de permettre la croissance des micro-organismes cibles/recherchés, le milieu réactionnel selon l'invention ensemencé avec l'échantillon biologique à tester est incubé. Par « incuber », l'on entend porter et maintenir entre 1 et 48 heures, préférentiellement entre 4 et 24 heures, plus préférentiellement entre 16 et 24 heures, à une température appropriée, généralement comprise entre 20°C et 50°C, préférentiellement entre 30 et 45 °C.

Par « au moins un micro-organisme cible», l'on entend, au sens de la présente invention, au moins un micro-organisme que l'on souhaite détecter et/ou identifier et/ou dénombrer.

A l'inverse, par « micro-organismes non-cibles », l'on entend des micro-organismes que l'on ne souhaite pas détecter et/ou identifier et/ou dénombrer et qui ne présentent pas d'intérêt dans la mesure où l'on ne souhaite pas les détecter et/ou identifier et/ou dénombrer. Il est primordial de s'affranchir de ce ou ces micro-organisme(s) non-cible(s) dans la mesure où ceux-ci sont susceptibles d'induire des résultats faussement positifs et donc d'affecter la spécificité de la détection et/ou de l'identification et/ou du dénombrement dudit au moins un micro-organisme cible.

### Brève description des dessins et des figures

La figure 1 présente la structure des six composés antimicrobiens A-F dont le pouvoir inhibiteur vis-à-vis des principaux groupes de bactéries est évalué dans l'exemple 2 infra (les nouveaux composés antimicrobiens selon l'invention étant illustrés par les structures C et E ; les structures A, B, D et F concernant des composés de l'art antérieur).
La figure 2 présente la structure de quatre autres composés antimicrobiens selon l'invention, à savoir les composés G-J.
La figure 3, quant à elle, illustre les différentes étapes d'un procédé de synthèse pour la préparation de l'acide β-chloro-L-alanyl-L-1-aminoéthylphosphonique **18** (représenté par la structure C sur la figure 1) et de l'acide β-chloro-L-alanyl-β-chloro-L-alanyl-L-1-aminoéthylphosphonique **24** (pour la structure représentée par la structure E sur la figure 1).
La figure 4 présente, de manière schématique, le procédé de synthèse d'un autre composé antimicrobien selon la présente invention, à savoir le composé L-Norvalinyl-L-β-chloroalanyl-D/L-fosfaline **37** (composé G sur la figure 2).
La figure 5 illustre les différentes étapes de synthèse d'un intermédiaire réactionnel permettant l'obtention du composé L-Norvalinyl-L-β-chloroalanyl-D/L-fosfaline **37,** à savoir l'ester O-benzylique de β-chloro-L-alanine **32** (à partir de tBoc-L-sérine **41**).
La figure 6, quant à elle, représente les différentes étapes de synthèse d'un autre intermédiaire réactionnel permettant l'obtention du composé L-Norvalinyl-L-β-chloroalanyl-D/L-fosfaline **37,** à savoir l'ester diéthylique de D/L-fosfaline **35.**

### Description détaillée de l'invention

Les exemples ci-après permettront de mieux appréhender la présente invention. Toutefois, ces exemples ne sont donnés qu'à titre illustratif.

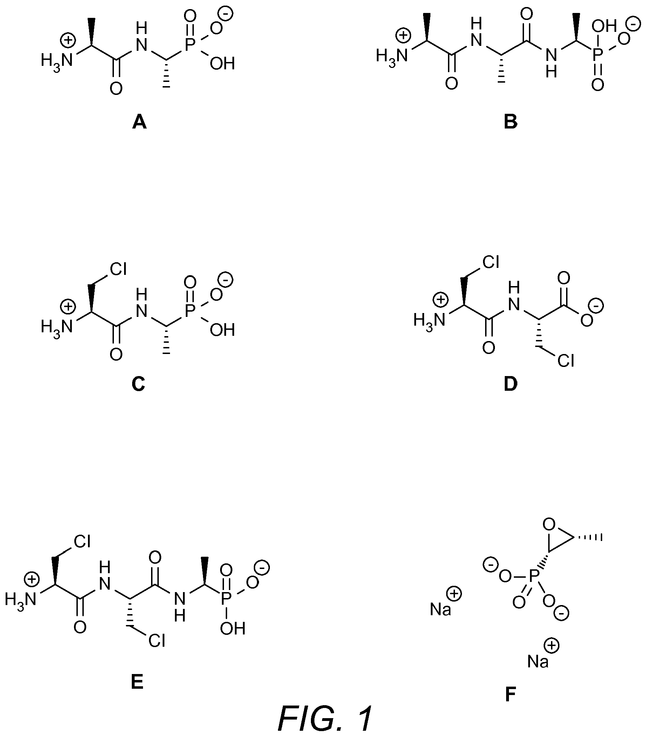

### Exemple 1: Procédé de synthèse des composés antimicrobiens C et E selon l'invention (figure 1)

### 1.1 Généralités

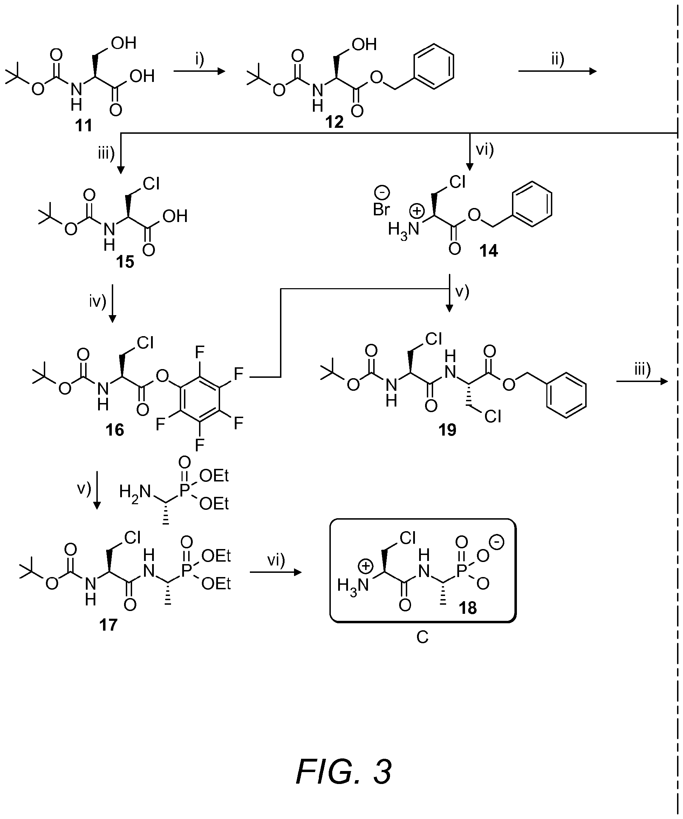

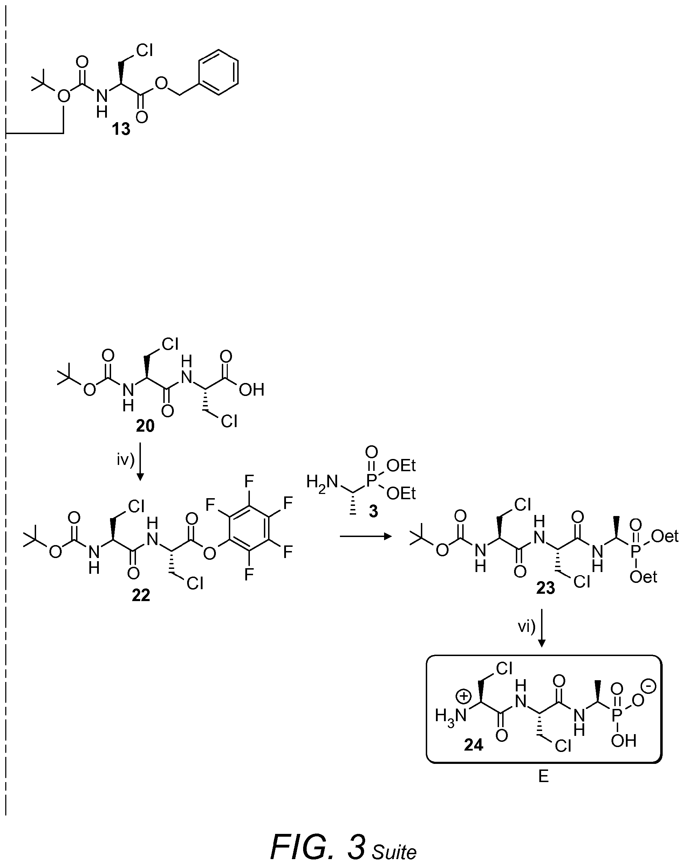

La synthèse des composés de l'art antérieur représentés par les formules A, B, D et F sur la figure 1 est bien connue de l'homme du métier. Le procédé de synthèse des nouveaux composés antimicrobiens selon l'invention est illustré ci-après, pour les composés représentés par les formules C et E sur la figure 1. Ce procédé de synthèse est en outre schématisé en figure 3. Les réactifs et conditions utilisés dans le cadre de ce procédé de synthèse sont les suivants :
i) bromure de benzyle, DBU, benzène ; ii) Cl₃CCN, PPh₃, DCM, N₂, t.a. ; iii) H₂, 10 % Pd/C, MeOH ; iv) PFP, DCC, EtOAc ; v) DCC, DCM, DMF ; vi) HBr, AcOH, puis oxyde de propylène Les spectres RMN ont été obtenus sur un spectromètre Bruker Ultrashield 300 (à 300 MHz pour les spectres ¹H et à 75 MHz pour les spectres ¹³C). Les déplacements chimiques sont indiqués en ppm en aval du tétraméthylsilane, en utilisant le chloroforme résiduel (δ=7,26 en ¹H RMN) ou le pic du milieu du triplet de carbone de CDCl₃ (δ=77,23 en ¹³C RMN) comme étalon interne. Les points de fusion ont été obtenus au moyen d'un microscope à platine chauffante Reichart-Kofler et sont non corrigés. Les spectres infrarouges ont été enregistrés à l'aide d'un instrument PerkinElmer Spectrum BX FT-IR. Les spectres de masse à faible résolution ont été enregistrés sur un analyseur Bruker Esquire 3000plus en utilisant une source d'électrovaporisation en mode ion positif. Les spectres à masse à haute résolution ont été obtenus sur un instrument LTQ Orbitrap XL en mode d'ionisation à nanopulvérisation. Les analyses élémentaires ont été effectuées à l'aide d'un Exeter Analytical CE-440 Elemental Analyzer. Tous les réactifs et solvants disponibles dans le commerce ont été obtenus auprès de Sigma-Aldrich, Alfa-Aesar, Fisher Scientific et Fluka et ont été utilisés sans aucune purification supplémentaire. La chromatographie sur couche mince a été effectuée sur des plaques au gel de silice Merck (60F-254).

### 1.2 Préparation de l'ester benzylique de ^{t}Boc-L-sérine 12

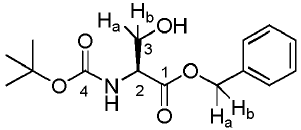

On a dissous de la ^{t}Boc-sérine **11** (16,60 g, 81,0 mmol) dans du benzène (250 mL) et on a ajouté du DBU (18,90 g, 21,6 mL, 124,0 mmol). Au mélange réactionnel agité, on a ajouté goutte à goutte du bromure de benzyle (21,25 g, 15 mL, 124,0 mmol). Après agitation à température ambiante toute une nuit, la réaction a été neutralisée avec une solution 1 M de HCl (150 mL). Le benzène a été éliminé sous pression réduite et le résidu a été repris dans de l'acétate d'éthyle. La solution a été lavée avec de la saumure, la couche organique a été séchée sur Na₂SO₄, puis le solvant a été éliminé sous vide pour donner le produit brut. Après chromatographie sur colonne (50 % d'éther de pétrole, 50 % d'acétate d'éthyle), on a obtenu l'ester benzylique de ^{t}Boc-sérine **12** sous forme de solide blanc (16,90 g, 57,0 mmol, 71 %) ; point de fusion 61-63 °C (point de fusion de la littérature 69-70 °C **[16]**) ; [Trouvé : C, 60,63 ; H, 7,16 ; N, 4,64. C₁₅H₂₁NO₅ demande C, 61,00 ; H, 7,17 ; N, 4,74 %] ; vₘₐₓ/cm⁻¹ 3417, 3356 (NH et OH), 1756 (C=O, ester), 1667 (C=O, carbamate), 1523 (amide II) ; ¹H RMN (300 MHz, CDCl₃) δ_{H} 1,44 (9H, s, C(CH₃)₃), 2,24 (1H, br, OH), 3,91 (1H, br d, *J*=11,1 Hz, CHₐ-3), 3,98 (1H, br d, *J*=11,1 Hz, CH_{b}-3), 4,41 (1H, br, CH-2), 5,19 (1H, d, *J*=12,3 Hz, COOCHₐ), 5,24 (1H, d, *J*=12,3 Hz, COOCH_{b}), 5,44 (1H, br, NH), 7,35-7,37 (5H, m, 5 x CH_{Ar}) ; ¹³C RMN (75 MHz, CDCl₃) δ_{C} 27,9 (CH₃, C(CH₃)₃), 55,6 (CH, C-2), 63,3 (CH₂), 67,1 (CH₂), 80,1 (quat., C(CH₃)₃), 127,8 (2 x CH_{Ar}), 128,1 (CH_{Ar}), 128,3 (2 x CH_{Ar}), 134,9 (quat., C_{Ar}), 153,0 (quat., C=O), 170,3 (quat., C=O) ; SM (ESI) *m*/*z* 318,3 (MNa⁺).

### 1.3 Préparation de l'ester benzylique de ^{t}Boc-β-chloro-L-alanine 13

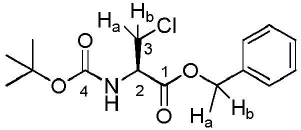

À une solution d'ester benzylique de ^{t}Boc-sérine **12** (15,58 g, 52,8 mmol) dans du dichlorométhane (200 mL), sous azote, on a ajouté du trichloro-acétonitrile (15,16 g, 10,5 mL, 105,0 mmol), suivi de 10 minutes d'agitation à température ambiante. On a dissous de la triphénylphosphine (27,54 g, 105,0 mmol) dans du dichlorométhane (150 mL) sous azote et on a ajouté goutte à goutte cette solution au mélange réactionnel agité. Après agitation à température ambiante toute une nuit, la réaction a été neutralisée avec de la saumure (250 mL) ; après séparation, la couche organique a été extraite avec de la saumure (3x 100 mL). La couche organique a été séchée sur sulfate de sodium anhydre et le solvant a été éliminé sous pression réduite pour donner le produit brut. Une purification par chromatographie sur colonne (70 % d'éther de pétrole, 30 % d'acétate d'éthyle) a donné le produit **13** sous forme de solide blanc (14,94 g, 47,6 mmol, 90 %) ; point de fusion 54-57 °C ; [Trouvé : C, 57,53 ; H, 6,49 ; N, 4,38. C₁₅H₂₀ClNO₄ demande C, 57,42 ; H, 6,42 ; N, 4,46 %] ; vₘₐₓ/cm⁻¹ 3364 (NH), 1725 (C=O, ester), 1680 (C=O, carbamate), 1518 (amide II) ; ¹H RMN (300 MHz, CDCl₃) δ_{H} 1,45 (9H, s, C(CH₃)₃), 3,84 (1H, dd, *J*=3,3 et 11,4 Hz, CHₐ-3), 4,00 (1H, dd, *J*=3,3 et 11,4 Hz, CH_{b}-3), 4,74 (1H, m, CH-2), 5,22 (1H, d, *J*=12,3 Hz, COOCHₐ), 5,27 (1H, d, *J*=12,3 Hz, COOCH_{b}), 5,43 (1H, d, *J*=7,2 Hz, NH), 7,36 (5H, m, 5 x CH_{Ar}) ; ¹³C RMN (75 MHz, CDCl₃) δ_{C} 28,3 (CH₃, C(CH₃)₃), 45,5 (CH₂, C-3), 54,6 (CH, C-2), 67,8 (COOCH₂), 80,5 (quat., C(CH₃)₃), 128,3 (CH), 128,6 (2 x CH), 128,6 (2 x CH), 134,9 (quat., C_{Ar}), 155,0 (quat., C=O), 169,0 (quat., C=O) ; SMHR (ionisation par nanopulvérisation) calculé pour (C₁₅H₂₁NO₄Cl)⁺ 314,1154, trouvé 314,1159.

### 1.4 Préparation de l'ester benzylique de β-chloro-L-alanine HBr 14

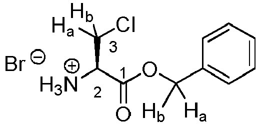

On a dissous de l'ester benzylique de ^{t}Boc-β-chloro-L-alanine **13** (1,40 g, 4,46 mmol) dans une quantité minimale d'acide acétique (5 mL), puis on a ajouté HBr dans AcOH (33 % m/m) (5,53 mL, 30,7 mmol de HBr) et on a agité le mélange réactionnel pendant 10 minutes à température ambiante. La réaction a été neutralisée avec de l'éther diéthylique (200 mL) et la solution a été gardée au congélateur toute une nuit. Pendant le repos, un solide blanc a précipité, lequel a été recueilli par filtration, et lavé avec de l'éther diéthylique froid pour donner le produit **14** (1,10 g, 3,6 mmol, 81 %) ; point de fusion 131-134 °C ; [Trouvé : C, 40,56 ; H, 4,51 ; N, 4,68. C₁₀H₁₃BrClNO₂ demande C, 40,77 ; H, 4,45 ; N, 4,75 %] ; vₘₐₓ/cm⁻¹ 2950, 2875, 2846 (br NH₃⁺), 1750 (C=O, ester), 1489, 1228, 1208 (C-O) ; ¹H RMN (300 MHz, D₂O) δ_{H} 4,17 (1H, dd, *J*=3,3 et 12,6 Hz, CHₐ-3), 4,31 (1H, dd, *J*=3,3 et 12,6 Hz, CH_{b}-3), 4,81 (1H, m, CH-2), 5,31 (1H, d, *J*=12,3 Hz, COOCHₐ), 5,39 (1H, d, *J*=12,3 Hz, COOCH_{b}), 7,55 (5H, m, 5 x CH_{Ar}) ; ¹³C RMN (75 MHz, D₂O) δ_{C} 41,8 (CH₂, C-3), 53,9 (CH, C-2), 69,2 (COOCH₂), 128,7 (2 x CH), 128,9 (2 x CH), 129,1 (CH), 134,5 (quat., C_{Ar}), 167,0 (quat., C=O) ; SMHR (ionisation par nanopulvérisation) calculé pour (C₁₀H₁₃³⁵ClNO₂)⁺ 214,0629, trouvé 214,0630.

### 1.5 Préparation du composé ^{t}Boc-β-chloro-L-alanine 15

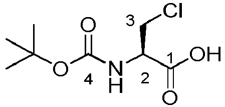

On a dissous de l'ester benzylique de ^{t}Boc-β-chloro-L-alanine **13** (1,57 g, 5,0 mmol) dans du méthanol (50 mL) et on a ajouté du palladium à 10 % sur charbon (0,16 g) dans de l'acétate d'éthyle (20 mL). La réaction a été agitée sous une pression de 1,5 bar de H₂ toute une nuit. Le catalyseur a été éliminé par filtration à travers un bouchon de Célite et lavé avec du méthanol (200 mL). Après élimination du méthanol sous pression réduite, le produit brut a été purifié par chromatographie sur colonne (95 % de dichlorométhane, 5 % de méthanol) pour donner le produit **15** sous forme de solide blanc (0,99 g, 4,4 mmol, 88 %) ; point de fusion 119-123 °C (point de fusion de la littérature 123-125 °C (13)) ; vₘₐₓ/cm⁻¹ 3434 (NH), 2975 (OH), 1752 (C=O), 1734 (C=O), 1677, 1521 (amide II), 1370, 1212 ; ¹H RMN (300 MHz, CDCl₃) δ_{H}1,47 (9H, s, C(CH₃)₃), 3,90 (1H, dd, *J*=2,7 et 11,1 Hz, CHₐ-3), 4,05 (1H, d, *J*=11,1 Hz, CH_{b}-3), 4,78 (1H, m, CH-2), 5,47 (1H, d, *J*=6,3 Hz, NH) ; ¹³C RMN (75 MHz, CDCl₃) δ_{C} 28,3 (CH₃, C(CH₃)₃), 45,2 (CH₂, C-3), 54,3 (CH, C-2), 80,9 (quat., C(CH₃)₃), 155,3 (quat., C=O, C-4), 173,2 (quat., C=O, C-1) ; SMHR (ionisation par nanopulvérisation) calculé pour (C₈H₁₃NO₄³⁵Cl)⁻ 222,0539, trouvé 222,0541.

### 1.6 Préparation de l'ester de pentafluorophénol de ^{t}Boc-β-chloro-L-alanine 16

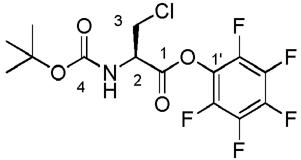

On a dissous de la ^{t}Boc-β-chloro-L-alanine **15** (1,16 g, 5,2 mmol) et du pentafluorophénol (0,95 g, 5,7 mmol) dans de l'acétate d'éthyle (25 mL) et on a refroidi dans un bain de glace. On a ajouté du dicyclohexylcarbodiimide (1,05 g, 5,7 mmol) et la solution a été agitée pendant 3 heures. Le sous-produit d'urée précipité a été éliminé par filtration. Le résidu a été concentré sous pression réduite et tout précipité supplémentaire a été éliminé par filtration. L'acétate d'éthyle restant a été éliminé par évaporation. L'huile ainsi obtenue a été triturée avec de l'éther de pétrole pour donner le produit **16** sous forme de solide blanc (3,35 g, 8,6 mmol, 69 %) qui a été recueilli par filtration; point de fusion 128-131 °C ; [Trouvé : C, 43,53 ; H, 3,49 ; N, 3,63. C₁₄H₁₃³⁵ClF₅NO₄ demande C, 43,15 ; H, 3,36 ; N, 3,59 %] ; vₘₐₓ/cm⁻¹ 3367 (NH), 1780 (C=O), 1681 (C=O), 1518 (amide II) ; ¹H RMN (300 MHz, CDCl₃) δ_{H} 1,48 (9H, s, C(CH₃)₃), 3,94 (1H, dd, *J*=3,6 et 11,4 Hz, CHₐ-3), 3,96 (1H, dd, *J*=3,6 et 11,4 Hz, CH_{b}-3), 5,10 (1H, br t, *J*=3,6 Hz, CH-2), 5,46 (1H, d, *J*=7,5 Hz, NH) ; ¹³C RMN (75 MHz, CDCl₃) δ_{C} 28,25 (CH₃, C(CH₃)₃), 44,78 (CH₂, C-3), 54,55 (CH, C-2), 81,26 (quat., C(CH₃)₃), 118,94 (m, C-F_{Ar}), 154,74 (quat., C-1'), 154,79 (quat., C=O, C-4), 166,77 (quat., C=O, C-1) ; ¹⁹F RMN (282 MHz, CDCl₃) δ_{F}-161,67 (2F, t, *J*=19,8 Hz, CF-3' et 5'), -156,73 (1F, t, *J*=23,1 Hz, CF-4'), - 151,63 (2F, d, *J*=18,9 Hz, CF-2' et 6') ; SM (ESI) *m*/*z* 388,8 (M-H)⁻.

### 1.7 Préparation de l'ester diéthylique de ^{t}Boc-β-chloro-L-alanyl-L-fosfaline 17

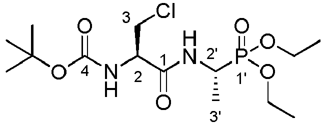

On a dissous de l'ester diéthylique de L-fosfaline (1,10 g, 6,1 mmol) dans du DCM sec (50 mL) et on a refroidi dans un bain de glace. On a ajouté par portions de l'ester de ^{t}Boc-β-chloro-alanylpentafluorophénol **16** (2,37 g, 6,1 mmol) et le mélange réactionnel a été agité à température ambiante jusqu'à achèvement. La réaction a été neutralisée avec de l'eau (150 mL), puis on a extrait avec du dichlorométhane (3 x 100 mL). La couche organique a été séchée sur MgSO₄ anhydre et le solvant a été éliminé sous vide. Le résidu a été purifié par chromatographie sur colonne (50 % d'éther de pétrole, 50 % d'acétate d'éthyle jusqu'à 90 % d'acétate d'éthyle, 10 % de méthanol) pour donner le produit **17** sous forme de solide blanc (1,45 g, 3,8 mmol, 62 %) ; point de fusion 80-83 °C ; [Trouvé : C, 43,46 ; H, 7,27 ; N, 7,21. C₁₄H₂₈ClN₂O₆P demande C, 43,47 ; H, 7,30; N, 7,24 %] ; vₘₐₓ/cm⁻¹ 3303, 3215, 3065, 2978 (NH), 1716 (C=O), 1667 (C=O), 1555, 1518 (amide II) ; ¹H RMN (300 MHz, CDCl₃) δ_{H} 1,25-1,42 (9H, m, 3 x CH₃), 1,46 (9H, s, C(CH₃)₃), 3,73 (1H, dd, *J*=4,2 et 11,1 Hz, CHₐ-3), 4,00 (1H, dd, *J*=4,2 et 11,1 Hz, CH_{b}-3), 4,07-4,18 (4H, m, 2 x OCH₂), 4,39-4,54 (2H, m, CH-2 et 2'), 5,35 (1H, d, *J*=8,4 Hz, NH), 6,77 (1H, d, *J*=8,7 Hz, NH) ; ¹³C RMN (75 MHz, CDCl₃) δ_{C} 15,8 (CH₃, C-3'), 16,3-16,5 (2 x CH₃, m, CH₃CH₂O), 28,2 (3 x CH₃, C(CH₃)₃), 40,3 (CH), 42,4 (CH), 44,9 (CH₂, C-3), 62,6 (CH₂, d, *J*=6,75 Hz, OCH₂CH₃), 62,9 (CH₂, d, *J*=6,6 Hz, OCH₂CH₃), 80,8 (quat., C(CH₃)₃), 155,0 (quat., C=O), 168,3 (quat., C=O) ; ³¹P RMN (121,5 MHz, CDCl₃) δ_{P} 24,6 (m) ; SM (ESI) *m*/*z* 387,3 (MH⁺), 409,3 (MNa⁺), 385,1 (M⁻).

### 1.8 Obtention du composé β-chloro-L-alanyl-L-fosfaline 18 (composé C sur la figure 1)

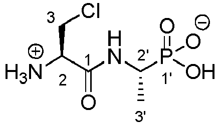

On a agité de l'ester diéthylique de tBoc-β-chloro-L-alanyl-L-fosfaline **17** (1,33 g, 3,4 mmol) dans 33 % m/m de HBr dans de l'acide acétique (17 mL) pendant 24 h. Le mélange réactionnel a été déversé dans de l'éther diéthylique (200 mL) et placé au congélateur (-15 °C) toute une nuit. L'éther diéthylique a été décanté et le précipité résiduel a été repris dans une quantité minimale de méthanol (environ 10 mL). Ensuite, on a ajouté de l'oxyde de propylène en grand excès (environ 250 mL). Le précipité hygroscopique a été filtré et recristallisé à partir d'eau et d'acétone pour donner le produit **18** sous forme de solide blanc (0,99 g, 3,2 mmol, 93 %) ; point de fusion 210-212 °C ; [Trouvé : C, 25,80 ; H, 5,21 ; N, 12,04. C₅H₁₂ClN₂O₄P demande C, 26,04; H, 5,25 ; N, 12,15 %] vₘₐₓ/cm⁻¹ 3258 (br NH), 3100, 2930 (br) (OH), 1654 (C=O), 1565, 1514 (amide II), 1038 ; ¹H RMN (300 MHz, D₂O) δ_{H} 1,30 (3H, dd, *J*=7,2 et 15 Hz, CH₃-3'), 3,97-4,11 (3H, m, CH₂-3 et CH-2'), 4,39 (1H, m, CH-2) ; ¹³C RMN (75 MHz, D₂O) δ_{C} 15,2 (CH₃, C-3'), 42,6 (CH₂, C-3), 44,2 (CH, d, *J*=147,9 Hz, C-2'), 54,2 (CH, C-2), 165,6 (quat., C=O, C-1) ; ³¹P RMN (121,5 MHz, CDCl₃) δ_{P} 18,6 (m) ; SMHR (ionisation par nanopulvérisation) calculé pour (C₅H₁₁N₂O₄P³⁵Cl)⁻ 229,0150, trouvé 229,0154.

### 1.9 Préparation de l'ester benzylique de ^{t}Boc-β-chloro-L-alanyl-β-chloro-L-alanine 19

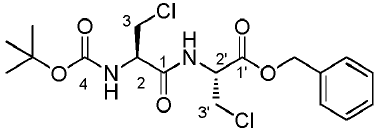

On a dissous du bromohydrate d'ester benzylique de β-chloro-L-alanine **14** (1,13 g, 3,9 mmol) dans du DMF (30 mL) et on a ajouté à une solution d'ester de pentafluorophénol de ^{t}Boc-β-chloro-L-alanine **16** (1,50 g, 3,9 mmol) dans du dichlorométhane (10 mL) à 0 °C. À cette solution, on a ajouté goutte à goutte de la diisopropyléthylamine (0,50 g, 0,66 mL, 3,9 mmol). Le mélange ainsi obtenu a été agité à température ambiante pendant 2 heures, puis chauffé à 35 °C, et l'avancement de la réaction a été surveillé par CCM (80 % d'éther de pétrole, 20 % d'acétate d'éthyle). Une fois la réaction terminée, elle a été neutralisée avec une solution 1M de HCl (50 mL) et la couche organique a été lavée avec de l'eau (100 mL) et de la saumure (100 mL). La couche organique a été séchée sur sulfate de sodium anhydre et le solvant a été évaporé sous vide. Le produit brut a été purifié par chromatographie sur colonne à gradient (de 80 % d'éther de pétrole, 20 % d'acétate d'éthyle ; à 50 % d'éther de pétrole, 50 % d'acétate d'éthyle) pour donner le produit **19** sous forme de solide blanc (1,16 g, 2,8 mmol, 70 %) ; point de fusion 89-91 °C ; [Trouvé : C, 51,83 ; H, 5,78 ; N, 6,78. C₁₈H₂₄³⁵Cl₂N₂O₅ demande C, 51,56; H, 5,77; N, 6,68 %] ; vₘₐₓ/cm⁻¹ 3336 (NH), 3321 (NH), 1739 (C=O), 1655 (m, C=O), 1508 (amide II) ; ¹H RMN (300 MHz, CDCl₃) δ_{H} 1,48 (9H, s, C(CH₃)₃), 3,73 (1H, dd, *J*=4,8 et 11,1 Hz, CH), 3,89-4,06 (3H, m, 3 x CH), 4,55 (1H, br, CH), 4,98 (1H, m, CH), 5,24 (2H, m, COOCH₂), 5,35 (1H, br, NH), 7,25 (1H, br, NH), 7,37 (5H, br s, 5 x CH_{Ar}) ; ¹³C RMN (75 MHz, CDCl₃) δ_{C} 28,24 (CH₃, C(CH₃)₃), 33,90 (CH₂Ph), 44,5 (CH), 44,60 (CH₂), 53,61 (CH), 68,13 (CH₂), 81,37 (quat., C(CH₃)₃), 128,22 (CH_{Ar}), 128,41 (CH_{Ar}), 128,69 (CH_{Ar}), 134,71 (quat.), 168,19 (2 x quat., C=O), 168,90 (quat., C=O) ; SMHR (ionisation par nanopulvérisation) calculé pour (C₁₈H₂₅N₂O₅³⁵Cl)⁺419,1135, trouvé 419,1139.

### 1.10 Préparation du composé ^{t}Boc-β-chloro-L-alanyl-β-chloro-L-alanine 20

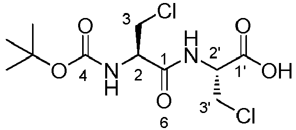

On a dissous de l'ester benzylique de ^{t}Boc-β-chloro-L-alanine-β-chloro-L-alanine **19** (1,00 g, 2,4 mmol) dans du méthanol (50 mL) et on a ajouté du palladium à 10 % sur charbon (0,10 g) dans de l'acétate d'éthyle (20 mL). Le mélange réactionnel a été agité sous une atmosphère de H₂ (1,8 bar) pendant 24 h, puis a été filtré à travers un bouchon de Célite et lavé avec du méthanol (200 mL). Les solvants ont été éliminés sous vide et, après purification par chromatographie sur colonne (95 % de DCM, 5 % de méthanol), on a obtenu lors d'une trituration avec de l'éther de pétrole le produit **20** sous forme de solide blanc (0,52 g, 1,6 mmol, 66 %) ; point de fusion 72-74 °C ; vₘₐₓ/cm⁻¹ 3320 (NH), 2978 (NH), 1724 (C=O), 1665 (C=O), 1530 (amide II) ; ¹H RMN (300 MHz, DMSO-*d₆*) δ_{H} 1,39 (9H, s, C(CH₃)₃), 3,67 (1H, dd, *J*=8,4 et 11,1 Hz, CH), 3,79-3,95 (3H, m, CH₂ et CH), 4,36 (1H, br, CH), 4,61-4,67 (1H, m, CH), 7,16 (1H, d, *J*=8,1 Hz, NH), 8,38 (1H, d, *J*=7,5 Hz, NH) ; SMHR (ionisation par nanopulvérisation) calculé pour (C₁₁H₁₇³⁵Cl₂N₂O₅)⁻ 327,0520, trouvé 327,0517 ;

### 1.11 Préparation de l'ester de pentafluorophénol de ^{t}Boc-β-chloro-L-alanyl-β-chloro-L-alanine 22

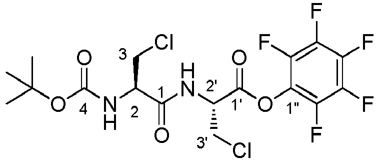

On a dissous de la ^{t}Boc-β-chloro-L-alanyl-β-chloro-L-alanine **20** (0,48 g, 1,46 mmol) dans de l'acétate d'éthyle (50 mL) et on a refroidi dans un bain de glace, suivi par l'addition de pentafluorophénol (0,29 g, 1,56 mmol) et de dicyclohexylcarbodiimide (0,33 g, 1,61 mmol). La solution a été agitée à 0 °C pendant 2 heures et l'urée précipitée a été éliminée par filtration. Le résidu a été concentré sous vide et toute nouvelle précipitation a également été éliminée par filtrage. L'acétate d'éthyle a été éliminé sous vide et l'huile ainsi obtenue a été triturée avec de l'éther de pétrole pour donner le produit **22** sous forme de solide blanc (0,65 g, 1,3 mmol, 89 %), qui a été recueilli par filtration et emmené à l'étape suivante sans autre purification ; vₘₐₓ/cm⁻¹ 3334 (NH), 3006, 2970, 2939 (NH), 1787 (C=O), 1688 (C=O), 1664 (C=O), 1514 (amide II) ; ¹H RMN (300 MHz, CDCl₃) δ_{H} 1,46 (9H, s, C(CH₃)₃), 3,76 (1H, dd, *J*=4,7 et 11,3 Hz, CHₐ), 3,98 (1H, dd, *J*=3,5 et 11,7 Hz, CH_{c}), 4,06 (1H, dd, *J*=4,4 et 11,3 Hz, CH_{b}), 4,16 (1H, dd, *J*=3,2 et 11,7 Hz, CH_{d}), 4,53 (1H, br, CH), 5,32-5,37 (2H, m, CH et NH), 7,31 (1H, d, *J*=7,1 Hz, NH) ;

### 1.12 Préparation de l'ester diéthylique de ^{t}Boc-β-chloro-L-alanyl-β-chloro-L-alanyl-L-fosfaline 23

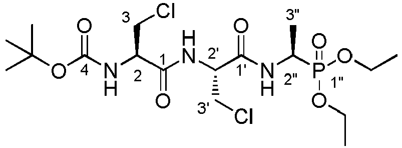

On a dissous de l'ester de pentafluorophénol de ^{t}Boc-β-chloro-L-alanyl-β-chloro-L-alanine **22** (0,95 g, 1,9 mmol) et du diéthyl 1-aminoéthylphosphonate **3** (0,32 g, 1,9 mmol) dans du dichlorométhane (25 mL) à 0 °C, puis on a agité à température ambiante jusqu'à achèvement de la réaction, suivi par une CCM. Après extraction avec de l'eau (100 mL), la couche organique a été séchée sur MgSO₄ anhydre et les composants volatils ont été éliminés sous pression réduite. Le solide brut a été purifié par chromatographie sur colonne (97 % de DCM, 3 % de MeOH) pour donner le produit **23** sous forme de solide blanc (0,62 g, 1,3 mmol, 66 %) ; point de fusion 154,6-155,9 °C ; vₘₐₓ/cm⁻¹ 3291, 3265, 2962, 2848 (NH), 1680 (C=O), 1639 (C=O), 1523 (amide II) ; ¹H RMN (300 MHz, CDCl₃) δ_{H} 1,35-1,41 (9H, m, 3 x CH₃), 1,48 (9H, s, C(CH₃)₃), 3,73-3,80 (2H, m, CHₐ-3 et CHₐ-3'), 4,01-4,2 (6H, m, CH_{b}-3, CH_{b}-3' et 2 x OCH₂), 4,43-4,54 (2H, m, CH-2" et CH-2), 4,79-4,84 (1H, m, CH-2'), 5,38 (1H, d, *J*=6,9 Hz, NH), 7,05 (1H, d, *J*=9,0 Hz, NH), 7,19 (1H, d, *J*=7,8 Hz, NH) ; ¹³C RMN (75 MHz, CDCl₃) δ_{C} 15,7 (CH₃, C-3'), 16,6 (CH₃, OCH₂CH₃), 16,7 (CH₃, OCH₂CH₃), 28,4 (3 x CH₃, C(CH₃)₃), 40,7 (CH), 44,5 (CH₂), 44,6 (CH₂), 49,4 (CH), 54,1 (CH), 62,8 (CH₂, d, *J*=15,5 Hz, OCH₂CH₃), 63,1 (CH₂, d, *J*=15,7 Hz, OCH₂CH₃), 81,6 (quat., C(CH₃)₃), 156,9 (quat., C=O), 168,9 (quat., C=O), 177,3 (quat., C=O) ; SM *m*/*z* 514,3, 515,2, 516,2 (MNa⁺) ; SMHR (ionisation par nanopulvérisation) calculé pour (C₁₇H₃₃N₂O₇P³⁵Cl₂)⁺ 492,1428, trouvé 492,1422.

### 1.13 Obtention de la β-chloro-L-alanyl-β-chloro-L-alanyl-L-fosfaline 24 (composé E sur la figure 1)

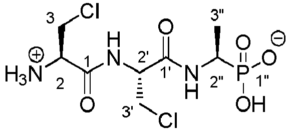

On a dissous de l'éther diéthylique de ^{t}Boc-β-chloro-L-alanyl-β-chloro-L-alanyl-L-fosfaline **23** (0,36 g, 0,7 mmol) dans de l'acide acétique (5 mL) et on a ajouté HBr dans de AcOH (33 % m/m (4 mL). La solution a été agitée à température ambiante toute une nuit, puis la réaction a été neutralisée avec de l'éther diéthylique (200 mL). Après repos au congélateur (-15 °C) pendant 4 h, l'huile brune ainsi obtenue a été séparée par décantation de l'éther diéthylique. Le produit brut a été ensuite lavé avec de l'éther diéthylique froid (5x 50 mL). Le résidu a été repris dans du méthanol (3 mL) et, à l'addition d'oxyde de propylène (150 mL), un précipité blanc s'est formé. La couche liquide a été décantée et le résidu a été trituré avec de l'éther diéthylique froid (5x 30 mL) pour donner le produit **24** sous forme de solide blanc (0,18 g, 0,4 mmol, 59 %) ; point de fusion 157-159 °C ; vₘₐₓ/cm⁻¹ 3285, 3258 (br NH), 1646 (br) (C=O), 1539 (large amide II), 1152, 1044; ¹H RMN (300 MHz, D₂O) δ_{H} 1,37 (3H, dd, *J*=7,2 et 15,3 Hz, CH₃-3"), 3,98-4,2 (5H, m, CH₂-3 et CH₂-3' et CH), 4,62 (1H, t, *J*=4,8 Hz, CH), 4,87-4,90 (1H, m, CH) ; ¹³C RMN (75 MHz, D₂O) δ_{C} 15,4 (CH₃, C-3"), 42,4 (CH₂), 43,4 (CH₂), 45,1 (CH, C-2"), 53,8 (CH), 55,1 (CH), 166,8 (quat., C=O), 168,9 (quat., C=O) ; SMHR (ionisation par nanopulvérisation) calculé pour (C₈H₁₅N₃O₅P³⁵Cl₂)⁻ 336,0102, trouvé 336,0096.

### Exemple 2 : Evaluation de l'activité antibactérienne des composés antimicrobiens C et E synthétisés selon l'Exemple 1

### 2.1 Introduction

Cet exemple 2 présente les résultats d'une étude visant à :
- évaluer l'activité antibactérienne des composés selon l'invention, tels que synthétisés à l'Exemple 1, à savoir les composés C et E tels que représentés sur la figure 1, et
- mettre en perspective cette activité antibactérienne avec celle obtenue par trois peptides mimétiques de l'art antérieur, à savoir les composés A, B et D de la figure 1 et la fosfomycine (composé F).

L'activité antibactérienne des cinq composés A-E a été évaluée avec une large collection de 297 bactéries qui incluaient une prédominance de souches multirésistantes y compris des entérobactéries productrices de carbapénémase (*n* = 128), des staphylocoques dorés résistants à la méticilline (*n* = 37) et les entérocoques résistants aux glycopeptides (*n* = 43). La fosfomycine, un antibiotique présent naturellement contenant également un groupe acide phosphonique (F) a été inclus à des fins de comparaison.

Pour rappel, les composés A-F sont les suivants :
A : acide L-alanyl-L-1-aminoéthylphosphonique (alafosfaline) ;
B : acide L-alanyl-L-alanyl-L-1-aminoéthylphosphonique (di-alanyl fosfaline) ;
C : acide β-chloro-L-alanyl-L-1-aminoéthylphosphonique (β-Cl-alafosfaline) ;
D : β-chloro-L-alanyl-β-chloro-L-alanine (β-Cl-Ala-β-Cl-Ala) ;
E : acide β-chloro-L-alanyl-β-chloro-L-alanyl-L-1-aminoéthylphosphonique (β-Cl-Ala-β-Cl-alafosfaline) ;
F : phosphonate de [(2R,3S)-3-méthyloxiran-2-yl] disodique (fosfomycine).

### 2.2 Matériels et méthodes

**Agents antibactériens et milieux.** La fosfomycine, l'alafosfaline, le glucose-6-phosphate et tous les ingrédients du milieu gélosé sans antagoniste ont été achetés auprès de Sigma Chemical Company, Poole, Royaume-Uni. Le milieu gélosé IsoSensitest a été acheté auprès d'Oxoid, Basingstoke, Royaume-Uni.
**Isolats bactériens.** Les entérobactéries (*n* = 197) ont été obtenues auprès de diverses sources internationales et toutes possédaient des β-lactamases qui ont été définies à un niveau moléculaire par des laboratoires de référence et/ou des experts reconnus dans le domaine. Celles-ci incluaient *Citrobacter freundii* (*n* = 5), d'autres espèces de *Citrobacter* (*n* = 4), *Enterobacter aerogenes* (*n* = 1), *Enterobacter cloacae* (*n* = 27), *Escherichia coli* (*n* = 53), *Klebsiella oxytoca* (*n* = 5), *Klebsiella pneumoniae* (*n* = 87), *Kluyvera* spp (*n*= 1), *Proteus mirabilis* (*n* = 8), *Providencia rettgeri* (*n* = 2), *Salmonella* spp (*n* = 3), et *Serratia marcescens* (*n* = 1). Parmi ces 197 isolats, il y avait 128 (65 %) producteurs de carbapénémase, y compris ; 87 avec NDM-1, 9 avec IMP, 11 avec KPC, 14 avec OXA-48 et 7 avec VIM. La plupart des producteurs de carbapénémase ont produit conjointement des *β*-lactamases à spectre étendu (BLSE) ou des céphalosporinases (AmpC β-lactamases), mais celles-ci ne sont pas documentées à des fins de clarté. Parmi les isolats restants, 47 possédaient des BLSE (20 avec CTX-M, 19 avec un type SHV, et 8 avec un type TEM) et 22 possédaient des AmpC (3 avec ACC-1, 6 avec un type CMY, 6 avec DHA-1, 3 avec un type FOX et 4 avec un type LAT).

Une collection de 50 isolats de staphylocoques dorés incluait 36 souches de *S. aureus* résistants à la méticilline (SARM) fréquemment rencontrées en Europe, y compris des souches isolées en Belgique, en Finlande, en France, en Allemagne, et au Royaume-Uni. Une autre souche de SARM, NCTC 11939, a été incluse en tant que témoin, ainsi qu'un témoin sensible à la méticilline (NCTC 6571). Douze autres isolats de staphylocoques dorés sensibles à la méticilline (SASM) récemment récupérés de cultures sanguines ont également été inclus. Enfin, 50 isolats d'entérocoques incluaient deux souches témoins (*Enterococcus faecalis* NCTC 755 et *Enterococcus faecium* NCTC 7171) et 48 isolats provenant d'échantillons cliniques obtenus auprès d'au moins trois hôpitaux différents. Les isolats cliniques incluaient *E. faecalis* (n = 10), *E. faecium* (n = 33), *Enterococcus casseliflavus* (n = 3), *Enterococcus gallinarum* (n = 2). Parmi les 50 isolats, 43 étaient résistants à la vancomycine, comme démontré par la valeur de CMI et la confirmation des gènes de résistance par PCR.

**Détermination des concentrations minimales inhibitrices (CMI).** Toutes les CMI ont été déterminées en utilisant un procédé de dilution en gélose **[16].** Celui-ci a nécessité l'utilisation d'un milieu sans antagoniste défini (sans peptone), préparé de la manière décrite précédemment avec l'inclusion de 2 % de sang de cheval lysé à la saponine, 25,0 µg/mL de NAD et 25,0 µg/mL d'hémine **[17].** Les composés testés ont été dissous dans de l'eau désionisée stérile et incorporés dans le milieu gélosé à une plage de concentration de 0,0031 à 8,0 µg/mL (0,016 à 32 µg/mL pour les bactéries à gram-positif). Tous les isolats ont été préparés à une densité équivalente à 0,5 unité McFarland dans de l'eau désionisée stérile à l'aide d'un densitomètre (environ 1,5 x 10⁸ UFC/mL), puis dilués à 1 pour 15. Une aliquote de 1 µL de chaque suspension diluée a été ensuite placée sur des plaques avec un inoculateur multipoint pour donner l'inoculation finale recommandée de 10 000 UFC/point (ou « spot » en langue anglaise) **[16].** Les CMI de la fosfomycine ont été déterminées par le même procédé, si ce n'est que l'on a utilisé le milieu IsoSensitest (Oxoid, Basingstoke, Royaume-Uni) complété avec 25,0 µg/mL de glucose-6-phosphate (Sigma, Poole, Royaume-Uni) et une plage étendue de concentrations en fosfomycine. Toutes les boîtes (y compris les témoins sans antimicrobien) ont été incubées 22 heures à 37 °C. Tous les essais ont été réalisés sur au moins deux réplicats indépendants afin d'examiner la reproductibilité.

### 2.3 Résultats

Les concentrations minimales inhibitrices (CMI) 50 et 90 des cinq composés A-E ont été calculées et sont présentées dans le Tableau 1 infra. Ces valeurs correspondent respectivement à la plus petite concentration d'antibiotique suffisante pour inhiber *in vitro* 50 et 90 % de la croissance d'une souche de bactéries, respectivement CMI₅₀ et CMI₉₀.

Plus précisément, le Tableau 1 infra indique les CMI des six antimicrobiens vis-à-vis des principaux groupes de bactéries testés. L'alafosfaline a présenté une bonne activité vis-à-vis de la plupart des isolats d'entérobactéries, bien que différentes espèces aient montré différents degrés de sensibilité. Une forte activité a été observée vis-à-vis de 53 isolats d'*E*. *coli,* dont 35 isolats (66 %) étaient des producteurs de carbapénémase. La CMI₉₀ pour *E*. *coli* était de 0,25 µg/mL et la croissance de tous les isolats a été inhibée par 2 µg/mL. On a trouvé que l'activité de l'alafosfaline était environ quatre fois plus élevée que celle de la fosfomycine.

Bien que l'activité antibactérienne de l'alafosfaline vis-à-vis de *E*. *coli* soit satisfaisante, tel qu'indiqué précédemment, l'activité de la β-Cl-alafosfaline vis-à-vis d'*E*. *coli* s'est avérée être au moins deux fois celle de l'alafosfaline et au moins huit fois plus élevée que celle de la fosfomycine. La CMI₉₀ était de 0,125 µg/mL et tous les isolats ont vu leur croissance inhibée par 0,5 µg/mL.

*K. pneumoniae* a été moins sensible à tous les composés testés lorsque l'on compare à *E*. *coli,* bien que de nombreux isolats aient présenté des CMI relativement basses. Par exemple, 87 % d'isolats de *K. pneumoniae* ont été inhibés par 8 µg/mL d'alafosfaline et 93 % ont été inhibés par 8 µg/mL de β-Cl-alafosfaline.

Tous les isolats d'*E*. *cloacae* (*n* = 27) ont été inhibés par 4 µg/mL d'alafosfaline, qui a été typiquement 32 fois plus active que la fosfomycine vis-à-vis de cette espèce. Comme précédemment, la β-Cl-alafosfaline s'est révélée être le composé le plus actif avec tous les isolats inhibés par 1 µg/mL. Les autres espèces d'entérobactéries sont exclues du Tableau 1, car il y a eu moins de 10 isolats testés.

Pour les taxa *Citrobacter* (*n* = 9), *E. aerogenes* (*n* = 1), *Kluyvera* sp. (*n* = 1), et *S. marcescens* (*n* = 1), tous les isolats ont été sensibles à ≤ 4 µg/mL d'alafosfaline et ≤ 2 µg/mL de β-Cl-alafosfaline. Un des cinq isolats de *K. oxytoca* a demandé une CMI >8 µg/mL d'alafosfaline, mais tous ont été inhibés par ≤ 2 µg/mL de β-Cl-alafosfaline, démontrant une fois encore l'activité antibactérienne très satisfaisante de cette dernière.

Huit isolats de *P. mirabilis* et deux isolats de *P. rettgeri* ont demandé des CMI ≥ 8 µg/mL pour tous les agents testés (y compris la fosfomycine).

Trois isolats de *Salmonella* ont montré des CMI ≥ 8,0 µg/mL pour l'alafosfaline mais uniquement de 2,0 à 4,0 µg/mL pour la β-Cl-alafosfaline.

La β-alafosfaline et la β-Cl-Ala-β-Cl-Ala ont eu la plus forte activité vis-à-vis des staphylocoques dorés, mais il y a eu une légère différence globale entre les cinq antimicrobiens peptidiques. 90% des isolats de SARM, provenant de diverses sources géographiques, ont été inhibés par 8,0 µg/mL d'alafosfaline et tous les isolats ont été inhibés par 2,0 µg/mL de β-Cl-alafosfaline, démontrant, une fois de plus, une activité antibactérienne supérieure à celle de l'alafosfaline.

Vis-à-vis des entérocoques, l'observation la plus visible a été l'activité élevée de la di-alanyl fosfaline, pour laquelle les CMI ont été (en moyenne) 16 fois plus basses que celles de l'alafosfaline et, dans certains cas, 256 fois plus basses. Parmi les 34 isolats d'*E. faecium* (y compris 31 isolats résistants à la vancomycine), tous ont été inhibés par 32 µg/mL d'alafosfaline ou 4 µg/mL de di-alanyl fosfaline. En tout état de cause, parmi les entérocoques, l'activité antibactérienne de la β-Cl-Ala-β-Cl-alafosfaline (composé selon l'invention) s'est avérée supérieure à celle de l'alafosfaline.

### 2.4 Conclusion

Comme nous l'avons montré dans cette étude, les composés antimicrobiens selon la présente invention, et notamment la β-Cl-alafosfaline, présentent une activité antibactérienne *in vitro* intéressante (très fréquemment supérieure à celle de l'alafosfaline) vis-à-vis de la plupart des bactéries et notamment des bactéries multirésistantes. Ainsi, à titre d'exemple, CMI₅₀ et CMI₉₀ de l'alafosfaline pour les EPC ont été respectivement de 1 µg/mL et 4 µg/mL, alors que le phosphonopeptide selon l'invention, la β-Cl-alafosfaline, a présenté une des valeurs de 0,5 µg/mL et 2 µg/mL. L'alafosfaline n'a été que modérément active vis-à-vis du SARM présentant un CMI₉₀ de 8 µg/mL, alors que la β-Cl-alafosfaline a été plus active avec un CMI₉₀ de 2 µg/mL.

Par rapport à l'alafosfaline, la β-Cl-Ala-β-Cl-alafosfaline permet un gain d'inhibition ciblé sur certaines espèces. Ceci peut être très utile pour inhiber une espèce sans altérer le reste de la flore microbienne, par exemple inhibition d'*E*. *coli* sans inhiber les autres entérobactéries ou d'*E. faecalis* sans inhiber *E. faecium.* Cela devient particulièrement avantageux pour isoler spécifiquement une espèce en inhibant les autres bactéries, par exemple pour rechercher *S. aureus.*

**Tableau 1 : Concentrations inhibitrices minimales de divers agents antimicrobiens vis-à-vis de groupes de bactéries, y compris des isolats dotés de mécanismes de résistance définis.**

| **Organisme (nombre testé) et agent antimicrobien** | **Concentration (µg/mL)** | | | |
|---|---|---|---|---|
| | **CMI modale (CMI la plus fréquente)** | **CMI₅₀** | **CMI₉₀** | **Plage** |
| ***Enterobacteriaceae* (197)** | | | | |
| Alafosfaline | 2 | 2 | > 8 | ≤0,031 - >8 |
| Di-alanyl fosfaline | > 8 | 8 | > 8 | ≤0,031 - >8 |
| β-Cl-Alafosfaline | 1 | 0,5 | 8 | ≤0,031 - >8 |
| β-Cl-Ala-β-Cl-Ala | > 8 | > 8 | > 8 | 2 - >8 |
| β-Cl-Ala-β-Cl-Alafosfaline | 4 | 2 | > 8 | ≤0,031 - >8 |
| Fosfomycine | 4 | 4 | > 32 | 0,125 - >32 |

| ***E. coli* (53)** | | | | |
|---|---|---|---|---|
| Alafosfaline | 0,063 | 0,125 | 0,25 | ≤0,031 - 2 |
| Di-alanyl fosfaline | 0,25 | 0,5 | 2 | ≤0,031 - >8 |
| β-Cl-Alafosfaline | 0,063 | 0,063 | 0,125 | ≤0,031 - 0,5 |
| β-Cl-Ala-β-Cl-Ala | 8 | 8 | > 8 | 2 - >8 |
| β-Cl-Ala-β-Cl-Alafosfaline | 0,25 | 0,25 | 1 | ≤0,031 - 1 |
| Fosfomycine | 0,5 | 0,5 | 1 | 0,125 - 8 |

| ***K. pneumoniae* (87)** | | | | |
|---|---|---|---|---|
| Alafosfaline | 2 | 2 | > 8 | 0,25 - >8 |
| Di-alanyl fosfaline | > 8 | > 8 | > 8 | 0,5 - >8 |
| β-Cl-Alafosfaline | 1 | 1 | 8 | 0,125 - >8 |
| β-Cl-Ala-β-Cl-Ala | > 8 | > 8 | > 8 | 8 - >8 |
| β-Cl-Ala-β-Cl-Alafosfaline | 4 | 4 | > 8 | 0,5 - >8 |
| Fosfomycine | 4 | 8 | > 32 | 2 - >32 |

| ***E. cloacae* (27)** | | | | |
|---|---|---|---|---|
| Alafosfaline | 1 | 1 | 1 | 0,125 - 4 |
| Di-alanyl fosfaline | 4 | > 8 | > 8 | 0,25 - >8 |
| β-Cl-Alafosfaline | 0,5 | 0,5 | 0,5 | 0,063 - 1 |
| β-Cl-Ala-β-Cl-Ala | > 8 | > 8 | > 8 | 8 - >8 |
| β-Cl-Ala-β-Cl-Alafosfaline | 1 | 1 | 4 | 0,25 - 8 |
| Fosfomycine | 16 | 16 | 32 | 4 - >32 |

| **EPC (128)** | | | | |
|---|---|---|---|---|
| Alafosfaline | 2 | 1 | 4 | ≤0,031 - >8 |
| Di-alanyl fosfaline | > 8 | 8 | > 8 | 0,063 - >8 |
| β-Cl-Alafosfaline | 1 | 0,5 | 2 | ≤0,031 - >8 |
| β-Cl-Ala-β-Cl-Ala | > 8 | > 8 | > 8 | 2 - >8 |
| β-Cl-Ala-β-Cl-Alafosfaline | 4 | 2 | 8 | ≤0,031 - >8 |
| Fosfomycine | 16 | 4 | 32 | 0,125 - >32 |

| **BLSE (47)** | | | | |
|---|---|---|---|---|
| Alafosfaline | 2 | 2 | > 8 | ≤0,031 - >8 |
| Di-alanyl fosfaline | > 8 | 8 | > 8 | ≤0,031 - >8 |
| β-Cl-Alafosfaline | 0,063 | 0,5 | > 8 | 0,031 - >8 |
| β-Cl-Ala-β-Cl-Ala | > 8 | > 8 | > 8 | 2 - >8 |
| β-Cl-Ala-β-Cl-Alafosfaline | > 8 | 4 | > 8 | 0,063 - >8 |
| Fosfomycine | > 32 | 8 | > 32 | 0,125 - >32 |

| **AmpC (22)** | | | | |
|---|---|---|---|---|
| Alafosfaline | > 8 | 4 | > 8 | 0,063 - >8 |
| Di-alanyl fosfaline | > 8 | > 8 | > 8 | 0,063 - >8 |
| β-Cl-Alafosfaline | > 8 | 2 | > 8 | ≤0,031 - >8 |
| β-Cl-Ala-β-Cl-Ala | > 8 | > 8 | > 8 | 8 - >8 |
| β-Cl-Ala-β-Cl-Alafosfaline | > 8 | 8 | > 8 | 0,25 - >8 |
| Fosfomycine | 32 | 8 | > 32 | 0,125->32 |

| **Tous les *S*. *aureus* (50)** | | | | |
|---|---|---|---|---|
| Alafosfaline | 4 | 4 | 8 | 0,125 - 16 |
| Di-alanyl fosfaline | 4 | 8 | 16 | 0,5 - 32 |
| β-Cl-Alafosfaline | 2 | 1 | 2 | 0,125 - 4 |
| β-Cl-Ala-β-Cl-Ala | 2 | 2 | 4 | 0,125 - 16 |
| β-Cl-Ala-β-Cl-Alafosfaline | 16 | 16 | 16 | 2 - 16 |
| Fosfomycine | 8 | 4 | 16 | 0,5 - >32 |

| **SARM (37)** | | | | |
|---|---|---|---|---|
| Alafosfaline | 4 | 4 | 8 | 0,125 - 16 |
| Di-alanyl fosfaline | 4 | 8 | 16 | 0,5 - 32 |
| β-Cl-Alafosfaline | 2 | 2 | 2 | 0,125 - 2 |
| β-Cl-Ala-β-Cl-Ala | 2 | 2 | 4 | 0,125 - 16 |
| β-Cl-Ala-β-Cl-Alafosfaline | 16 | 16 | 16 | 2 - 16 |
| Fosfomycine | 8 | 4 | 16 | 0,5 - >32 |

| **SASM (13)** | | | | |
|---|---|---|---|---|
| Alafosfaline | 4 | 4 | 8 | 0,25 - 16 |
| Di-alanyl fosfaline | 16 | 8 | 16 | 0,5 - 32 |
| β-Cl-Alafosfaline | 1 | 1 | 2 | 0,5 - 4 |
| β-Cl-Ala-β-Cl-Ala | 1 | 1 | 2 | 0,5 - 2 |
| β-Cl-Ala-β-Cl-Alafosfaline | 16 | 16 | 16 | 4 - 16 |
| Fosfomycine | 4 | 4 | 16 | 2 - 16 |

| **Tous les Enterococci (50)** | | | | |
|---|---|---|---|---|
| Alafosfaline | 8 | 16 | > 32 | 4 - >32 |
| Di-alanyl fosfaline | 0,5 | 0,5 | 2 | ≤0,016 - >32 |
| β-Cl-Alafosfaline | 8 | 8 | 16 | 2 - >32 |
| β-Cl-Ala-β-Cl-Ala | 16 | 16 | 32 | 2 - 16 |
| β-Cl-Ala-β-Cl-Alafosfaline | 8 | 8 | 16 | 0,125 - >32 |
| Fosfomycine | > 32 | > 32 | > 32 | 16 - >32 |

| ***E. faecalis* (11)** | | | | |
|---|---|---|---|---|
| Alafosfaline | 8 | 8 | 32 | 4 - >32 |
| Di-alanyl fosfaline | 0,031 | 0,063 | 0,5 | ≤0,016 - >32 |
| β-Cl-Alafosfaline | 8 | 8 | 16 | 4 - >32 |
| β-Cl-Ala-β-Cl-Ala | 8 | 8 | 16 | 4 - 16 |
| β-Cl-Ala-β-Cl-Alafosfaline | 0,25 | 0,25 | 4 | 0,125 - >32 |
| Fosfomycine | 32 | 32 | > 32 | 32 - >32 |

| ***E. faecium* (34)** | | | | |
|---|---|---|---|---|
| Alafosfaline | 16 | 16 | 16 | 4 - 32 |
| Di-alanyl fosfaline | 0,5 | 0,5 | 2 | ≤0,016 - 4 |
| β-Cl-Alafosfaline | 4 | 4 | 8 | 2 - 32 |
| β-Cl-Ala-β-Cl-Ala | 16 | 16 | 32 | 2 - >32 |
| β-Cl-Ala-β-Cl-Alafosfaline | 8 | 8 | 8 | 0,125 - >32 |
| Fosfomycine | > 32 | > 32 | > 32 | 16 - >32 |

| **ERG (43)** | | | | |
|---|---|---|---|---|
| Alafosfaline | 16 | 16 | > 32 | 4 - >32 |
| Di-alanyl fosfaline | 0,5 | 0,5 | > 32 | ≤0,016 - >32 |
| β-Cl-Alafosfaline | 4 | 8 | 16 | 2 - >32 |
| β-Cl-Ala-β-Cl-Ala | 16 | 16 | > 32 | 4 - >32 |
| β-Cl-Ala-β-Cl-Alafosfaline | 8 | 8 | 16 | 0,125 - >32 |
| Fosfomycine | > 32 | > 32 | > 32 | 32 - >32 |

Abréviations : EPC : *Enterobacteriaceae* productrices de carbapénémase ; BLSE: *Enterobacteriaceae* avec une β-lactamase à spectre étendu ; SARM : *Staphylococcus aureus* résistants à la méticilline ; SASM : *Staphylococcus aureus* sensibles à la méticilline ; ERG : enterocoques résistants aux glycopeptides.

### Exemple 3 : Milieu réactionnel selon l'invention pour la détection de bactéries du genre Salmonella

### 3.1 Composition du milieu

Le présent Exemple 3 a pour but de comparer la spécificité de détection d'un milieu réactionnel selon l'invention, destiné à détecter les bactéries du genre Salmonella, à savoir le milieu «chromID Salmonella modifié » par rapport à un milieu de détection de référence pour ces mêmes bactéries, à savoir le milieu « chromID Salmonella ».

Les compositions respectives du milieu réactionnel selon la présente invention et du milieu réactionnel de référence sont présentées ci-après, au sein du Tableau 2.

**Tableau 2 : Compositions du milieu réactionnel selon l'invention et du milieu de référence**

| **Milieu chromID Salmonella modifié=beta** | | **Milieu chromID Salmonella** = **IDSalm** | |
|---|---|---|---|
| | g/l | | g/l |
| Peptones | 6,25g | Peptones | 6,25g |
| Glucose | 0,5 | Glucose | 0,5 |
| Sels biliaires | 1,5 | Sels biliaires | 1,5 |
| NaCl | 5,0 | NaCl | 5,0 |
| Tampon | 0,2 | Tampon | 0,2 |
| Agar | 14,0 | Agar | 14,0 |
| Mélange chromogène | 9,6 | Mélange chromogène | 9,6 |
| β-Cl-Alafosfaline | 0,002 | | |

### 3.2 Résultats

Les résultats obtenus sont présentés dans le tableau 3 infra :

**Tableau 3 : Résultats**

| | | **IDSalm** | | **beta** | |
|---|---|---|---|---|---|
| **Reference #** | **Bactérie** | **Croissance** | **Couleur** | **Croissance** | **Couleur** |
| | *Salmonella montevideo* | ++ | ++ Mauve | ++ | ++ Mauve |
| | *Salmonella seftenberg* | ++ | ++ Mauve | ++ | ++ Mauve |
| | *Salmonella choleraesuis* | ++ | ++ Mauve | ++ | ++ Mauve |
| | *Salmonella meleagridis* | ++ | ++ Mauve | ++ | ++ Mauve |
| NCTC 12023 | *Salmonella typhimurium* | ++ | ++ Mauve | ++ | ++ Mauve |
| | *Salmonella berta* | ++ | ++ Mauve | ++ | ++ Mauve |
| | *Salmonella stanley* | ++ | ++ Mauve | ++ | ++ Mauve |
| NCTC 6676 | *Salmonella enteritidis* | ++ | ++ Mauve | ++ | ++ Mauve |
| | *Salmonella simsburg* | ++ | ++ Mauve | ++ | ++ Mauve |
| | *Salmonella lexington* | ++ | ++ Mauve | ++ | ++ Mauve |
| | *Salmonella limete* | ++ | ++ Mauve | ++ | ++ Mauve |
| | *Salmonella corvalis* | ++ | ++ Mauve | ++ | ++ Mauve |
| | *Salmonella haifa* | ++ | ++ Mauve | ++ | ++ Mauve |
| | *Salmonella zanzibar* | ++ | ++ Mauve | ++ | ++ Mauve |
| | *Salmonella indiana* | ++ | ++ Mauve | ++ | ++ Mauve |
| | *Salmonella javiana* | ++ | ++ Mauve | ++ | ++ Mauve |
| NCTC 11304 | *Salmonella indiana* | ++ | ++ Mauve | ++ | ++ Mauve |
| | *Salmonella oranienburg* | ++ | ++ Mauve | ++ | ++ Mauve |
| NCTC 4840 | *Salmonella poona* | ++ | ++ Mauve | ++ | ++ Mauve |
| | *Salmonella tennessee* | ++ | ++ Mauve | ++ | ++ Mauve |
| | *Salmonella emek* | ++ | ++ Mauve | ++ | + Mauve |
| | *Salmonella virohrady* | ++ | ++ Mauve | ++ | ++ Mauve |
| | *Salmonella java* | ++ | ++ Mauve | ++ | ++ Mauve |
| | *Salmonella augustenborg* | ++ | ++ Mauve | ++ | ++ Mauve |
| | *Salmonella alachia* | ++ | ++ Mauve | ++ | ++ Mauve |
| | *Salmonella panama* | ++ | ++ Mauve | ++ | ++ Mauve |
| | *Salmonella virchow* | ++ | ++ Mauve | ++ | ++ Mauve |
| | *Salmonella paratyphi* | ++ | ++ Mauve | ++ | ++ Mauve |
| | *Salmonella orthmarschen* | ++ | ++ Mauve | ++ | ++ Mauve |
| | *Salmonella vilvorde* | ++ | ++ Mauve | ++ | ++ Mauve |
| | *Salmonella gallinarium* | + | +/- Mauve | + | +/- Mauve |
| NCTC 8385 | *Salmonella typhi* | ++ | + Mauve | + | - |
| NCTC 9528 | *K. pneumoniae* | ++ | ++ Bleue | ++ | ++ Bleue |
| NCTC 11936 | *E. cloacae* | ++ | + Bleue | ++ | + Bleue |
| NCTC 10322 | *S. marcescens* | ++ | ++ Bleue | ++ | ++ Bleue |
| NCTC 10662 | *P. aeruginosa* | - | - | - | - |
| NCTC | *S. maltophilia* | +/- | - | +/- | - |
| NCTC 19606 | *A. baumannii* | ++ | ++ Mauve | ++ | ++ Mauve |
| NCTC 7475 | *P. rettgeri* | - | - | - | - |
| WILD 462213 | *M. morganii* | ++ | - | ++ | - |
| 69052 | *Escherichia coli* | ++ | - | ++ | - |
| 68957 | *Escherichia coli* | ++ | - | ++ | - |
| 68958 | *Escherichia coli* | ++ | +/- Bleue | ++ | - |
| 68805 | *Escherichia coli* | ++ | +/- Bleue | + | - |
| 69367 | *Escherichia coli* | + | +/- Bleue | + | - |
| 69371 | *Escherichia coli* | ++ | - | ++ | - |
| 69035 | *Escherichia coli* | ++ | Tr. Bleue | ++ | - |
| 69051 | *Escherichia coli* | + | +/- Bleue | + | - |
| 69017 | *Escherichia coli* | ++ | - | ++ | - |
| 69148 | *Escherichia coli* | - | - | - | - |
| 68880 | *Escherichia coli* | ++ | - | ++ | - |
| 68886 | *Escherichia coli* | ++ | - | ++ | - |
| 69102 | *Escherichia coli* | ++ | - | ++ | - |
| 69157 | *Escherichia coli* | 1 colonie | - | +/- | - |
| 69285 | *Escherichia coli* | 2 colonies | - | +/- | - |
| 69130 | *Escherichia coli* | ++ | - | ++ | - |
| 69134 | *Escherichia coli* | ++ | - | ++ | - |
| 69135 | *Escherichia coli* | + | - | + | - |
| 69174 | *Escherichia coli* | - | - | - | - |
| 69176 | *Escherichia coli* | +/- | +/- Bleue | +/- | - |

### 3.3 Conclusion

L'ajout de β-Cl-Alafosfaline dans le milieu chromID Salmonella permet d'inhiber sélectivement les activités osidases des souches d'*Escherichia coli.* Dans la mesure où il s'avère plus aisé de détecter une à quelques colonies mauves au milieu de colonies incolores qu'au milieu de colonies bleues, lorsqu'une souche de salmonelle est présente à faible concentration en mélange avec une ou plusieurs souches d'*Escherichia coli,* il devient dès lors significativement plus aisé de la détecter sur le milieu réactionnel de la présente invention.

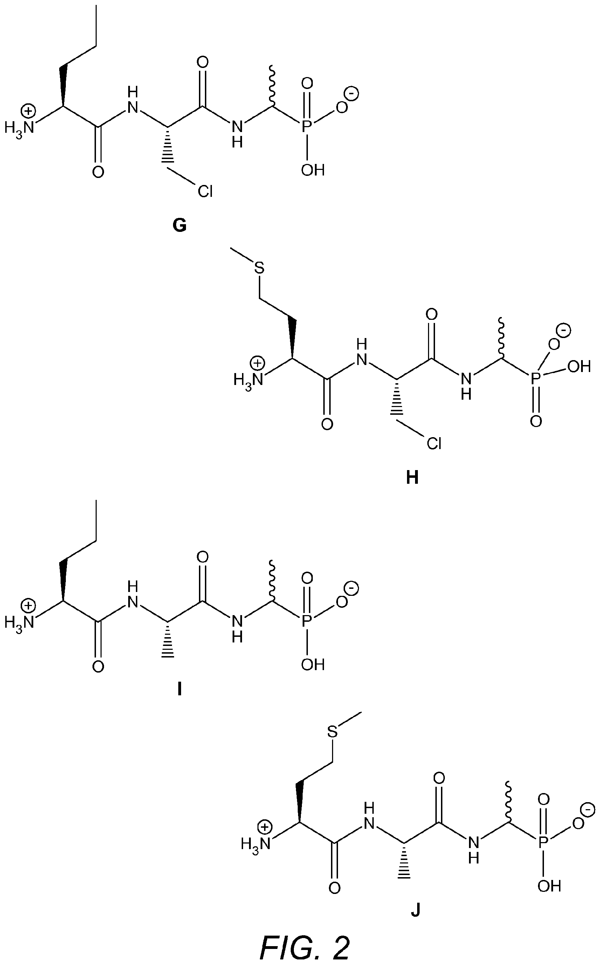

### Exemple 4 : Procédé de synthèse du composé antimicrobien G selon l'invention (figure 2) : la L-Norvalinyl-β-chloro-L-alanyl-D/L-fosfaline

La L-Norvalinyl-β-chloro-L-alanyl-D/L-fosfaline (L-Nva-β-Cl-L-Ala-D/L-fosfaline) est représentée par la formule suivante :

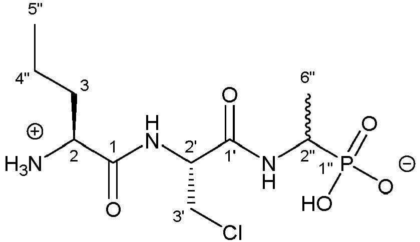

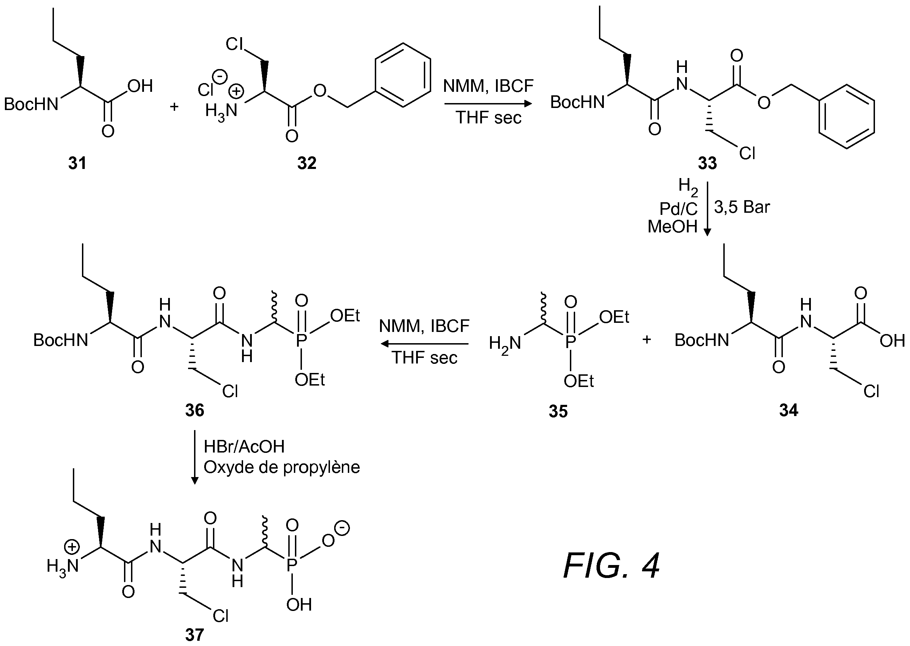

La synthèse de ce composé antimicrobien est détaillée ci-après. Le procédé de synthèse correspondant est en outre illustré en figure 4.

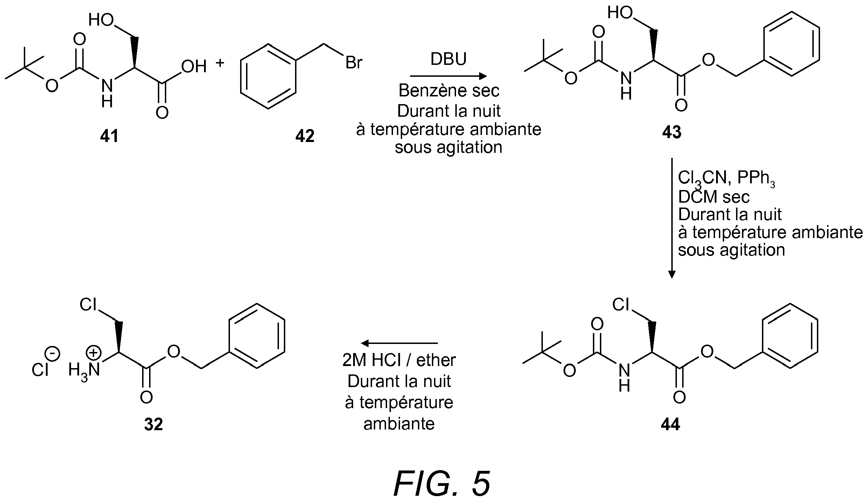

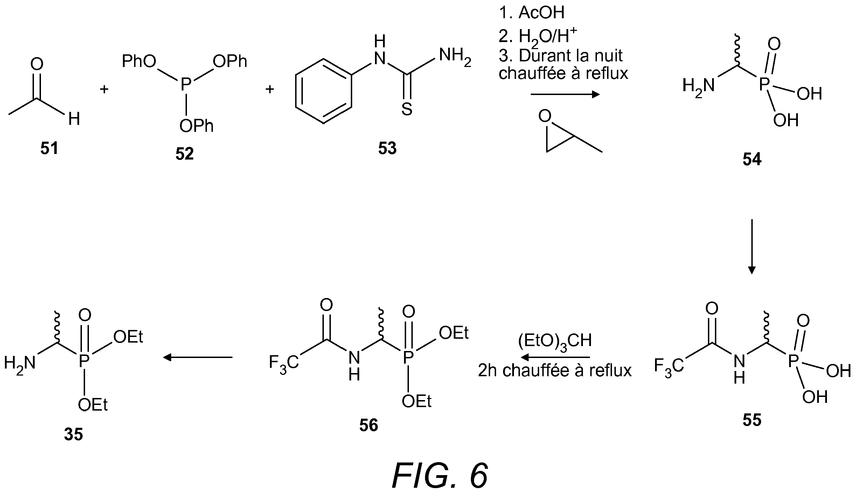

Tel que représenté sur la figure 4, le susdit composé selon l'invention a été synthétisé à partir de ^{t}Boc-L-norvaline (disponible dans le commerce), d'ester *O*-benzylique de **β**-chloro-L-alanine (dont la synthèse est représentée en figure 5) et d'ester diéthylique de D/L-fosfaline (dont la synthèse est représentée en figure 6).

### 4.1 Synthèse de l'intermédiaire réactionnel ester O-benzylique de β-chloro-L-alanine 32 à partir du composé ^{t}Boc-L-sérine 41 (comme illustré en figure 5).

### 4.1.1 Synthèse du (S)-benzyl 2-((tert-butoxycarbonyl)amino)-3-hydroxypropanoate (ester O-benzylique de ^{t}Boc-L-sérine) 43 [9]

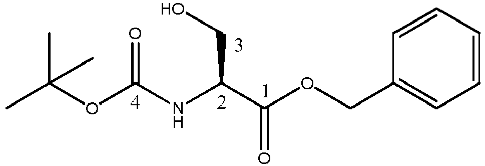

De la *^{t}*Boc-L-sérine (disponible dans le commerce) **41** (30 mmol, 6,16 g) a été dissoute dans du benzène sec (100 mL), auquel on a ensuite ajouté du 1,8-diazabicyclo[5,4,0]undéc-7-ène (DBU) (36 mmol, 5,5 mL) et du bromure de benzyle **42** (36 mmol, 4,4 mL). La solution a été agitée toute une nuit à température ambiante sous azote, et le solvant a été éliminé plus tard sous pression réduite pour donner un résidu liquide blanc cassé. On a ajouté de l'acétate d'éthyle (200 mL), le contenu du flacon a été ultrasoniqué, puis lavé avec une solution 1 M de HCl (2 x 50 mL), une solution aqueuse à 10 % poids/volume de K₂CO₃ (2 x 50 mL) et de la saumure (2 x 50 mL). La couche organique a été séchée sur MgSO₄, filtrée, concentrée sous vide et purifiée par chromatographie sur colonne [essence/acétate d'éthyle (1:1)] pour donner le produit **43** sous forme de solide blanc (8,05 g, 27,3 mmol, 91 %) ; p.f. 61 - 66 °C (p.f. lit. **[10]** 59 - 60 °C) ; [α]²¹D -18,5° (c 1,0, CH₃OH) ; *v*ₘₐₓ/cm⁻¹ 3419 (NH), 3361 (OH), 2978 (CH), 1758 (C=O), 1668 (C=O), 1524 (flexion NH), 1155 (C-O), 1068 (C-O) ; ¹H RMN (300 MHz, DMSO) δ_{H} 1,38 (9H, s, C(CH₃)₃), 3,68 (2H, t, *J*=6,0 Hz, CH₂OH), 4,10-4,16 (1H, m, CH-2), 4,91 (1H, t, *J*=6,0 Hz, OH), 5,10 (1H, d, *J*=12,0 Hz, OCH₂ₐAr), 5,17 (1H, d, *J*=12,0 Hz, OCH_{2b}Ar), 6,97 (1H, d, *J*=9,0 Hz, NH), 7,32-7,38 (5H, m, 5 x CH_{Ar}) ; ¹³C RMN (75 MHz, DMSO) δ_{C} 28,6 (C(CH₃)₃), 57,0 (CH-2), 61,8 (CH₂-3), 66,2 (OCH₂Ar), 78,8 (C(CH₃)₃), 128,0 (CH_{Ar}), 128,4 (CH_{Ar}), 128,8 (CH_{Ar}), 136,5 (CH_{Ar} quat), 155,8 (C-4, quat), 171,4 (C-1, quat).

### 4.1.2 Synthèse de (R)-benzyl 2-((tert-butoxycarbonyl)amino)-3-chloropropanoate (ester O-benzylique de ^{t}Boc-β-chloro-L-alanine) 44 [11]

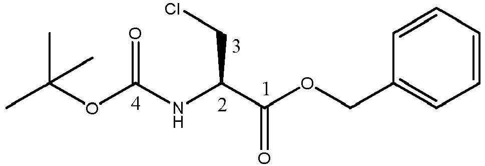

Le ((*S*)-benzyl 2-((*tert*-butoxycarbonyl)amino)-3-hydroxypropanoate **43** (25 mmol, 7,39 g), obtenu à l'étape 4.1.1 supra, a été dissous dans du DCM sec (100 mL), puis du trichloro-acétonitrile (50 mmol, 5,0 mL) a été ajouté. La solution a été agitée à température ambiante pendant 2 heures. À cette solution, on a ajouté lentement de la triphénylphosphine (50 mmol, 13,15 g) dans du DCM sec (50 mL). La solution ainsi obtenue a été agitée toute une nuit à température ambiante sous azote. On a ajouté de la saumure (100 mL) pour arrêter la réaction. Après séparation, la couche organique a été lavée avec de la saumure (3 x 60 mL), séchée sur MgSO₄, filtrée et concentrée sous vide pour donner un résidu liquide orange. Le résidu a été purifié par chromatographie sur colonne [essence/acétate d'éthyle (7:3)] pour donner le produit **44** sous forme de solide blanc cassé (7,41 g, 23,6 mmol, 95 %) ; p.f. 53 - 58 °C ; [α]²²D -23,0° (c 1,0, CH₃OH) ; *v*ₘₐₓ/cm⁻¹ 3365 (NH), 2917 (CH), 1727 (C=O), 1679 (C=O), 1522 (flexion NH), 1181 (C-O), 1158 (C-O) ; ¹H RMN (300 MHz, CDCl₃) δ_{H} 1,45 (9H, s, C(CH₃)₃), 3,85 (1H, dd, *J*=12,0 Hz, 3,0 Hz, CH₂ₐ-3), 3,99 (1H, dd, *J*=12,0 Hz, 3,0 Hz, CH_{2b}-3), 4,74 (1H, m, CH-2), 5,20 (1H, d, *J*=12,0 Hz, OCH₂ₐAr), 5,25 (1H, d, *J*=12,0 Hz, OCH_{2b}Ar), 5,44 (1H, d, *J*=6,0 Hz, NH), 7,33-7,38 (5H, m, 5 x CH_{Ar}) ; ¹³C RMN (75 MHz, CDCl₃) δ_{C} 27,3 (C(CH₃)₃), 44,5 (CH₂-3), 53,5 (CH-2), 66,8 (OCH₂Ar), 79,5 (C(CH₃)₃), 127,3 (CH_{Ar}), 127,6 (CH_{Ar}), 127,6 (CH_{Ar}), 133,9 (CH_{Ar} quat), 154,0 (C-4, quat), 168,0 (C-1, quat) ; CHN [Trouvé : C, 57,71 ; H, 6,46 ; N, 4,18. C₁₅H₂₀ClNO₄ demande C, 57,42 ; H, 6,42 ; N, 4,46 %].

### 4.1.3 Synthèse de chlorure de (R)-1-(benzyloxy)-3-chloro-loxopropan-2-aminium (chlorhydrate de β-chloro-L-alanine) 32

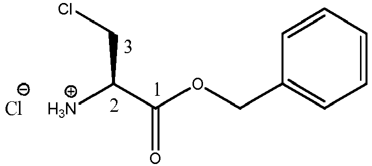

Le composé **44** (10 mmol, 3,14g), obtenu à l'étape 4.1.2. supra, a été dissous dans une solution 2M de HCl dans de l'éther (200 mL). La solution a été agitée à température ambiante toute une nuit. Le solide ainsi obtenu a été filtré et lavé avec de l'éther diéthylique pour donner le produit **32** sous forme de solide blanc (2,36 g, 9,5 mmol, 95 %) ; p.f. 145 °C (secondaire) ; *v*ₘₐₓ/cm⁻¹ 2848 (CH), 1749 (C=O), 1593 (Ar C-C), 1490 (Ar C-C), 1231 (C-O) ; ¹H RMN (300 MHz, DMSO) δ_{H} 4,16 (1H, dd, *J*=12,0 Hz, 3,0 Hz, CH₂ₐ-3), 4,22 (1H, dd, *J*=12,0 Hz, 3,0 Hz, CH_{2b}-3), 4,77 (1H, t, *J*=3,0 Hz, CH-2), 5,26 (1H, d, *J*=12,0 Hz, OCH₂ₐAr), 5,31 (1H, d, *J*=15,0 Hz, OCH_{2b}Ar), 7,33-7,46 (5H, m, 5 x CH_{Ar}), 9,09 (3H, br, NH₃⁺) ; ¹³C RMN (75 MHz, DMSO) δ_{C} 43,3 (CH₂-3), 53,5 (CH-2), 68,0 (OCH₂Ar), 128,6 (CH_{Ar}), 128,8 (CH_{Ar}), 128,9 (CH_{Ar}), 135,4 (CH_{Ar} quat), 167,0 (C-1, quat) ; CHN [Trouvé : C, 47,16 ; H, 5,43 ; N, 5,43. C₁₀H₁₃Cl₂NO₂·0,2H₂O demande C, 47,34 ; H, 5,32 ; N, 5,52 %].

### 4.2 Synthèse du (R)-benzyl 2-((S)-2-((tert-butoxycarbonyl)amino)pentanamido)-3-chloropropanoate (ester O-benzylique de ^{t}Boc-L-Norvalinyl-β-chloro-L-alanine) 33

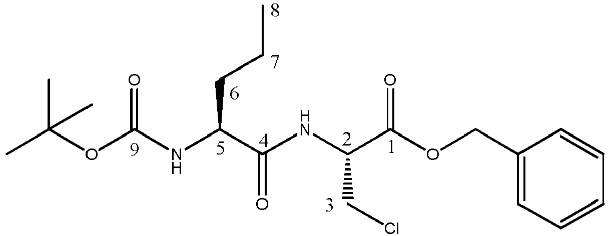

De la *^{t}*Boc-L-Norvaline (disponible dans le commerce) **31** (6,0 mmol, 1,31 g) a été dissoute dans du THF sec (50 mL) et de la *N*-méthyl morpholine (6,0 mmol, 0,66 mL) a été ajoutée. La solution a ensuite été refroidie à 0 °C et on a ajouté goutte à goutte du chloroformiate d'isobutyle (6,0 mmol, 0,78 mL). Le mélange a été agité à 0 °C sous azote pendant 1 heure. À la solution agitée, on a ajouté du chlorure de (*R*)-1-(benzyloxy)-3-chloro-loxopropan-2-aminium (sel chlorure d'ester benzylique de β-Cl-L-alanine) **32** (5,4 mmol, 1,36 g), obtenu à l'étape 4.1.3 supra, dans du DCM sec (30 mL) précédemment neutralisé par de la *N*-méthyl morpholine (5,4 mmol, 0,59 mL) à 0 °C. La solution ainsi obtenue a été agitée toute une nuit sous azote à température ambiante. La solution a été filtrée et concentrée sous vide. Le résidu a été dissous dans du DCM (60 mL) et lavé par une solution à 10 % poids/volume d'acide citrique (2 x 25 mL), puis par de l'eau (25 mL). La couche organique a été séchée sur MgSO₄, filtrée et concentrée sous vide pour donner un liquide jaune qui a été purifié par chromatographie sur colonne [essence/acétate d'éthyle (7:3)] pour donner le produit **33** sous forme de solide blanc (1,74 g, 4,2 mmol, 78 %) ; p.f. 95 - 98 °C ; [α]²⁵_{D} -25,0° (c 1,0, CH₃OH) ; *v*ₘₐₓ/cm⁻¹ 3327 (NH), 2960 (CH), 1738 (C=O), 1668 (C=O), 1518 (flexion NH), 1169 (C-O); ¹H RMN (300 MHz, CDCl₃) δ_{H} 0,92 (3H, t, *J*=9,0 Hz, CH₃-8), 1,35-1,45 (11H, [m, CH₂-7], [s, C(CH₃)₃]), 1,52-1,65 (1H, m, CH₂ₐ-6), 1,75-1,82 (1H, m, CH_{2b}-6), 3,89 (1H, dd, *J*=12,0 Hz, 3,0 Hz, CH₂ₐ-3), 3,99 (1H, dd, *J*=12,0 Hz, 3,0 Hz, CH_{2b}-3), 4,11-4,15 (1H, m, CH-5), 4,96-5,00 (2H, [m, CH-2], [m, HNCO₂]), 5,20 (1H, d, *J*=12,0 Hz, OCH₂ₐAr), 5,25 (1H, d, *J*=12,0 Hz, OCH₂ₐ-Ar), 6,97 (1H, d, *J*=6,0 Hz, HNCO), 7,33-7,37 (5H, m, 5 x CH_{Ar}) ; ¹³C RMN (75 MHz, CDCl₃) δ_{C} 12,7 (CH₃-8), 17,8 (CH₂-7), 27,3 (C(CH₃)₃), 33,4 (CH₂-6), 43,8 (CH₂-3), 52,2 (CH-2), 53,4 (CH-5), 67,0 (OCH₂Ar), 79,3 (C(CH₃)₃), 127,4 (CH_{Ar}), 127,6 (CH_{Ar}), 127,7 (CH_{Ar}), 133,8 (CH_{Ar} quat), 154,5 (C-9, quat), 167,5 (C-1, quat), 171,2 (C-4, quat) ; CHN [Trouvé : C, 58,49 ; H, 7,22 ; N, 6,81. C₂₀H₂₉ClN₂O₅ demande C, 58,18 ; H, 7,08 ; N, 6,78 %] ; SMHR (ionisation par nanopulvérisation) calculé pour (C₂₀H₃₀ClN₂O₅)⁺413,1838, trouvé MH⁺ 413,1837.

### 4.3 Synthèse de l'acide (R)-2-((S)-2-((tert-butoxycarbonyl)amino)pentanamido)-3-chloropropanoïque (^{t}Boc-L-norvalinyl- β-chloro-L-alanine) 34

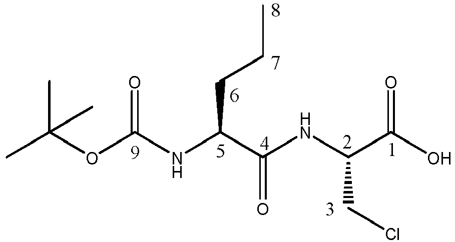

Le produit **33,** (1,8 mmol, 0,75 g), obtenu à l'étape 4.2 supra, a été dissous dans du méthanol (60 mL) et on l'a ajouté dans le récipient sous pression en acier inoxydable. On a ajouté 10 % de palladium sur charbon (0,0941 g) et la solution ainsi obtenue a été agitée à une pression de H₂ de 3,5 bar à température ambiante pendant 72 heures. Le catalyseur a été éliminé par filtration sur Célite, lavé avec du méthanol et concentré sous vide pour donner le produit **34** sous forme de solide jaune clair (0,573 g, 1,78 mmol, 99 %) ; p.f. 59 - 63 °C ; [α]²⁵_{D} -12,0° (c 1,0, CH₃OH) ; *v*ₘₐₓ/cm⁻¹ 3313 (NH), 2964 (CH), 1655 (C=O), 1509 (flexion NH), 1161 (C-O) ; ¹H RMN (300 MHz, CDCl₃) δ_{H} 0,87(3H, t, *J*=4,2 Hz, CH₃-8), 1,38 (11H, [m, CH₂-7], [s, C(CH₃)₃]), 1,51-1,62 (1H, m, CH₂ₐ-6), 1,67-1,79 (1H, m, CH_{2b}-6), 3,91 (2H, m, CH₂-3), 4,19 (1H, m, CH-5), 4,85 (1H, m, CH-2), 5,20 (1H, m, NH carbamate), 6,45 (1H, br, OH), 7,25 (1H, m, NH amide) ; ¹³C RMN (75 MHz, CDCl₃) δ_{C} 12,7 (CH₃-8), 17,9 (CH₃-7), 27,3 (C(CH₃)₃), 33,6 (CH₂-6), 43,4 (CH-3), 52,4 (CH-2), 53,2 (CH-5), 79,7 (C(CH₃)₃), 155,1 (C-9, quat), 170,7 (C-1/4, quat), 172,0 (C-1/4, quat) ; CHN [Trouvé : C, 48,77 ; H, 7,61 ; N, 8,22. C₁₃H₂₃ClN₂O₅ demande C, 48,37 ; H, 7,18 ; N, 8,68 %].

### 4.4 Synthèse de l'intermédiaire réactionnel ester diéthylique de D/L-fosfaline 35

Comme représenté sur la figure 6, l'ester diéthylique de D/L-fosfaline **35** a été synthétisé à partir de *N*-phenylthio-urée **53,** de triphénylphosphite **52** et d'acétaldéhyde **51** en conditions acides, selon le procédé de Kudzin et Stec **[9]**. La synthèse de cet intermédiaire réactionnel est détaillée ci-après.

### 4.4.1 Synthèse d'acide (β)-(1-aminoéthyl)phosphonique (D/L-fosfaline) 54 [12]

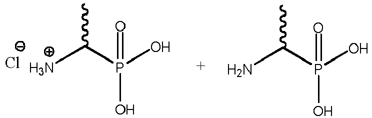

De la *N*-phénylthio-urée **53** (40 mmol, 6,10 g) a été dissoute dans de l'acide acétique glacial (20 mL). On a ajouté goutte à goutte de l'acétaldéhyde **51** (60 mmol, 3,40 mL), puis on a ajouté du triphényl phosphite **52** (40 mmol, 11 mL). La solution a été agitée à température ambiante pendant 5 min, puis chauffée à reflux à 85°C pendant 1 heure. Un mélange d'acide acétique glacial (2 mL) et d'acide chlorhydrique (37 %, 20 mL) a été ajouté et la réaction a été chauffée à reflux toute une nuit à 145°C. La solution a été refroidie à température ambiante, transférée et lavée avec de l'éthanol dans un flacon à fond rond de 500 mL. Une petite quantité de sel chlorhydrate de fosfaline a été obtenue par filtration et le filtrat a été concentré sous vide pour donner un résidu liquide orange sombre. Le résidu a été dissous dans une quantité minimale d'éthanol (20 mL) et on a ajouté de l'oxyde de propylène (120 mL) pour produire un précipité blanc. Le solide blanc a été filtré sous azote et séché dans un dessiccateur (sur pentoxyde de phosphore) pendant 3 jours, ce qui a été suivi d'une recristallisation à partir d'eau chaude/éthanol pour donner le produit zwittérionique **54** sous forme de solide blanc (4,39 g, 35 mmol, 88 %) ; p.f. 265 - 268 °C (s) (p.f. lit. [13] 271 - 275 °C) ; *v*ₘₐₓ/cm⁻¹ 2910 (br OH), 1616 (P-OH), 1532 (flexion NH), 1143 (P=O), 1035 (P-O), 930 (P-O) ; ¹H RMN (500 MHz, D₂O) δ_{H} 1,47 (3H, dd, *J_{P-H}*=14,9 Hz et J_{H-H} = 7,3 Hz, CH₃), 3,40 (1H, m, CH) ; ¹³C RMN (125 MHz, D₂O) δ_{C} 13,5 (CH₃, d, *J_{P-C}* =2,7 Hz), 44,7 (CH, d, *J_{P-C}* =144,2 Hz).

### 4.4.2 Synthèse de diéthyl (1-(2,2,2-trifluoro-acétamido)éthyl)phosphonate 56 [14]

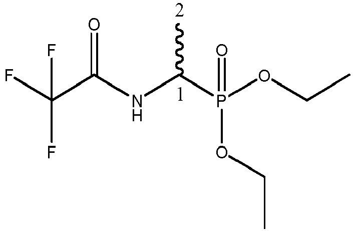

De l'acide 1-aminoéthylphosphonique **44** (40 mmol, 6,47 g), obtenu à l'étape 4.4.1. supra, a été ajouté à un mélange d'acide trifluoroacétique (5 mL) et d'anhydride trifluoroacétique (25 mL). La solution a été agitée et chauffée à reflux à 60 °C. Après 1 heure, la solution a été refroidie et on a ajouté lentement de l'orthoformiate de triéthyle (150 mL). La solution a été chauffée à reflux à 110 °C pendant 2 heures, puis refroidie à température ambiante. Le solvant a été éliminé sous vide pour donner un solide brun. Le solide a été redissous dans du DCM et purifié par chromatographie sur colonne [DCM/MeOH (9:1)] pour donner le produit **56** sous forme de solide blanc cassé (11,00 g, 39,6 mmol, 99 %) ; p.f. 98 - 103 °C (s) (p.f. lit.³ 101 - 102 °C) ; vₘₐₓ/cm⁻¹ 3202 (NH), 1715 (C=O), 1565 (flexion NH), 1210 (P=O), 1011 (C-F), 968 (P-O) ; ¹H RMN (300 MHz, CDCl₃) δ_{H} 1,23 (3H, t, *J*=6,0 Hz, OCH₂CH₃-a), 1,28 (3H, t, *J*=9,0 Hz, OCH₂CH₃-b), 1,38 (3H, dd, *J_{P-H}* =15,0 Hz et *J_{H-H}* = 6,0 Hz, CH₃-2), 3,98-4,13 (4H, m, 2 x OCH₂CH₃), 4,32-4,47 (1H, m, CH-1), 8,11 (1H, d, *J*=9,0 Hz, NH) ; ¹³C RMN (75 MHz, CDCl₃) δ_{C} 13,7 (CH₃-2), 15,2 (OCH₂CH₃, d, *J_{P-C}* =2,3 Hz), 15,3 (OCH₂CH₃, d, *J_{P-C}* =1,5 Hz), 40,8 (CH-1, d, *J_{P-C}* =159,0 Hz), 61,8 (OCH₂CH₃, d, *J_{P-C}* =6,8 Hz), 62,2 (OCH₂CH₃, d, *J_{P-C}* =7,5 Hz), 114,9 (CF₃, q, *J_{F-C}* =285,8 Hz), 156,0 (C=O, q, *J_{F-C}* =6,0 Hz) ; ³¹P-¹H_{decoup} RMN (121 MHz, CDCl₃) δ_{P} 23,0; ¹⁹F-¹H_{decoup} RMN (282 MHz, CDCl₃) δ_{P}-75,5.

### 4.4.3 Synthèse du (β)-diéthyl (1-aminoéthyl)phosphonate (ester diéthylique de D/L-fosfaline) 35 [15]

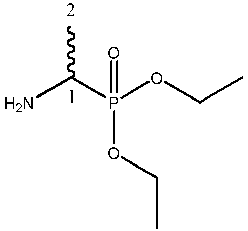

Le diéthyl (1-(2,2,2-trifluoro-acétamido)éthyl)phosphonate **56** (20 mmol, 5,55 g), obtenu à l'étape 4.4.2. supra, a été dissous dans de l'éthanol (200 mL) et du borhydrate de sodium (200 mmol, 7,57 g) a été lentement ajouté. Le mélange ainsi obtenu a été agité à température ambiante pendant 1 heure, puis on a chauffé à reflux (90 °C) pendant 3 heures. La solution a été refroidie à température ambiante et le solvant a été éliminé sous pression réduite pour donner un résidu solide blanc. Le résidu a été traité avec une solution saturée de NaHCO₃ (96 g/L) (150 mL) et extrait dans du DCM (6 x 50 mL). La couche organique a été séchée sur MgSO₄ et filtrée. Le filtrat a été concentré sous vide pour donner un liquide jaune clair, et purifié par chromatographie sur colonne [DCM/MeOH (9,0:1,0)] pour donner le produit **35** sous forme de liquide jaune (2,52 g, 13,9 mmol, 70 %) ; *v*ₘₐₓ/cm⁻¹ 3431 (NH), 2980 (CH), 1215 (P=O), 1020 (P-O), 957 (P-O) ; ¹H RMN (300 MHz, CDCl₃) δ_{H} 1,19-1,30 (9H, [dd, *J_{P-H}* =17,4 Hz, 7,2 Hz, CH₃-2], [t, *J*=7,2 Hz, 2 x OCH₂CH₃), 1,65 (2H, br, NH₂), 2,99-3,09 (1H, m, CH-1), 4,02-4,14 (4H, m, 2 x OCH₂CH₃); ¹³C RMN (75 MHz, CDCl₃) δ_{C} 15,5 (OCH₂CH₃-a), 15,6 (OCH₂CH₃-b), 16,3 (CH₃-2), 43,3 (CH-1, d, *J_{P-C}* =148,5 Hz), 61,1 (OCH₂CH₃-a, d, *J_{P-C}* =1,5 Hz), 61,2 (OCH₂CH₃-b, d, *J_{P-C}* =1,5 Hz) ; ³¹P-¹H_{decoup} RMN (121 MHz, CDCl₃) δ_{P} 29,6.

### 4.5 Synthèse de tert-butyl ((2S)-1-(((2R)-3-chloro-1-((1-(diéthoxyphosphoryl)éthyl)amino)-1-oxopropan-2-yl)amino)-1-oxopentan-2-yl)carbamate (ester diéthylique de ^{t}Boc-L-norvalinyl-β-chloro-L-alanyl-D/L-fosfaline) 36

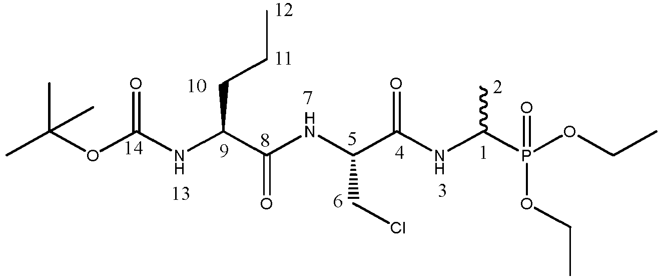

L'acide (*R*)-2-((*S*)-2-((*tert*-butoxycarbonyl)amino)pentanamido)-3-chloropropanoïque (^{t}Boc-L-norvalinyl-β-chloro-L-alanine) **34** (1,8 mmol, 0,58 g), obtenu à l'étape 4.3 supra, a été dissous dans du THF sec (35 mL), puis de la *N*-méthyl morpholine (1,9 mmol, 0,21 mL) a été ajoutée. La solution a été refroidie à 0 °C et on a ajouté goutte à goutte du chloroformiate d'isobutyle (1,9 mmol, 0,25 mL). Le mélange a été agité à 0 °C pendant 1 heure. À la solution agitée, on a ajouté du diéthyl 1-aminoéthylphosphonate (ester diéthylique de D/L-fosfaline) **35** (1,8 mmol, 0,33 g) - obtenu à l'étape 4.4.3. supra - dans du THF sec (10 mL) et la solution ainsi obtenue a été agitée toute une nuit sous azote à température ambiante. La solution a été filtrée et concentrée sous vide. Le résidu a été dissous dans du DCM (60 mL) et lavé par une solution à 10 % poids/volume d'acide citrique (2 x 30 mL), 10 % poids/volume de carbonate de potassium (30 mL) et de l'eau (30 mL). La couche organique a été séchée sur MgSO₄, filtrée et concentrée sous vide pour donner un résidu liquide jaune clair. Le résidu a été purifié par chromatographie sur colonne [acétate d'éthyle/méthanol (96:4)] pour donner le produit **36** sous forme de solide blanc collant (0,45 g, 0,93 mmol, 52 %) ; p.f. 196 °C (décomposition) ; [α]²⁴_{D} -22,5° (c 1,0, CH₃OH) ; *v*ₘₐₓ/cm⁻¹ 3272 (NH), 2977 (CH), 1709 (C=O), 1644 (C=O), 1530 (flexion NH), 1229 (C-O), 1165 (P-O), 1019 (P-O), 972 (P-O) ; ¹H RMN (300 MHz, CDCl₃) δ_{H} 0,86 (3H, t, *J*=6,0 Hz, CH₃-12), 1,17-1,38 (20H, [m, 2 x OCH₂CH₃], [m, CH₃-2], [s, C(CH₃)₃], [m, CH₂-11]), 1,53-1,59 (1H, m, CH₂ₐ-10), 1,70-1,86 (1H, m, CH_{2b}-10), 3,69 (1H, dd, *J*=12,0 Hz, 3,0 Hz, CH₂ₐ-6), 3,91 (1H, dd, *J*=12,0 Hz, 3,0 Hz, CH₂ₐ-6), 3,97-4,13 (5H, [m, 2 x OCH₂CH₃], [m, CH-9]), 4,35-4,46 (1H, m, CH-1), 4,73-4,79 (1H, m, CH-5), 4,97-5,03 (1H, m, NH-13), 7,00 -7,10 (1H, 2 x d, *J*=9,0 Hz, 9,0 Hz, NH-7, diastéréo-isomères L,L,L et L,L,D), 7,23-7,34 (1H, 2 x d, *J*=9,0 Hz, 9,0 Hz, NH-3, diastéréo-isomères L,L,L et L,L,D) ; ¹³C RMN (75 MHz, CDCl₃) δ_{C} 12,7 (CH₃-12), 14,5 (CH₃-2), 15,3, 15,5 (2 x OCH₂CH₃), 17,9 (CH₂-11), 27,3 (C(CH₃)₃), 33,2 (CH₂-10), 40,4 (d, *J_{P-C}*=157,5 Hz, CH-1), 43,4 (CH₂-6), 52,7 (CH-5), 61,4, 61,9 (2 x OCH₂CH₃), 79,4 (C(CH₃)₃), 155,0 (C-14, quat), 166,8 (C-4, quat), 171,4 (C-8, quat) ; ³¹P-¹H_{decoup} RMN (121 MHz, CDCl₃) δ_{P} 24,8 ; SMHR (ionisation par nanopulvérisation) calculé pour (C₁₉H₃₈ClN₃O₇P)⁺ 486,2130, trouvé 486,2124 ; CHN [Trouvé : C, 46,51 ; H, 7,76 ; N, 8,21. C₁₉H₃₇ClN₃O₇P demande C, 46,96 ; H, 7,67 ; N, 8,65 %].

### 4.6 Synthèse d'hydrogéno (1-((R)-2-((S)-2-ammoniopentanamido)-3-chloropropanamido)éthyl)phosphonate (L-Norvalinyl- β-chloro-L-alanyl-D/L-fosfaline) 37

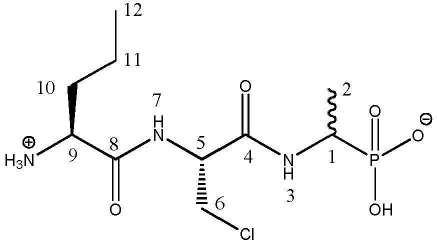

Du tert-Butyl ((2*S*)-1-(((2*R*)-3-chloro-1-((1-(diéthoxyphosphoryl)éthyl)amino)-1-oxopropan-2-yl)amino)-1-oxopentan-2-yl)carbamate (ester diéthylique de ^{t}Boc-L-norvalinyl- β -chloro-L-alanyl-D/L-fosfaline) **36** (2,0 mmol, 0,99 g), obtenu à l'étape 4.5. supra, a été dissous dans du HBr et de l'acide acétique (33 %) (3,0 mL). La solution a été agitée toute une nuit à température ambiante. On a ajouté de l'éther diéthylique sec (150 mL) et le mélange a été stocké à -20 °C toute une nuit. Le solvant a été décanté et le produit brut brun huileux a été trituré avec de l'éther diéthylique sec (5 x 60 mL). Le résidu hygroscopique orange-brun a été dissous dans du méthanol sec (5 mL), suivi par l'addition d'oxyde de propylène en excès. La solution a été filtrée et lavée avec de l'éther diéthylique pour donner un solide vert pâle qui a été filtré plus tard pour donner le produit final 37 sous forme de solide vert pâle (0,64 g, 1,94 mmol, 97 %) ; p.f. 175 °C (secondaire) [α]²¹_{D} -2,50° (c 1,0, H₂O+DIEA, 9,9:0,1) ; vₘₐₓ/cm⁻¹ 3294 (NH⁺), 2963 (CH), 1668 (C=O), 1645 (C=O), 1538 (flexion NH), 1132 (P-O), 1039 (P-O), 998 (P-O) ; ¹H RMN (300 MHz, D₂O) δ_{H} 1,01 (3H, t, *J*=9,0 Hz, CH₃-12), 1,30-1,37 (3H, m, CH₃-2), 1,44-1,54 (2H, m CH₃-11), 1,90-1,98 (2H, m, CH₂-10), 3,91-4,15 (4H, [m, CH₂-6], [m, CH-9], [m, CH-1]), 4,79 (1H, m, CH-5) ; ¹³C RMN (75 MHz, D₂O) δ_{C} 12,9 (CH₃-12), 15,7 (CH₃-2), 17,6 (CH₂-11), 33,0 (CH₂-10), 43,3 (CH₂-6), 53,1 (CH-1, CH-9), 55,0 (CH-5), 170,4 (C-4, C-8, quat) ; ³¹P-¹H_{decoup} RMN (121 MHz, CDCl₃) δ_{P} 18,5 ; SMHR (ionisation par nanopulvérisation) calculé pour (C₁₀H₂₀ClN₃O₅P)⁻ 328,0835, trouvé 328,0833.

### Exemple 5 : Procédé de synthèse du composé antimicrobien H selon l'invention (figure 2) : L-méthionyl-β-chloro-L-alanyl-D/L-fosfaline

La L-méthionyl-β-chloro-L-alanyl-D/L-fosfaline (L-Met- β -Cl-L-Ala-D/L-fosfaline) est représentée par la formule suivante :

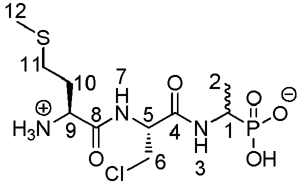

La synthèse de ce composé antimicrobien est détaillée ci-après.

### 5.1. Synthèse de l'intermédiaire réactionnel Tert-butyl((2R)-3-chloro-1-((1-(diéthoxyphosphoryl)éthyl)amino)-1-oxopropan-2-yl)carbamate (Ester diéthylique de ^{t}Boc-β-Cl-L-Ala-D/L-Fos)

L'ester diéthylique de *^{t}*Boc-(3-Cl-L-Ala-D/L-Fos est représenté par la formule suivante :

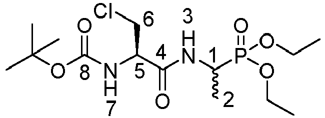

À une suspension de *^{t}*Boc-β-Cl-L-Ala-OH (7,8 mmol, 1,74 g) dans du THF sec (60 mL), on a ajouté de la N-méthylmorpholine (7,8 mmol, 0,90 mL) à -5 °C. On a ajouté lentement du chloroformiate d'isobutyle (7,8 mmol, 1,00 mL) et le mélange résultant a été agité à -5 °C pendant 1 heure. Au mélange agité, on a ajouté du 1-aminoéthylphosphonate de diéthyle (8,6 mmol, 1,57 g) dans du THF sec (20 mL) à -5 °C. Le mélange résultant a été agité sous azote à -5 °C pendant 30 minutes, puis à température ambiante toute une nuit. Le mélange a été filtré et le solvant a été éliminé sous vide pour donner un liquide jaune pâle, qui a été lavé avec 10 % poids/volume d'acide citrique (2 x 25 mL), 10 % poids/volume de carbonate de potassium (25 mL) et de l'eau (25 mL). Les couches organiques combinées ont été séchées sur sulfate de magnésium, filtrées et concentrées sous vide pour donner un liquide jaune pâle. Le liquide a été purifié par chromatographie sur colonne, en utilisant 100 % de DCM, puis en augmentant progressivement à 90:10 DCM/MeOH, pour donner le produit sous forme de sirop jaune composé de 2 diastéréoisomères, l'ester diéthylique de *^{t}*Boc-β-Cl-L-Ala-L-Fos et l'ester diéthylique de *^{t}*Boc-β-Cl-L -Ala-D-Fos (2,70 g, 7,0 mmol, 90 %) ; *v*ₘₐₓ/cm⁻¹ 3261 (NH), 1713 (C=O), 1670 (C=O), 1517 (flexion NH), 1225 (P=O), 1164 (P-O), 1020 (P-O), 970 (P-O) ; ¹H RMN (300 MHz, CDCl₃) δ_{H} 1,15-1,39 (18H, [s, C(C**H₃**)₃], [m, C**H**₃-2], [m, 2 x OCH₂C**H**₃]), 3,64-3,71 (1H, m, C**H_{a/b}**-6), 3,89-3,96 (1H, m, C**H_{a/b}**-6), 4,01-4,12 (4H, m, 2 x OC**H₂**CH₃), 4,42-4,49 (2H, [m, C**H**-1], [m, C**H**-5]), 5,40 (1H, d, *J*=3,0 Hz, N**H**-3 ou N**H**-7-A), 5,43 (1H, d, *J*=6,0 Hz, N**H**-3 ou N**H**-7-A), 7,00 (1H, d, *J*=9,0 Hz, N**H**-3 ou N**H**-7-A), 7,07 (1H, d, *J*=9,0 Hz, N**H**-3 ou N**H**-7-B) ; ¹³C RMN (75 MHz, CDCl₃) δ_{C} 15,6 (d, *J_{P-C}*=5,3 Hz, **C**H₃-2), 16,3 (d, *J_{P-C}*=2,3 Hz, OCH₂**C**H₃), 16,5 (d, *J_{P-C}*=2,3 Hz, OCH₂**C**H₃), 28,3 (C(**C**H₃)₃) 41,2 (d, *J_{P-C}*=157,5 Hz, **C**H-1-A), 41,3 (d, *J_{P-C}*=156,8 Hz, **C**H-1-B), 44,9 (**C**H₂-6-A), 45,0 (**C**H₂-6-B), 55,3 (**C**H-5), 62,5 (d, *J_{P-C}*=3,0 Hz, O**C**H₂CH₃-A), 62,6 (d, *J_{P-C}*=3,8 Hz, O**C**H₂CH₃-B), 62,9 (d, *J_{P-C}*=3,0 Hz, O**C**H₂CH₃-A), 63,0 (d, *J_{P-C}*=3,0 Hz, O**C**H₂CH₃-B), 80,7 (**C**(CH₃)₃), 154,9 (C=O-8), 168,3 (**C**=O-4) ; ³¹P-¹H_{decoup} RMN (121 MHz, CDCl₃) δ_{P} 24,8.

### 5.2. Synthèse de l'intermédiaire réactionnel Chlorure de (2R)-3-chloro-1-((1-(diéthoxyphosphoryl)éthyl)amino)-1-oxopropan-2-aminium (Chlorhydrate d'ester diéthylique de β-Cl-L-Ala-D/L-Fos)

Le chlorhydrate d'ester diéthylique de β-Cl-L-Ala-D/L-Fos est représenté par la formule suivante :

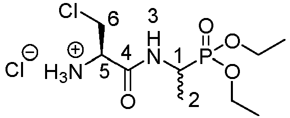

Une solution d'ester diéthylique de *^{t}*Boc-β-Cl-L-Ala-D/L-Fos (obtenu à l'étape 5.1. ; 6,7 mmol, 2,59 g) dans une solution 2 M HCl dans l'éther diéthylique (100 mL) a été agitée sous azote à température ambiante toute la nuit. Le mélange a ensuite été filtré et le solide hygroscopique blanc cassé a été lavé avec de l'éther diéthylique. Le solide a ensuite été séché toute une nuit dans un dessiccateur contenant de l'oxyde de phosphore (V) et trituré avec de l'essence pour donner le produit sous forme de solide vert pâle composé de 2 diastéréoisomères, le chlorhydrate d'ester diéthylique de β-Cl-L-Ala-L-Fos et le chlorhydrate d'ester diéthylique de β-Cl-L-Ala-D-Fos (1,51 g, 4,7 mmol, 70 %) ; p.f. 127 - 131 °C (décomp.) ; *v*ₘₐₓ/cm⁻¹ 3204 (NH), 1687 (C=O), 1562 (flexion NH), 1204 (P=O), 1010 (P-O), 961 (P-O) ; ¹H RMN (300 MHz, D₂O) δ_{H} 1,28 (3H, t, *J*=6,0 Hz, OCH₂C**H₃**), 1,29 (3H, t, *J*=6,0 Hz, OCH₂C**H₃**), 1,37 (3H, dd, *³J_{P-H}*=18,0 Hz, *³J_{H-H}*=6,0 Hz, C**H₃**-2), 3,92-4,04 (2H, m, C**H₂**-6), 4,07-4,21 (4H, m, 2 x OC**H₂**CH₃), 4,38-4,48 (2H, [m, C**H**-1], [m, C**H**-5]) ; ¹³C RMN (75 MHz, D₂O) δ_{C} 13,7 (**C**H₃-2), 14,0 (**C**H₃-2), 15,7 (OCH₂**C**H₃), 15,7 (OCH₂**C**H₃), 41,7 (d, *J_{P-C}*=158,3 Hz, **C**H-1), 42,0 (d, *J_{P-C}*=157,5 Hz, **C**H-1), 42,4 (**C**H₂-6), 53,7 (**C**H-5), 53,8 (**C**H-5), 64,3 (d, *J_{P-C}*=6**,**8 Hz, O**C**H₂CH₃), 64,5 (d, *J_{P-C}*=6**,**8 Hz, O**C**H₂CH₃-B), 165,7 (**C**=O-4-A), 165,8 (**C**=O-4-B) ; ³¹P-¹H_{decoup} RMN (121 MHz, CDCl₃) δ_{P}26,1.

### 5.3. Synthèse de l'intermédiaire réactionnel Tert-butyl((2S)-1-(((2R)-3-chloro-1-((1-(diéthoxyphosphoryl)éthyl)amino)-1-oxopropan-2-yl)amino)-4-(méthylthio)-1-oxobutan-2-yl)carbamate (Ester diéthylique de ^{t}Boc-L-Met-β-Cl-L-Ala-D/L-Fos)

L'ester diéthylique de *^{t}*Boc-L-Met-β-Cl-L-Ala-D/L-Fos est représenté par la formule suivante :

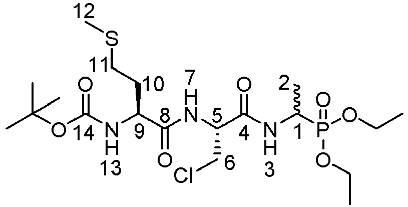

À une solution de tBoc-L-Met-OH (3,4 mmol, 0,85 g) dans du THF sec (60 mL), on a ajouté de la N-méthylmorpholine (3,4 mmol, 0,40 mL). La solution a été refroidie à -5 °C et on a ajouté, goutte à goutte, du chloroformiate d'isobutyle (3,4 mmol, 0,45 mL). Le mélange a été agité à -5 °C pendant 1 heure. Au mélange agité, on a ajouté, goutte à goutte, du chlorhydrate d'ester diéthylique de β-Cl-L-Ala-D/L-Fos obtenu à l'étape 5.2. (3,4 mmol, 1,10 g) dans du DCM sec (20 mL), qui avait été neutralisé avec de la *N*-méthylmorpholine (3,4 mmol, 0,40 mL) à -5 °C. Le mélange résultant a été agité sous azote à -5 °C pendant 30 minutes, puis toute une nuit à température ambiante. Le mélange a ensuite été filtré et concentré sous vide, puis lavé avec 10 % poids/volume d'acide citrique (2 x 25 mL), 10 % poids/volume de carbonate de potassium (25 mL), de l'eau (25 mL) et de la saumure (30 mL). La couche organique a été séchée sur MgSO₄, filtrée et le solvant a été éliminé sous vide pour donner un liquide jaune, qui a été purifié par chromatographie sur colonne [DCM/MeOH (95:5)] pour donner un liquide incolore. Une recristallisation à partir d'éther diéthylique/essence a donné le produit sous forme de solide blanc composé de 2 diastéréoisomères, l'ester diéthylique de *^{t}*Boc-L-Met-β-Cl-L-Ala-L-Fos et l'ester diéthylique de *^{t}*Boc-L-Met-β-Cl-L-Ala-D-Fos (0,88 g, 1,7 mmol, 50 %) ; p.f. 96 - 99 °C ; *v*ₘₐₓ/cm⁻¹ 3278 (NH), 1709 (C=O), 1639 (C=O), 1523 (flexion NH), 1228 (P=O), 1018 (P-O), 970 (P-O) ; ¹H RMN (300 MHz, CDCl₃) δ_{H} 1,17-1,36 (9H, [m, C**H₃**-2], [m, 2 x OCH₂C**H₃**]), 1,38 (9H, s, C(C**H₃**)₃), 1,87-2,07 (5H, [s, C**H₃**-12], [m, C**H₂**-10]), 2,48-2,54 (2H, m, C**H₂**-11), 3,71 (1H, dd, *J*=12,0 Hz, 6,0 Hz, C**H_{a/b}**-6), 3,88 (1H, dd, *J*=12,0 Hz, 6,0 Hz, C**H_{a/b}**-6), 3,99-4,13 (4H, m, 2 x OC**H₂**CH₃), 4,20 (1H, m, C**H**-9), 4,37-4,47 (1H, m, C**H**-1), 4,78-4,84 (1H, m, C**H-**5), 5,39 (1H, d, *J*=6,0 Hz, N**H**-13-A), 5,41 (1H, d, *J*=6,0 Hz, N**H**-13-B), 7,15 (1H, d, *J*=6,0 Hz, N**H**-7-A), 7,24 (1H, d, *J*=6,0 Hz, N**H**-7-B), 7,52 (1H, m, N**H**-3) ¹³C RMN (75 MHz, CDCl₃) δ_{C} 14,4 (**C**H₃-2 ou **C**H₃-12), 14,5 (**C**H₃-2 ou **C**H₃-12), 15,4 (OCH₂**C**H₃), 15,5 (OCH₂**C**H₃), 27,3 (**C**(CH₃)₃), 29,2 (**C**H₂-11-A), 29,3 (**C**H₂-11-B), 30,2 (**C**H₂-10-A), 30,4 (**C**H₂-10-B), 40,3 (d, *J_{P-C}*=159,0 Hz, CH-1), 43,5 (**C**H₂-6-A), 43,7 (**C**H₂-6-B), 52,7 (CH-5), 53,1 (CH-9), 61,6 (d, *J_{P-C}*=6,8 Hz, O**C**H₂CH₃-A), 61,7 (d, *J_{P-C}*=6,0 Hz, O**C**H₂CH₃-B), 62,0 (d, *J_{P-C}=*6**,**8 Hz, O**C**H₂CH₃-A), 62,1 (d, *J_{P-C}*=7,5 Hz, O**C**H₂CH₃-B), 79,6 (**C**(CH₃)₃), 154,8 (**C**=O-14), 166,7 (**C**=O-4-A), 166,8 (**C**=O-4-B), 170,7 (**C**=O-8-A), 170,8 (**C**=O-8-B) ; ³¹P-¹H_{decoup} RMN (121 MHz, CDCl₃) δ_{P} 24,8 ; CHN [Trouvé : C, 44,08 ; H, 7,47 ; N, 8,18. C₁₉H₃₇ClN₃O₇PS demande C, 44,06 ; H, 7,20 ; N, 8,11 %].

### 5.4. Synthèse du composé antimicrobien H (figure 2), à savoir le composé Hydrogéno(1-((R)-2-((S)-2-ammonio-4-(méthylthio)butanamido)-3-chloropropanamido)éthyl)phosphonate (L-Met-β-Cl-L-Ala-D/L-Fos)

Le composé antimicrobien L-Met-β-Cl-L-Ala-D/L-Fos est représenté par la formule suivante :

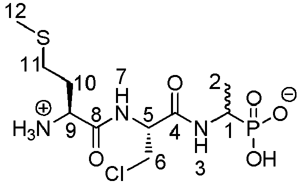

Une solution d'ester diéthylique de *^{t}*Boc-L-Met-β-Cl-L-Ala-D/L-Fos obtenu à l'étape 5.3. (1,4 mmol, 0,71 g) dans du bromure d'hydrogène/acide acétique glacial (33 %) (8,0 mL) a été agitée toute une nuit à température ambiante. On a ensuite ajouté de l'éther diéthylique sec (70 mL) et le mélange a été placé dans un congélateur toute une nuit. Le solvant a été décanté et le produit brut a été trituré avec de l'éther diéthylique sec (5 x 50 mL). Le produit brut brunâtre-orange a été dissous dans du méthanol (5 mL) et on a ajouté de l'oxyde de propylène en excès. Le mélange a été filtré et lavé avec de l'éther diéthylique sec pour donner un solide vert, qui a été séché dans un dessiccateur contenant de l'oxyde de phosphore (V) et recristallisé à partir d'eau chaude/éthanol pour donner le produit sous forme de solide vert pâle composé de 2 diastéréoisomères, L-Met-β-Cl-L-Ala-L-Fos et L-Met-β-Cl-L-Ala-D-Fos (0,17 g, 0,48 mmol, 35 %) ; p.f. 175 - 179 °C (décomp.) ; *v*ₘₐₓ/cm⁻¹ 3264 (NH⁺), 2829 (OH large), 1641 (C=O), 1546 (flexion NH), 1149 (P-O), 1041 (P-O), 921 (P-O) ; ¹H RMN (300 MHz, D₂O) δ_{H} 1,10-1,50 (3H, m, C**H₃**-2), 2,08-2,32 (5H, [m, C**H₃**-12], [m, C**H₂**-10]) 2,61 (2H, C**H₂**-11), 3,37-4,16 (4H, [m, C**H₂**-6], [m, C**H**-1], [m, C**H**-9]), 4,48 (1H, m, C**H**-5) ; ¹³C RMN (75 MHz, D₂O) δ_{C} 14,0 (**C**H₃-12), 15,5 (**C**H₃-2), 28,2 (**C**H₂-11), 30,0 (**C**H₂-10), 43,3 (**C**H₂-6), 44,8 (**C**H-1), 52,3 (**C**H-9), 55,0 (**C**H-5), 169,4 (**C**=O-4 et **C**=O-8) ; ³¹P-¹H_{decoup} RMN (121 MHz, CDCl₃) δ_{P} 18,7.

### Exemple 6 : Evaluation de l'activité antibactérienne de la L-Norvaliny(β-chloro-alanyl-D/L-fosfaline (composé G ; cf. figure 2) et de la L-Méthionyl-β-chloro-alanyl-D/L-fosfaline (composé H ; cf. figure 2) synthétisées respectivement selon les Exemples 4 et 5

### 6.1. Introduction

Les concentrations minimales inhibitrices des composés G et H pour 12 souches de bactéries à Gram négatif et 6 souches de bactéries à Gram positif ont été déterminées après 22 heures d'incubation en utilisant un procédé de dilution en gélose comme décrit dans l'exemple 2 (cf. notamment section « 2.2 Matériels et méthodes »).

### 6.2 Résultats

Les résultats obtenus sont présentés dans le tableau 4 *infra*

**Tableau 4 : Concentrations minimales inhibitrices des composés antimicrobiens G et H vis-à-vis de différentes espèces de bactéries**

| Espèce | Référence de la souche | Concentration minimale inhibitrice (CMI) du composé G (µg/mL) | Concentration minimale inhibitrice (CMI) du composé H (µg/mL) |
|---|---|---|---|
| *Acinetobacter baumannii* | ATCC 19606 | >8 | >8 |
| *Burkholderia cepacia* | ATCC 25416 | >8 | >8 |
| *Enterobacter cloacae* | NCTC 11936 | 4 | 4 |
| *Escherichia coli* | NCTC 10418 | 1 | 0.5 |
| *Escherichia coli* | NCTC 12241 | 0,5 | 0.5 |
| *Klebsiella pneumoniae* | NCTC 9528 | 0,5 | 0.5 |
| *Providencia rettgeri* | NCTC 7475 | >8 | >8 |
| *Pseudomonas aeruginosa* | NCTC 10662 | >8 | >8 |
| *Salmonella typhimurium* | NCTC 74 | >8 | >8 |
| *Salmonella enteritidis* | NCTC 6676 | >8 | >8 |
| *Serratia marcescens* | NCTC 10211 | 0,5 | 0.25 |
| *Yersinia enterocolitica* | NCTC 11176 | 0,25 | 0.25 |
| *Enterococcus faecalis* | NCTC 775 | 0,063 | 0.032 |
| *Enterococcus faecium* | NCTC 7171 | 1 | 1 |
| *Listeria monocytogenes* | NCTC 11994 | >8 | >8 |
| *Staphylococci epidermidis* | NCTC 11047 | 2 | 1 |
| *Staphylococcus aureus* | NCTC 6571 | 4 | 2 |
| *Staphylococcus aureus* (SARM) | NCTC 11939 | >8 | >8 |

D'après les données présentées dans le tableau 4 supra, il apparaît clairement que les composés G et H sont fortement inhibiteurs vis-à-vis des souches testées de certaines espèces de bactéries à Gram négatif et de certaines espèces de bactéries à Gram positif, notamment concernant *Escherichia coli, Klebsiella pneumoniae, Serratia marcescens, Yersinia enterocolitica, Enterococcus faecalis,* et moins inhibiteurs par rapport à d'autres espèces de bactéries à Gram négatif et à Gram positif, notamment en ce qui concerne *Acinetobacter baumannii, Burkholderia cepacia, Pseudomonas aeruginosa, Salmonella typhimurium, Salmonella enteritidis, Listeria monocytogenes.*

### 6.3 Conclusion

Les différences très significatives en matière de concentration inhibitrice observées entre différentes espèces de bactéries font des composés G et H des composés particulièrement bien adaptés pour être incorporés dans des milieux réactionnels permettant la recherche et/ou l'isolement sélective/sélectif de certaines espèces de bactéries dans des échantillons biologiques, et notamment la recherche sélective de :
- bactéries à Gram négatif (bactéries cibles à Gram négatif) telles que *Acinetobacter baumannii, Burkholderia cepacia, Pseudomonas aeruginosa, Salmonella typhimurium, Salmonella enteritidis ;* ou
- de bactéries à Gram positif (bactéries cibles à Gram positif) telles que *Listeria monocytogenes.*

### Exemple 7 : Procédé de synthèse du composé antimicrobien I selon l'invention (figure 2) : L-norvalinyl-L-alanyl-D/L-fosfaline

La L-norvalinyl-L-alanyl-D/L-fosfaline (L-Nva-L-Ala-D/L-fosfaline) est représentée par la formule suivante :

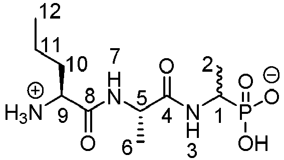

La synthèse de ce compose antimicrobien est détailée ci-après.

### 7.1. Synthèse de l'intermédiaire réactionnel (S)-Benzyl 2-((S)-2-((tert-butoxycarbonyl)amino)pentanamido)propanoate (^{t}Boc-L-Nva-L-Ala-OBzl)

Le composé *^{t}*Boc-L-Nva-L-Ala-OBzl est représenté par la formule suivante :

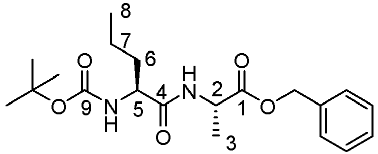

À une solution de *^{t}*Boc-L-Nva-OH (10,0 mmol, 2,17 g) dans du THF sec (60 mL), on a ajouté de la N-méthylmorpholine (15,0 mmol, 1,65 mL). La solution a été refroidie à -5 °C et on a ajouté, goutte à goutte, du chloroformiate d'isobutyle (15,0 mmol, 1,95 mL). Le mélange a été agité à -5 °C pendant 1 heure. Au mélange agité, on a ajouté, goutte à goutte, du sel *p*-toluènesulfonate d'ester benzylique de L-alanine (10,0 mmol, 3,52 g) dans du DCM sec (30 mL), qui avait été neutralisé avec de la diisopropyléthylamine (15,0 mmol, 2,60 mL) à -5 °C. La solution résultante a été agitée sous azote à -5 °C pendant 30 minutes, puis toute une nuit à température ambiante. La solution a été filtrée et concentrée sous vide, puis lavée avec 10 % poids/volume d'acide citrique (2 x 25 mL), 10 % poids/volume de carbonate de potassium (25 mL) et de l'eau (25 mL). La couche organique a été séchée sur MgSO₄, filtrée et concentrée sous vide pour donner un liquide hygroscopique jaune, qui a été purifié par chromatographie sur colonne [40-60 essence/acétate d'éthyle (7:3)] pour donner le produit sous forme de solide blanc cassé (2,40 g, 6,3 mmol, 63 %) ; p.f. 60 - 63 °C ; *v*ₘₐₓ/cm⁻¹ 3299 (NH), 1743 (C=O), 1655 (C=O), 1527 (flexion NH), 1245 (C-O), 1162 (C-O) ; ¹H RMN (300 MHz, CDCl₃) δ_{H} 0,83 (3H, t, *J*=9,0 Hz, C**H₃**-8), 1,25-1,36 (14H, [d, *J*=6,0 Hz, C**H₃**-3], [m, C**H₂**-7], [s, C(C**H₃**)₃]), 1,42-1,54 (1H, m, C**H_{a/b}**-6), 1,64-1,73 (1H, m, C**H_{a/b}**-6), 4,02 (1H, m, C**H**-5), 4,54 (1H, pentet, *J*=6,0 Hz, C**H**-2), 4,96 (1H, d, *J*=9,0 Hz, N**H**CO₂), 5,07 (1H, d, *J*=12,0 Hz, OC**H_{a/b}**Ar), 5,12 (1H, d, *J*=12,0 Hz, O**CH_{a/b}**Ar), 6,56 (1H, d, *J*=6,0 Hz, N**H**CO), 7,27 (5H, m, 5xC**H_{Ar}**) ; ¹³C RMN (75 MHz, CDCl₃) δ_{C} 12,7 (**C**H₃-8), 17,3 (**C**H₃-3), 17,8 (**C**H₂-7), 27,3 (C(**C**H₃)₃), 33,7 (**C**H₂-6), 47,1 (**C**H-2), 53,4 (**C**H-5), 66,1(O**C**H2_{A}r), 79,0 (C(**C**H₃)₃), 127,1-127,6 (5x**C**H_{Ar}), 134,3 (**C**H_{Ar} quat.), 154,6 (**C**=O-9), 170,8 (**C**=O-4), 171,5 (**C**=O-1); CHN [Trouvé : C, 63,75 ; H, 8,37 ; N, 7,86. C₂ₒH₃₀N₂O₅ demande C, 63,47 ; H, 7,99 ; N, 7,40 %] ; SMHR (ionisation par nanopulvérisation) calculé pour (C₂₀H₃₁N₂O₅)⁺379,2227, trouvé 379,2222.

### 7.2. Synthèse de l'intermédiaire réactionnel Acide (S)-2-((S)-2-(((tert-butoxycarbonyl)amino)pentanamido)propanoïque (^{t}Boc-L-Nva-L-Ala-OH)

Le composé *^{t}*Boc-L-Nva-L-Ala-OH est représenté par la formule suivante :

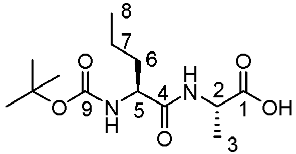

Du *^{t}*Boc-L-Nva-L-Ala-OBzl obtenu à l'étape 7.1. (6,0 mmol, 2,27 g) a été dissous dans du méthanol (60 mL) et hydrogéné en présence de 5 % de palladium sur charbon (0,23 g) à une pression de 3,5 bar de H₂ à température ambiante toute une nuit. Le catalyseur a été éliminé par filtrage au moyen de Célite et lavé avec du méthanol. La solution a été concentrée sous vide pour donner le produit sous forme de solide blanc (1,66 g, 5,7 mmol, 96.0 %) ; p.f. 53 - 58 °C (décomp.) ; *v*ₘₐₓ/cm⁻¹ 3500-2500 (br, OH), 3300 (NH), 2961 (NH), 1688 (C=O), 1655 (C=O), 1522 (flexion NH), 1245 (C-O), 1164 (C-O); ¹H RMN (300 MHz, CDCl₃) δ_{H} 0,85 (3H, t, *J*=9,0 Hz, C**H**₃-8), 1,27-1,39 (14H, [m, C**H**₃-3], [m, C**H**₂-7], [s, C(C**H**₃)₃]), 1,48-1,53 (1H, m, C**H**_{a/b}-6), 1,67-1,71 (1H, m, C**H**_{a/b}-6), 4,10 (1H, m, C**H**-5), 4,50 (1H, m, C**H**-2), 5,27 (1H, m, N**H**CO₂), 6,93 (1H, m, N**H**CO), 8,87 (1H, br, O**H**) ; ¹³C RMN (75 MHz, CDCl₃) δ_{C} 13,7 (**C**H₃-8), 18,0 (**C**H₃-3), 18,8 (**C**H₂-7), 28,3 (C(**C**H₃)₃), 34,5 (**C**H₂-6), 48,1 (**C**H-2), 54,3 (**C**H-5), 80,4 (**C**(CH₃)₃), 156,0 (**C**=O-9), 172,5 (**C**=O-4), 175,5 (**C**=O-1); CHN [Trouvé : C, 54,18 ; H, 8,78 ; N, 9,62. C₁₃H₂₄N₂O₅ demande C, 54,15 ; H, 8,39 ; N, 9,72 %]; SMHR (ionisation par nanopulvérisation) calculé pour (C₁₃H₂₅N₂O₅)⁺289,1758, trouvé 289,1758.

### 7.3. Synthèse de l'intermédiaire réactionnel tert-Butyl((2S)-1-(((2S)-1-((1-(diethoxyphosphoryl)éthyl)amino)-1-oxopropan-2-yl)amino)-1-oxopentan-2-yl)carbamate (Ester diéthylique de ^{t}Boc-L-Nva-L-Ala-D/L-Fos)

L'ester diéthylique de *^{t}*Boc-L-Nva-L-Ala-D/L-Fos est représenté par la formule suivante :

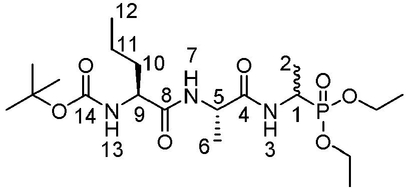

À une solution de *^{t}*Boc-L-Nva-L-Ala-OH (5,0 mmol, 1,45 g ; obtenu à partir de l'étape 7.2.) dans du THF sec (50 mL), on a ajouté de la N-méthylmorpholine (5,3 mmol, 0,58 mL) à -5 °C. On a ajouté lentement du chloroformiate d'isobutyle (5,3 mmol, 0,70 mL) et le mélange résultant a été agité à -5 °C pendant 1 heure. Au mélange agité, on a ajouté du 1-aminoéthylphosphonate de diéthyle (4,8 mmol, 0,87 g ; dont la synthèse est décrite dans l'exemple 4.4 et représentée en figure 6) dans du THF sec (15 mL) à -5 °C. Le mélange résultant a été agité sous azote à -5 °C pendant 30 minutes, puis à température ambiante toute la nuit. Le mélange a été filtré et le solvant a été éliminé sous vide pour donner un solide blanc, qui a été redissous dans du DCM (50 mL) et lavé avec 10 % poids/volume d'acide citrique (2 x 25 mL), 10 % poids/volume de carbonate de potassium (25 mL) et de l'eau (25 mL). La couche organique a été séchée sur sulfate de magnésium, filtrée et concentrée sous vide pour donner un solide blanc, qui a été purifié par chromatographie sur colonne à l'aide de 100 % de DCM, en augmentant à 90:10 DCM/méthanol, pour donner un solide blanc composé de 2 diastéréoisomères, l'ester diéthylique de *^{t}*Boc-L-Nva-L-Ala-L-Fos et l'ester diéthylique de *^{t}*Boc-L-Nva-L-Ala-D-Fos (1,70 g, 3,8 mmol, 78 %) ; p.f. 165 - 168 °C ; *v*ₘₐₓ/cm⁻¹ 3267 (NH), 1708 (C=O), 1638 (C=O), 1537 (flexion NH), 1227 (P=O), 1019 (P-O), 966 (P-O) ; ¹H RMN (300 MHz, CDCl₃) δ_{H} 0,85 (3H, t, *J*=9,0 Hz, C**H**₃-12), 1,18-1,34 (14H, [m, 2 x CH₂C**H**₃], [C**H**₃-2], [m, C**H**₃-6], [m, C**H**₂-11]), 1,37 (s, C(C**H**₃)₃), 1,47-1,54 (1H, m, C**H_{a/b}**-10), 1,65-1,73 (1H, m, C**H**_{a/b}-10), 3,98-4,12 (5H, [m, 2 x OC**H**₂CH₃], [m, C**H**₃-5 ou C**H**₃-9]), 4,33-4,44 (1H, m, C**H**-1), 4,48-4,54 (1H, m, C**H**-5 ou C**H**-9), 5,18-5,23 (1H, d, *J*=6,0 Hz, N**H**-7 ou N**H**-13), 5,18-5,23 (1H, d, *J*=6,0 Hz, N**H**-7 ou N**H**-13), 6,77-6,88 (1H, d, *J*=6,0 Hz, N**H**-7 ou N**H**-13), 6,77-6,88 (1H, d, *J*=6,0 Hz, N**H**-7 ou N**H**-13), 7,13-7,24 (1H, d, *J*=9,0 Hz, 9,0 Hz, N**H**-3), 7,13-7,24 (1H, d, *J*=9,0 Hz, 9,0 Hz, N**H-**3); ¹³C RMN (75 MHz, CDCl₃) δ_{C} 12,7 (**C**H₃-12), 14,5 (**C**H₃-2, d, *J_{P-C}* =6,0 Hz), 15,3 (OCH₂**C**H₃-A), 15,4 (OCH₂**C**H₃-A), 15,5 (OCH₂**C**H₃-B), 15,6 (OCH₂**C**H₃-B), 17,6 (**C**H₃-6-A), 17,7 (**C**H₃-6-B), 17,8 (**C**H₂-11-A), 17,9 (**C**H₂-11-B), 27,3 (C(**C**H₃)₃), 33,8 (**C**H₂-10-A), 33,9 (**C**H₂-10-B), 39,9 (**C**H-1-A, d, *J_{P-C}*=157,5 Hz), 40,0 (**C**H-1-B, d, *J_{P-C}*=156,8 Hz), 47,7 (**C**H-5 ou **C**H-9-A), 47,9 (**C**H-5 ou **C**H-9-B), 53,5 (**C**H-5 ou **C**H-9-A), 53,5 (**C**H-5 ou **C**H-9-B), 61,4-62,0 (2 x O**C**H₂CH₃-A et B, 4 x d, *J*=7,5 Hz, 6,8 Hz, 7,5 Hz, 7,5 Hz), 78,9 (**C**(CH₃)₃), 154,7 (C=O-14), 170,6 (**C**=O-4 ou **C**=O-8-A), 170,7 (**C**=O-4 ou **C**=O-8-B), 171,0 (**C**=O-4 ou **C**=O-8-A), 171,1 (**C**=O-4 ou **C**=O-8-B) ; ³¹P-¹H_{decoup} RMN (121 MHz, CDCl₃) δ_{P} 24,8 ; CHN [Trouvé : C, 50,74 ; H, 8,55 ; N, 9,51. C₁₉H₃₈N₃O₇P demande C, 50,54 ; H, 8,48 ; N, 9,31 %] ; SMHR (ionisation par nanopulvérisation) calculé pour (C₁₉H₃₉N₃O₇P)⁺452,2520, trouvé 452,2518.

### 7.4. Synthèse du composé I, à savoir du composé Hydrogéno(1-((S)-2-((S)-2-ammoniopentanamido)propanamido)éthyl)phosphonate (L-Nva-L-Ala-D/L-Fos)

Tel qu'indiqué précédemment, le composé L-Nva-L-Ala-D/L-Fos est représenté par la formule suivante :

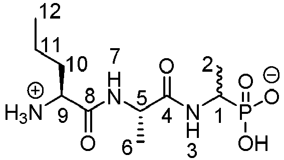

L'ester diéthylique de *^{t}*Boc-L-Nva-L-Ala-D/L-Fos obtenu à l'étape 7.3. (1,6 mmol, 0,72 g) a été dissous dans de l'acide acétique glacial (30 mL) et on a ajouté du bromure d'hydrogène/acide acétique (33 %) (25 mL). La solution a été agitée toute la nuit à température ambiante. On a ajouté de l'éther diéthylique sec (150 mL) et le mélange a été placé dans un congélateur toute une nuit. Le solvant a été décanté et le produit brut a été trituré avec de l'éther diéthylique sec (5 x 100 mL). Le produit brut jaunâtre-orange a été dissous dans du méthanol (5 mL) et on a ajouté de l'oxyde de propylène en excès. La solution a été filtrée et lavée avec de l'éther diéthylique pour donner un solide vert pâle, qui a été recristallisé à partir d'eau chaude/acétone pour donner le produit sous forme de solide vert pâle composé de 2 diastéréoisomères, L-Nva-L-Ala-L-Fos et L-Nva-L-Ala-D-Fos (0,22 g, 0,75 mmol, 47 %) ; p.f. 200 - 210 °C (décomp.) ; *v*ₘₐₓ/cm⁻¹ 3280 (NH⁺), 1643 (C=O), 1552 (flexion NH), 1149 (P-O), 1037 (P-O), 922 (P-O); ¹H RMN (300 MHz, D₂O) δ_{H} 0,96 (3H, t, C**H**₃-12), 1,29 (3H, m, C**H**₃-6) 1,42-1,40 (5H, [m, C**H**₃-2], [m, C**H**₂-11]), 1,88-1,86 (2H, m, C**H**₂-10), 4,02-4,00 (2H, [m, C**H**-5], [m, C**H**-9]), 4,34-4,39 (1H, m, C**H**-1); ¹³C RMN (75 MHz, D₂O) δ_{C} 13,4 (**C**H₃-12), 16,0 (**C**H₃-2), 17,1 (**C**H₃-6), 17,2 (**C**H₃-6), 18,1 (**C**H₂-11), 33,5 (**C**H₂-10), 50,7 (d, *J*_{P-C}=22,5 Hz, **C**H-1), 53,5 (**C**H-5 ou **C**H-9), 170,4 (**C**=O-8), 174,7 (**C**=O-4) ; SMHR (ionisation par nanopulvérisation) calculé pour (C₁₀H₂₃N₃O₅P)⁺ 296,1370, trouvé 296,1373 ; ³¹P-¹H_{decoup} RMN (121 MHz, CDCl₃) δ_{P} 18,5 ; CHN [Trouvé : C, 37,61 ; H, 7,51 ; N, 12,91. C₁₀H₂₂N₃O₅P·1,4H₂O demande C, 37,48 ; H, 7,80 ; N, 13,11 %].

### Exemple 8 : Procédé de synthèse du composé antimicrobien J (figure 2) : L-méthionyl-L-alanyl-D/L fosfaline

Le composé L-méthionyl-L-alanyl-D/L fosfaline est représenté par la formule suivante :

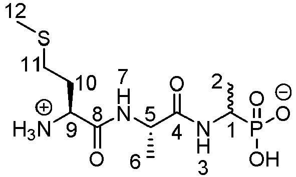

La synthèse de ce composé antimicrobien est détaillée ci-après.

### 8.1. Synthèse de l'intermédiaire réactionnel Tert-butyl ((S)-1-(((R)-1-(diéthoxyphosphoryl)éthyl)amino)-1-oxopropan-2-yl)carbamate (Ester diéthylique de ^{t}Boc-L-Ala-D/L-Fos)

L'ester diéthylique de *^{t}*Boc-L-Ala-D/L-Fos est représenté par la formule suivante :

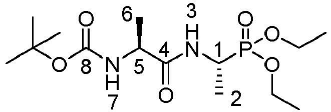

À une solution de *^{t}*Boc-L-Ala-OH (10,0 mmol, 1,90 g) dans du THF sec (60 mL), on a ajouté de la N-méthylmorpholine (15,0 mmol, 1,65 mL) à -5 °C. Du chloroformiate d'isobutyle (15,0 mmol, 1,95 mL) et le mélange résultant ont été agités à -5 °C pendant 1 heure. Au mélange agité, on a ajouté du 1-aminoéthylphosphonate de diéthyle (10,0 mmol, 1,84 g) dans du THF sec (20 mL) à -5 °C. Le mélange résultant a été agité sous azote à -5 °C pendant 30 minutes, puis à température ambiante toute la nuit. La solution a été filtrée et concentrée sous vide pour donner un sirop jaune pâle, qui a été redissous dans du DCM (60 mL) et lavé avec 10 % poids/volume d'acide citrique (2 x 25 mL), 10 % poids/volume de carbonate de potassium (25 mL) et de l'eau (25 mL). Les couches organiques combinées ont été séchées sur sulfate de magnésium, filtrées et concentrées sous vide pour donner un sirop jaune pâle, qui a été purifié par chromatographie sur colonne, en utilisant initialement 100 % de DCM et en augmentant à 95:5 DCM/méthanol, pour donner le produit sous forme de solide blanc cassé composé de 2 diastéréoisomères, l'ester diéthylique de *^{t}*Boc-L-Ala-L-Fos et l'ester diéthylique de *^{t}*Boc-L-Ala-D-Fos (2,49 g, 7,1 mmol, 71 %) ; p.f. 102 - 105 °C ; *v*ₘₐₓ/cm⁻¹ 3280 (NH), 1710 (C=O), 1652 (C=O), 1556 (flexion NH), 1229 (P=O), 1173 (P-O), 1013 (P-O), 973 (P-O) ; ¹H RMN (300 MHz, CDCl₃) δ_{H} 1,23-1,44 (21H, [s, C(C**H**₃)₃], [m, C**H**₃-2], [m, C**H**₃-6], [m, 2 x OCH₂C**H**₃]), 4,06-4,23 (5H, [m, 2 x OC**H**₂CH₃], [m, C**H**-5]), 4,40-4,52 (1H, m, C**H**-1), 5,11-5,15 (1H, 2 x d, *J*=1,5 Hz, 1,5 Hz, NH-7), 6,70-6,78 (1H, 2 x d, *J*=2,3 Hz, 2,3 Hz, N**H**-3) ; ¹³C RMN (75 MHz, CDCl₃) δ_{C} 15,6 (**C**H₃-2), 16,3 (d, *J*=3,0 Hz, OCH₂**C**H₃), 16,4 (d, *J*=2,3 Hz, OCH₂**C**H₃), 16,4 (d, *J*=3,8 Hz, OCH₂**C**H₃), 16,5 (d, *J*=1,5 Hz, OCH₂**C**H₃), 18,4 (**C**H₃-6), 28,3 (C(**C**H₃)₃) 40,8 (d, *J_{P-C}*=156,8 Hz, **C**H-1), 41,0 (d, *J_{P-C}*=156,8 Hz, **C**H-1), 50,0 (**C**H-5), 62,4-62,8 (4 x d, *J_{P-C}*=6,8 Hz, 6,8 Hz, 6,8 Hz, 6,8 Hz, 2 x OCH₂**C**H₃), 80,0 (**C**(CH₃)₃), 155,2 (C=O-8), 172,1 (**C**=O-4) ; CHN [Trouvé : C, 48,22 ; H, 8,58 ; N, 7,87. C₁₄H₂₉N₂O₆P demande C, 47,72 ; H, 8,30 ; N, 7,95 %].

### 8.2. Synthèse de l'intermédiaire réactionnel Chlorure de (S)-1-(((R)-1-(diéthoxyphosphoryl)éthyl)amino)-1-oxopropan-2-aminium (Chlorhydrate d'ester diéthylique de L-Ala-D/L-Fos)

Le chlorhydrate d'ester diéthylique de L-Ala-D/L-Fos est représenté par la formule suivante :

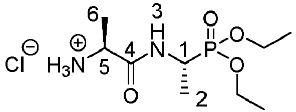

Une solution d'ester diéthylique de *^{t}*Boc-L-Ala-D/L-Fos (obtenu à l'étape 8.1. ; 6,0 mmol, 2,13 g) dans 2 M HCl dans de l'éther diéthylique (100 mL) a été agitée sous azote à température ambiante toute la nuit. Le solide résultant a été recueilli par filtration et lavé avec de l'éther diéthylique sec. Le solide hygroscopique blanc cassé a été séché toute une nuit dans un dessiccateur contenant de l'oxyde de phosphore (V) et lavé avec de l'essence pour donner le produit sous forme de solide vert pâle composé de 2 diastéréoisomères, le chlorhydrate d'ester diéthylique de L-Ala-L-Fos et le chlorhydrate d'ester diéthylique de L-Ala-D-Fos (1,46 g, 5,1 mmol, 84 %) ; p.f. 102 - 105 °C ; *v*ₘₐₓ/cm⁻¹ 2986 (NH⁺), 1673 (C=O), 1555 (flexion NH), 1017 (P-O), 950 (P-O) ; ¹H RMN (300 MHz, d₄-CH₃OH) δ_{H} 1,29-1,44 (9H, [m, 2 x OCH₂C**H**₃], [m, C**H**₃-2], 1,51 (3H, d, *J*=6,0 Hz, C**H**₃-6), 3,90-3,98 (1H, m, C**H**-5), 4,08-4,22 (4H, m, 2 x OCH₂**C**H₃), 4,28-4,47 (1H, m, C**H**-1); ; ¹³C RMN (75 MHz, d₄-CH₃OH) δ_{C} 13,7 (**C**H₃-2-A), 14,0 (**C**H₃-2-B), 15,4 (2 x OCH₂**C**H₃), 16,3 (**C**H₃-6), 41,1 (d, *J*_{P-C}=158,3 Hz, **C**H-1), 41,4 (d, *J_{P-C}*=158,3 Hz, **C**H-1), 48,8 (**C**H-5), 48,9 (C**H**-5), 62,7-63,0 (2 x OCH₂**C**H₃), 169,0 (**C**=O-4).

### 8.3. Synthèse de l'intermédiaire réactionnel Tert-butyl((2S)-1-(((2S)-1-((1-(diéthoxyphosphoryl)éthyl)amino)-1-oxopropan-2-yl)amino-4-(méthylthio)-1-oxobutan-2-yl)carbamate (Ester diéthylique de ^{t}Boc-L-Met-L-Ala-D/L-Fos)

Le composé ester diéthylique de *^{t}*Boc-L-Met-L-Ala-D/L-Fos est représenté par la formule suivante :

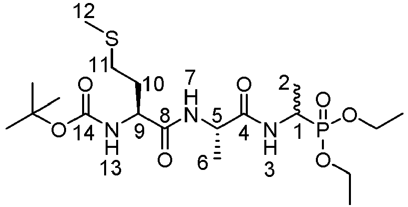

À une solution de *^{t}*Boc-L-Met-OH (3,4 mmol, 0,88 g) dans du THF sec (50 mL), on a ajouté de la N-méthylmorpholine (5,1 mmol, 0,60 mL). La solution a été refroidie à -5 °C et on a ajouté goutte à goutte du chloroformiate d'isobutyle (5,1 mmol, 0,70 mL). Le mélange a été agité à -5 °C pendant 1 heure. À la solution agitée, on a ajouté goutte à goutte du chlorhydrate d'ester diéthylique de L-Ala-D/L-Fos obtenu à l'étape 8.2. (3,4 mmol, 0,97 g) dans du THF sec (15 mL), qui avait été neutralisé avec de la diisopropyléthylamine (5,1 mmol, 1,00 mL) à -5 °C. La solution résultante a été agitée sous azote à -5 °C pendant 30 minutes, puis toute une nuit à température ambiante. La solution a été filtrée et concentrée sous vide, puis lavée avec 10 % poids/volume d'acide citrique (2 x 25 mL), 10 % poids/volume de carbonate de potassium (25 mL) et de l'eau (25 mL). La couche organique a été séchée sur MgSO₄, filtrée et concentrée sous vide pour donner un solide jaune, qui a été purifié par chromatographie sur colonne [DCM/MeOH (95:5)] pour donner le produit sous forme de solide blanc cassé composé de 2 diastéréoisomères, l'ester diéthylique de *^{t}*Boc-L-Met-L-Ala-L-Fos et l'ester diéthylique de *^{t}*Boc-L-Met-L-Ala-D-Fos (0,53 g, 1,1 mmol, 32 %) ; p.f. 172 - 176 °C ; *v*ₘₐₓ/cm⁻¹ 3272 (NH), 1708 (C=O), 1637 (C=O), 1530 (flexion NH), 1226 (P=O), 1165 (P-O), 1020 (P-O), 966 (P-O) ; ¹H RMN (300 MHz, CDCl₃) δ_{H} 1,16-1,36 (12H, [m, C**H**₃-2], [m, C**H**₃-6], [m, 2 x OCH₂C**H**₃]), 1,36 (9H, s, C(C**H**₃)₃), 1,82-2,01 (2H, m, C**H**₂-10), 2,04 (3H, s, C**H**₃-12), 2,49 (2H, t, *J*=9,0 Hz, C**H**₂-11 ), 4,00-4,12 (4H, m, 2 x OCH₂**C**H₃), 4,21 (1H, m, C**H**-9), 4,33-4,43 (1H, m, C**H**-1), 4,45-4,53 (1H, m, C**H**-5), 5,40 (1H, d, *J*=9,0 Hz, N**H**-13-A), 5,44 (1H, d, *J*=6,0 Hz, N**H**-13-B), 6,85 (1H, d, *J*=6,0 Hz, N**H**-7-A), 6,92 (1H, d, *J*=6,0 Hz, N**H**-7-B), 7,07 (1H, d, *J*=9,0 Hz, N**H**-3-A), 7,16 (1H, d, *J*=9,0 Hz, N**H**-3-B); ; ¹³C RMN (75 MHz, CDCl₃) δ_{C} 14,3 (**C**H₃-2-A), 14,3 (**C**H₃-2-B), 15,4-15,5 (2 x OCH₂**C**H₃), 17,7 (**C**H₃-6), 27,3 (C(**C**H₃)₃), 29,2 (**C**H₂-11-A), 29,3 (**C**H₂-11-B), 30,8 (**C**H₂-10-A), 30,8 (**C**H₂-10-B), 39,9 (d, *J_{P-C}*=156,8 Hz, **C**H-1-A), 40,0 (d, *J_{P-C}*=156,8 Hz, **C**H-1-B), 47,9 (**C**H-5-A), 48,0 (**C**H-5-B), 52,6 (**C**H-9), 61,5 (d, *J*_{P-C}=4,5 Hz, O**C**H₂CH₃-A), 61,6 (d, *J*_{P-C}=4,5 Hz, O**C**H₂CH₃-B), 61,7 (d, *J_{P-C}*=6,8 Hz, O**C**H₂CH₃-A), 61,9 (d, *J_{P-C}*=6,8 Hz, O**C**H₂CH₃-B), 79,1 (**C**(CH₃)₃), 154,6 (**C**=O-14), 170,3 (**C**=O-4 ou **C**=O-8-A), 170,4 (**C**=O-4 ou **C**=O-8-B), 170,5 (**C**=O-4 ou **C**=O-8-A), 170,6 (**C**=O-4 ou **C**=O-8-B).

### 8.4. Synthèse du composé J selon l'invention, à savoir le composé Hydrogéno(1-((S)-2-((S)-2-ammonio-4-(méthylthio)butanamido)propanamido)éthyl)phosphonate (L-méthionyl-L-alanyl-D/L fosfaline)

Tel qu'indiqué précédemment, le composé L-méthionyl-L-alanyl-D/L-fosfaline est représenté par la formule suivante :

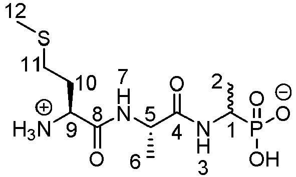

Une solution d'ester diéthylique de *^{t}*Boc-L-Met-L-Ala-D/L-Fos (obtenu à l'étape 8.3 ; 0,9 mmol, 0,43 g) dans du bromure d'hydrogène/acide acétique glacial (33 %) (10 mL) a été agitée toute une nuit à température ambiante. On a ajouté de l'éther diéthylique sec (150 mL) et le mélange a été placé dans un congélateur toute une nuit. Le solvant a été décanté et le produit brut a été trituré avec de l'éther diéthylique sec (5 x 100 mL). Le solide brut orange brunâtre a été dissous dans du méthanol (5 mL), suivi par l'addition d'oxyde de propylène en excès. La solution a été filtrée et lavée avec de l'éther diéthylique pour donner un solide vert, qui a été recristallisé à partir d'éthanol chaud et séché encore dans un dessiccateur contenant de l'oxyde de phosphore (V) pour donner le produit sous forme de solide vert pâle composé de 2 diastéréoisomères, L-Met-L-Ala-L-Fos et L-Met-L-Ala-D-Fos (0,13 g, 0,41 mmol, 46 %) ; p.f. 213 - 217 °C (décomp.) ; *v*ₘₐₓ/cm⁻¹ 3263 (NH⁺), 2834 (OH large), 1641 (C=O), 1552 (flexion NH), 1150 (P-O), 1041 (P-O), 919 (P-O) ¹H RMN (300 MHz, D₂O) δ_{H} 1,12-1,29 (3H, m, C**H**₃-2), 1,38 (3H, d, *J*=6,0 Hz, C**H**₃-6), 2,11-2,34 (5H, [s, C**H**₃-12], [m, C**H**₂-10]), 2,62 (2H, m, C**H**₂-11), 3,95-4,11 (2H, [m, C**H**-1], [m, C**H**-9]), 4,32-4,46 (1H, m, C**H**-5) ; ¹³C RMN (75 MHz, D₂O) δ_{C} 14,2 (**C**H₃-12), 15,5 (**C**H₃-2), 16,6 (**C**H₃-6), 28,5 (**C**H₂-11), 29,5 (**C**H₂-10), 49,9 (**C**H-1, **C**H-5 et **C**H-9), 169,4 (**C**=O-4 et **C**=O-8); ³¹P-¹H_{decoup} RMN (121 MHz, CDCl₃) δ_{P} 20,7.

### Exemple 9: Evaluation de l'activité antibactérienne de la L-Norvalinyl-L-alanyl-D/L-fosfaline (composé I en figure 2) et de la L-Méthionyl-L-alanyl-D/L-fosfaline (composé J en figure 2) synthétisées respectivement selon les exemples 7 et 8.

### 9.1. Introduction

Comme pour l'exemple 6, Les concentrations minimales inhibitrices (CMI) des composés I et J vis-à-vis de 12 souches de bactéries à Gram négatif et 6 souches de bactéries à Gram positif ont été déterminées après 22 heures d'incubation en utilisant un procédé de dilution en gélose comme décrit dans l'exemple 2 (cf. notamment section « 2.2 Matériels et méthodes).

Les 18 souches de bactéries testées aux fins du présent exemple sont identiques à celles testées dans l'exemple 6.

### 9.2. Résultats

Les résultats obtenus sont présentés dans le tableau 5 *infra*

**Tableau 5 : Concentrations minimales inhibitrices des composés antimicrobiens I et J vis-à-vis de différentes espèces de bactéries**

| Espèce | Référence de la souche | Concentration minimale inhibitrice (CMI) du composé I (µg/mL) | Concentration minimale inhibitrice (CMI) du composé J (µg/mL) |
|---|---|---|---|
| *Acinetobacter baumannii* | ATCC 19606 | >8 | >8 |
| *Burkholderia cepacia* | ATCC 25416 | >8 | >8 |
| *Enterobacter cloacae* | NCTC 11936 | >8 | >8 |
| *Escherichia coli* | NCTC 10418 | 1 | 2 |
| *Escherichia coli* | NCTC 12241 | 1 | 2 |
| *Klebsiella pneumoniae* | NCTC 9528 | 0,25 | 0,5 |
| *Providencia rettgeri* | NCTC 7475 | >8 | >8 |
| *Pseudomonas aeruginosa* | NCTC 10662 | >8 | >8 |
| *Salmonella Typhimurium* | NCTC 74 | >8 | >8 |
| *Salmonella Enteritidis* | NCTC 6676 | >8 | >8 |
| *Serratia marcescens* | NCTC 10211 | 0,25 | 0,5 |
| *Yersinia enterocolitica* | NCTC 11176 | 0,125 | 0,5 |
| *Enterococcus faecalis* | NCTC 775 | 0,063 | 0,125 |
| *Enterococcus faecium* | NCTC 7171 | 1 | 4 |
| *Listeria monocytogenes* | NCTC 11994 | >8 | >8 |
| *Staphylococci epidermidis* | NCTC 11047 | 1 | 2 |
| *Staphylococcus aureus* | NCTC 6571 | 1 | 1 |
| *Staphylococcus aureus* (SARM) | NCTC 11939 | >8 | >8 |

D'après les données présentées dans le tableau 5 supra, il apparaît clairement que les composés I et J sont fortement inhibiteurs vis-à-vis des souches testées de certaines espèces de bactéries à Gram négatif et de certaines espèces de bactéries à Gram positif, notamment concernant *Escherichia coli, Klebsiella pneumoniae, Serratia marcescens, Yersinia enterocolitica, Enterococcus faecalis,* et moins inhibiteurs par rapport à d'autres espèces de bactéries à Gram négatif et à Gram positif, notamment en ce qui concerne *Acinetobacter baumannii, Burkholderia cepacia, Pseudomonas aeruginosa, Salmonella typhimurium, Salmonella enteritidis, Listeria monocytogenes.*

### Références bibliographiques

[1] Rao J, Lahiri J, Weis RM, Whitesides GM. 2000. Design, synthesis, and characterization of a high-affinity trivalent system derived from vancomycin and L-Lys-D-Ala-D-Ala. J. Am. Chem. Soc. 122:2698-2710.
[2] Kametani T, Suzuki Y, Kigasawa K, Hiiragi M, Wakisaka K, Sugi H, Tanigawa K, Fukawa K, Irino O, Saita O, Yamabe S. 1982. Studies on the synthesis of chemotherapeutics. Part XIII. Synthesis and biological studies on phosphonopeptides having alkyl-, phenyl-, and heterocyclic substituents. Heterocycles 18:295-319.
[3] J. Bacteriol. 1984, 160(1), 122-130 Gibson et al
[4] Cheung et al, Chloroalanyl and Propargylglycyl Diperptides. Suicide Substrate Containing Antibacterials. J. Med. Chem. 1983, 26, 1733-1741
[5] Cheung KS, Boisvert W, Lerner SA, Johnston M. 1986. Chloroalanyl antibiotic peptides: antagonism of their antimicrobial effects by L-alanine and L-alanyl peptides in gram-negative bacteria. J. Med. Chem. 29:2060-2068.
[6] Atherton et al., Synthesis and Structure-Activity Relationships of Antibacterial Phosphonopeptides Incorporating (1-Aminoethyl)phosphonic Acid and (Aminomethyl)phosphonic Acid, Chemistry Department, Roche Products Limited, 1984
[7] Arfin et al., Inhibition of Growth of Salmonella typhimurium and of Threonine Deaminase and Transamine B by ß-Chloroalanine
[8] Orenga et al., 2009 ; J. Microbiol. Methods ; 79(2 :139-55)
[9] Lavielle, S. ; Ling, N. C. ; Saltman, R. ; Guillemn, R. C. Carbohydrate Research, 1981, 89(2), 229
[10] Nakata, T. ; Nakatani, M. ; Takahashi, M. ; Okai, J. ; Kawaoka, Y. ; Kouge, K. ; Okai, H. Bulletin of Chemical Society of Japan, 1996, 69(4), 1099
[11] Thèse de doctorat de Varadi, L., Université de Sunderland, 2012
[12] Kudzin, Z. H. ; Stec, W. J. Synthesis, 1978, 469.
[13] Boduszek, B. ; Soroka, M. Polish Journal of Chemistry, 2002, 76(8), 1105
[14] Kudzin, Z. H. ; Luczak, J. Synthesis, 1995, 1995(5), 509.
[15] Yuan, C. ; Xu, C. ; Zhang, Y. Tetrahedron, 2003, 59(32), 6095
[16] Andrews JM. 2001. Détermination of minimum inhibitory concentrations. J. Antimicrob. Chemother. 48 Suppl 1:5-16.
[17] Atherton FR, Hall MJ, Hassall CH, Lambert RW, Ringrose PS. 1979. Phosphonopeptides as antibacterial agents: rationale, chemistry, and structure-activity relationships. Antimicrob. Agents Chemother. 15:677-683.

## Revendications

1. Composé antimicrobien, ainsi que ses sels, ledit composé antimicrobien étant représenté par la formule générale (I) : 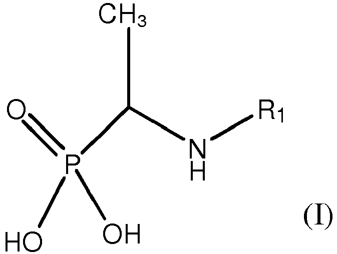 dans laquelle R₁ représente :
- une partie peptidique P₁ consistant en une séquence linéaire d'un à cinq résidus d'acide aminé ; ladite partie peptidique P1 comprenant au moins un résidu de β-chloroalanine, de préférence de β-chloro-L-alanine, ou
- une partie peptidique P2, ladite partie peptidique P2 étant représentée par la formule générale (II) suivante : 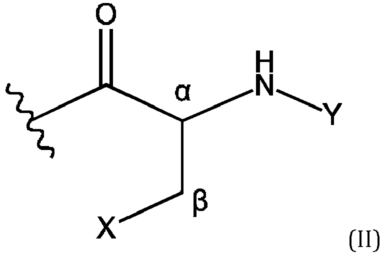
dans laquelle X représente un atome d'hydrogène ou de chlore, et
Y représente un atome d'hydrogène ou une séquence linéaire d'un à quatre résidus d'acide aminé, comprenant avantageusement au moins un résidu de β-chloroalanine, de préférence de β-chloro-L-alanine, et/ou au moins un résidu de norvaline, de préférence de L-norvaline, et/ou au moins un résidu de méthionine, de préférence de L-méthionine, et dans laquelle lorsque X représente un atome d'hydrogène, Y est tel que défini précédemment à l'exception d'un atome d'hydrogène ou d'un résidu d'alanine en position N-terminale ou lié à un autre résidu d'acide aminé par une liaison peptidique entre la fonction aminé en α dudit résidu d'alanine et la fonction acide carboxylique en α de l'autre résidu d'acide aminé.

2. Composé antimicrobien selon la revendication 1, dans lequel R₁ est représenté par la formule générale (III) : 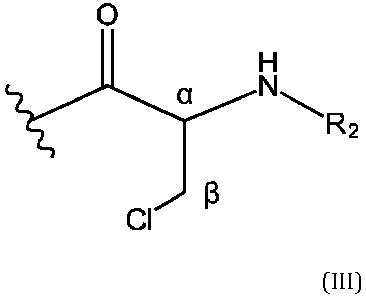 dans laquelle R₂ est un atome d'hydrogène ou une séquence linéaire d'un à quatre résidus d'acide aminé, comprenant avantageusement au moins un résidu de β-chloroalanine, de préférence de β-chloro-L-alanine, et/ou au moins un résidu de norvaline, de préférence de L-norvaline, et/ou au moins un résidu de méthionine, de préférence de L-méthionine.

3. Composé antimicrobien selon la revendication 1 ou 2, dans lequel R₁ est représenté par la formule générale (IV) : 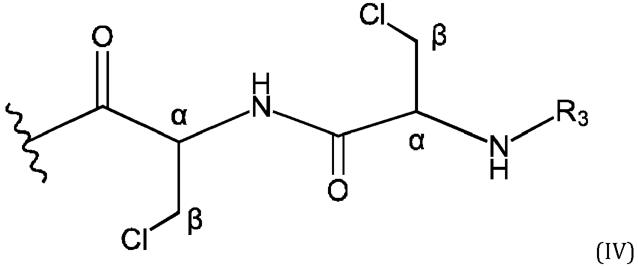 dans laquelle R₃ est un atome d'hydrogène ou une séquence linéaire d'un à trois résidus d'acide aminé, comprenant avantageusement au moins un résidu de β-chloroalanine, de préférence de β-chloro-L-alanine.

4. Composé antimicrobien selon l'une des revendications 1 à 3, dans lequel R₁ consiste en un enchaînement linéaire de deux ou trois, de préférence de deux, résidus d'acide aminé.

5. Composé antimicrobien selon l'une des revendications 1 à 4, dans lequel ledit/lesdits résidu(s) d'acide aminé est/sont sélectionnées) parmi la glycine, la sarcosine et les formes L et D, de préférence L, des résidus de β-chloroalanine, d'alanine, d'arginine, d'asparagine, d'acide aspartique, de cystéine, d'acide glutamique, d'acide gamma-glutamique, de glutamine, d'histidine, d'isoleucine, de leucine, de lysine, de méthionine, de norvaline, de phénylalanine, de proline, d'acide pyroglutamique, de sérine, de thréonine, de tryptophane, de tyrosine et de valine.

6. Composé antimicrobien selon l'une des revendications 1 à 5, dans lequel ledit/lesdits résidu(s) d'acide aminé est/sont sélectionnées) parmi les formes L et D, de préférence L, des résidus de β-chloroalanine, d'alanine, de méthionine, de norvaline et de valine.

7. Composé antimicrobien selon la revendication 1, dans lequel R₁ représente ladite partie peptidique P2 dans laquelle X représente un atome d'hydrogène.

8. Composé antimicrobien selon la revendication 7, dans lequel Y est un résidu d'acide aminé sélectionné parmi la glycine, la sarcosine, les formes L et D, de préférence L, des résidus de β-chloroalanine, d'arginine, d'asparagine, d'acide aspartique, de cystéine, d'acide glutamique, d'acide gamma-glutamique, de glutamine, d'histidine, d'isoleucine, de leucine, de lysine, de méthionine, de norvaline, de phénylalanine, de proline, d'acide pyroglutamique, de sérine, de thréonine, de tryptophane, de tyrosine et de valine ; avantageusement Y est un résidu d'acide aminé sélectionné parmi les formes L et D, de préférence L, des résidus de méthionine et de norvaline.

9. Composé antimicrobien selon la revendication 1, ledit composé antimicrobien consistant en :
- l'acide β-chloro-L-alanyl-D/L-1-aminoéthylphosphonique, de préférence l'acide β-chloro-L-alanyl-L-1-aminoéthylphosphonique, et/ou
- l'acide β-chloro-L-alanyl-β-chloro-L-alanyl-D/L-1-aminoéthylphosphonique, de préférence l'acide β-chloro-L-alanyl-β-chloro-L-alanyl-L-1-aminoéthylphosphonique, et/ou
- l'acide L-norvalinyl-β-chloro-L-alanyl-D/L-1-aminoéthylphosphonique, de préférence l'acide L-norvalinyl-β-chloro-L-alanyl-L-1-aminoéthylphosphonique, et/ou
- l'acide L-méthionyl-β-chloro-L-alanyl-D/L-1-aminoéthylphosphonique, de préférence l'acide L-méthionyl-β-chloro-L-alanyl-L-1-aminoéthylphosphonique, et/ou
- l'acide L-norvalinyl- L-alanyl-D/L-1-aminoéthylphosphonique, de préférence l'acide L-norvalinyl-L-alanyl-L-1-aminoéthylphosphonique, et/ou
- l'acide L-méthionyl-L-alanyl-D/L-1-aminoéthylphosphonique, de préférence l'acide L-méthionyl-L-alanyl-L-1-aminoéthylphosphonique ;
avantageusement ledit composé antimicrobien consistant en l'acide β-chloro-L-alanyl-D/L-1-aminoéthylphosphonique, de préférence l'acide β-chloro-L-alanyl-L-1-aminoéthylphosphonique, et/ou l'acide β-chloro-L-alanyl-β-chloro-L-alanyl-D/L-1-aminoéthylphosphonique, de préférence l'acide β-chloro-L-alanyl-β-chloro-L-alanyl-L-1-aminoéthylphosphonique.

10. Composé antimicrobien selon l'une des revendications 1 à 9, dans lequel la fonction amine N-terminale dudit composé antimicrobien est protégée par un groupement protecteur tel qu'un groupement tertiobutylocarbonyle, 9-fluorenylmethoxycarbonyl ou benzyloxycarbonyl.

11. Milieu réactionnel comprenant au moins un composé antimicrobien selon l'une des revendications 1 à 10, de préférence ledit au moins un composé antimicrobien étant présent dans une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L.

12. Milieu réactionnel selon la revendication 11, ledit milieu réactionnel étant un milieu permettant la détection et/ou l'identification et/ou le dénombrement d'au moins un micro-organisme cible, de préférence d'au moins une bactérie cible, dans un échantillon susceptible de le contenir, tel qu'un échantillon d'origine industrielle ou d'origine clinique, dans lequel ledit au moins un composé antimicrobien est au moins un agent sélectif permettant d'inhiber la survie et/ou la croissance de(s) micro-organisme(s) non-cible(s) de façon à privilégier la survie et/ou la croissance dudit au moins un micro-organisme cible.

13. Milieu réactionnel selon la revendication 12, ledit milieu réactionnel étant un milieu de culture comprenant au moins un nutriment permettant la croissance dudit au moins un micro-organisme cible, dans lequel ledit au moins un agent sélectif permet d'inhiber la croissance de(s) micro-organisme(s) non-cible(s) de façon à privilégier la croissance dudit au moins un micro-organisme cible.

14. Milieu réactionnel selon la revendication 12 ou 13, ledit milieu réactionnel comprenant en outre un substrat enzymatique spécifique d'une activité enzymatique dudit au moins un micro-organisme cible.

15. Milieu réactionnel selon l'une des revendications 12 à 14, dans lequel ledit au moins un agent sélectif consiste en :
- l'acide β-chloro-L-alanyl-L-1-aminoéthylphosphonique, de préférence à une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L, ou
- l'acide β-chloro-L-alanyl-β-chloro-L-alanyl-L-1-aminoéthylphosphonique, de préférence à une concentration finale comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L, ou
- un mélange des deux.

16. Milieu réactionnel selon l'une des revendications 12 à 15, ledit au moins un micro-organisme cible étant :
- au moins un micro-organisme cible à Gram négatif appartenant au genre *Salmonella,* par exemple appartenant à l'espèce *Salmonella enterica,* au sérotype *Salmonella Typhimurium* ou au sérotype *Salmonella Enteridis,* au genre *Acinetobacter,* par exemple à l'espèce *Acinetobacter baumannii,* au genre *Burkholderia,* par exemple à l'espèce *Burkholderia cepacia,* au genre *Pseudomonas,* par exemple à l'espèce *Pseudomonas aeruginosa* ; avantageusement ledit au moins un micro-organisme cible à Gram négatif appartenant au genre *Salmonella ;* ou
- au moins un micro-organisme cible à Gram positif, appartenant au genre *Listeria,* par exemple appartenant à l'espèce *Listeria monocytogenes,* ou au genre *Streptococcus,* par exemple *Streptococcus agalactiae* et/ou *Streptococcus pneumoniae* et/ou *Streptococcus pyogenes* ; avantageusement ledit au moins un micro-organisme cible à Gram positif appartenant au genre *Listeria.*

17. Milieu réactionnel selon la revendication 16, ledit au moins un micro-organisme cible appartenant au genre *Salmonella* et ledit milieu comprenant :
- au moins un agent nutritif, tel que des peptones, par exemple d'origine porcine ou bovine, à une concentration comprise entre 0,2 et 30,0 g/L,
- éventuellement un tampon,
- au moins un marqueur chromogène, tel qu'un substrat enzymatique d'estérase et/ou un substrat enzymatique d'alpha-galactosidase, à une concentration comprise entre 0,05 et 15 ,0 g/L,
- de l'agar à une concentration comprise entre 9,0 et 28,0 g/L, et
- au moins un agent sélectif tel que défini dans l'une des revendications 12 à 16, de préférence à une concentration comprise entre 0,002 et 1024,0 mg/L, préférablement entre 0,003 et 32,0 mg/L, avantageusement entre 0,2 et 8,0 mg/L, de manière préférée entre 0,2 et 2,0 mg/L.

18. Milieu réactionnel selon l'une des revendications 12 à 17, dans lequel le(s) micro-organisme(s) non-cible(s) est/sont compris dans le groupe constitué par :
- des genres de la famille des *Enterobacteriaceae,* tels que le genre *Enterobacter* et/ou le genre *Escherichia* et/ou le genre *Klebsiella* et/ou le genre *Serratia* et/ou le genre *Yersinia,* et/ou
- le genre *Enterococcus,* et/ou
- le genre *Staphylococcus* ;
de préférence le(s) micro-organisme(s) non-cible(s) est/sont résistant(s) à au moins un antibactérien conventionnel.

19. Utilisation *in vitro* d'un milieu réactionnel selon l'une des revendications 11 à 18 pour détecter et/ou identifier et/ou dénombrer au moins un micro-organisme cible, de préférence au moins une bactérie cible, dans un échantillon susceptible de le contenir, tel qu'un échantillon d'origine industrielle ou d'origine clinique.

20. Procédé de détection et/ou d'identification et/ou de dénombrement d'au moins un micro-organisme cible, de préférence d'au moins une bactérie cible, dans un échantillon susceptible de le contenir, tel qu'un échantillon d'origine industrielle ou d'origine clinique, ledit procédé comprenant les étapes suivantes :
a) ensemencer le milieu réactionnel selon l'une des revendications 11 à 18 avec ledit échantillon,
b) si nécessaire incuber le tout durant un laps de temps suffisant pour permettre la détection et/ou l'identification et/ou le dénombrement d'au moins un micro-organisme cible,
c) identifier les colonies formées par ledit au moins un micro-organisme cible.

21. Composé antimicrobien selon l'une des revendications 1 à 10 pour son utilisation en tant que médicament à usage humain ou vétérinaire.

22. Composé antimicrobien selon l'une des revendications 1 à 10 pour son utilisation en tant médicament dans le traitement des infections microbiennes, de préférence des infections bactériennes, humaines ou vétérinaires.

## Patentansprüche

1. Eine antimikrobielle Verbindung sowie ihre Salze, wobei die antimikrobielle Verbindung durch die folgende allgemeine Formel (I) dargestellt wird: 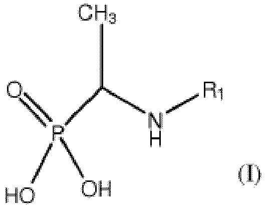 wobei R₁ Folgendes darstellt:
- einen Peptidteil P1, bestehend aus einer linearen Sequenz von einem bis fünf Aminosäureresten; wobei der Peptidteil P1 mindestens einen Rest von β-Chloralanin, vorzugsweise von β-Chlor-L-alanin, beinhaltet, oder
- einen Peptidteil P2, wobei der Peptidteil P2 durch die folgende allgemeine Formel (II) dargestellt wird: 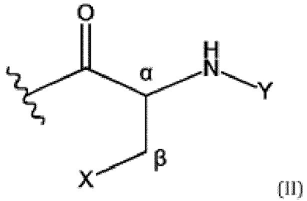
wobei X ein Wasserstoff- oder Chloratom darstellt und
Y ein Wasserstoffatom oder eine lineare Sequenz von einem bis vier Aminosäureresten darstellt, die vorteilhafterweise mindestens einen Rest von β-Chloralanin, vorzugsweise von β-Chlor-L-alanin, und/oder mindestens einen Rest von Norvalin, vorzugsweise von L-Norvalin, und/oder mindestens einen Rest von Methionin, vorzugsweise von L-Methionin, beinhalten, und wobei, wenn X ein Wasserstoffatom darstellt, Y wie oben definiert ist, aber weder ein Wasserstoffatom noch ein Alaninrest in der N-terminalen Position ist noch durch eine Peptidbindung zwischen der α-Aminfunktion des Alaninrests und der α-Carbonsäurefunktion des anderen Aminosäurerests an einen anderen Aminosäurerest gebunden ist.

2. Antimikrobielle Verbindung gemäß Anspruch 1, wobei R₁ durch die folgende allgemeine Formel (III) dargestellt wird: 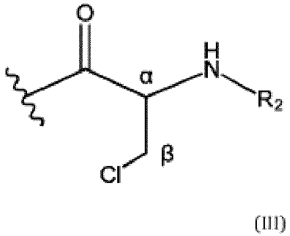 wobei R₂ ein Wasserstoffatom oder eine lineare Sequenz von einem bis vier Aminosäureresten ist, vorteilhafterweise beinhaltend mindestens einen Rest von β-Chloralanin, vorzugsweise von β-Chlor-L-alanin, und/oder mindestens einen Rest von Norvalin, vorzugsweise von L-Norvalin, und/oder mindestens einen Rest von Methionin, vorzugsweise von L-Methionin.

3. Antimikrobielle Verbindung gemäß Anspruch 1 oder 2, wobei R₁ durch die folgende allgemeine Formel (IV) dargestellt wird: 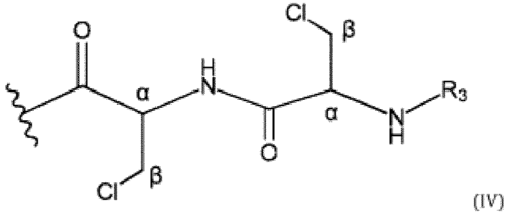 wobei R₃ ein Wasserstoffatom oder eine lineare Sequenz von einem bis drei Aminosäureresten ist, vorteilhafterweise beinhaltend mindestens einen Rest von β-Chloralanin, vorzugsweise von β-Chlor-L-alanin.

4. Antimikrobielle Verbindung gemäß einem der Ansprüche 1 bis 3, wobei R₁ aus einer linearen Verkettung von zwei oder drei, vorzugsweise zwei, Aminosäureresten besteht.

5. Antimikrobielle Verbindung gemäß einem der Ansprüche 1 bis 4, wobei der Aminosäurerest/die Aminosäurereste ausgewählt ist/sind aus Glycin, Sarcosin und den L- und D-Formen, vorzugsweise den L-Formen, von Resten von β-Chloralanin, Alanin, Arginin, Asparagin, Asparaginsäure, Cystein, Glutaminsäure, gamma-Glutaminsäure, Glutamin, Histidin, Isoleucin, Leucin, Lysin, Methionin, Norvalin, Phenylalanin, Prolin, Pyroglutaminsäure, Serin, Threonin, Tryptophan, Tyrosin und Valin.

6. Antimikrobielle Verbindung gemäß einem der Ansprüche 1 bis 5, wobei der Aminosäurerest/die Aminosäurereste ausgewählt ist/sind aus den L- und D-Formen, vorzugsweise den L-Formen, von Resten von β-Chloralanin, Alanin, Methionin, Norvalin und Valin.

7. Antimikrobielle Verbindung gemäß Anspruch 1, wobei R₁ den Peptidteil P2 darstellt, wobei X ein Wasserstoffatom darstellt.

8. Antimikrobielle Verbindung gemäß Anspruch 7, wobei Y ein Aminosäurerest ist, ausgewählt aus Glycin, Sarcosin, den L- und D-Formen, vorzugsweise den L-Formen, von Resten von β-Chloralanin, Arginin, Asparagin, Asparaginsäure, Cystein, Glutaminsäure, gamma-Glutaminsäure, Glutamin, Histidin, Isoleucin, Leucin, Lysin, Methionin, Norvalin, Phenylalanin, Prolin, Pyroglutaminsäure, Serin, Threonin, Tryptophan, Tyrosin und Valin; wobei vorteilhafterweise Y ein Aminosäurerest ist, ausgewählt aus den Formen L und D, vorzugsweise den L-Formen, von Resten von Methionin und Norvalin.

9. Antimikrobielle Verbindung gemäß Anspruch 1, wobei die antimikrobielle Verbindung aus Folgendem besteht:
- β-Chlor-L-alanyl-D/L-1-aminoethylphosphonsäure, vorzugsweise β-Chlor-L-alanyl-L-1-aminoethylphosphonsäure, und/oder
- β-Chlor-L-alanyl- β-chlor-L-alanyl-D/L-1-aminoethylphosphonsäure, vorzugsweise β-Chlor-L-alanyl- β-chlor-L-alanyl-L-1-aminoethylphosphonsäure und/oder
- L-Norvalinyl-β-chlor-L-alanyl-D/L-1-aminoethylphosphonsäure, vorzugsweise L-Norvalinyl-β-chlor-L-alanyl-L-1-aminoethylphosphonsäure, und/oder
- L-Methionyl-β-chlor-L-alanyl-D/L-1-aminoethylphosphonsäure, vorzugsweise L-Methionyl-β-chlor-L-alanyl-L-1-aminoethylphosphonsäure, und/oder
- L-Norvalinyl-L-alanyl-D/L-1-aminoethylphosphonsäure, vorzugsweise L-Norvalinyl-L-alanyl-L-1-aminoethylphosphonsäure, und/oder
- L-Methionyl-L-alanyl-D/L-1-aminoethylphosphonsäure, vorzugsweise L-Methionyl-L-alanyl-L-1-aminoethylphosphonsäure;
wobei die antimikrobielle Verbindung vorteilhafterweise aus β-Chlor-L-alanyl-D/L-1-aminoethylphosphonsäure, vorzugsweise β-Chlor-L-alanyl-L-1-aminoethylphosphonsäure, und/oder β-Chlor-L-alanyl-β-chlor-L-alanyl-D/L-1-aminoethylphosphonsäure, vorzugsweise β-Chlor-L-alanyl-β-chlor-L-alanyl-L-1-aminoethylphosphonsäure, besteht.

10. Antimikrobielle Verbindung gemäß einem der Ansprüche 1 bis 9, wobei die N-terminale Aminfunktion der antimikrobiellen Verbindung durch eine Schutzgruppe, wie beispielsweise eine Tertiobutylocarbonyl-, 9-Fluorenylmethoxycarbonyl- oder Benzyloxycarbonylgruppe, geschützt ist.

11. Ein Reaktionsmedium, das mindestens eine antimikrobielle Verbindung gemäß einem der Ansprüche 1 bis 10 beinhaltet, wobei die mindestens eine antimikrobielle Verbindung vorzugsweise in einer Endkonzentration von zwischen 0,002 und 1024,0 mg/l, vorzugsweise von zwischen 0,003 und 32,0 mg/l, vorteilhafterweise von zwischen 0,2 und 8,0 mg/l, auf bevorzugte Weise von zwischen 0,2 und 2,0 mg/l, vorhanden ist.

12. Reaktionsmedium gemäß Anspruch 11, wobei es sich bei dem Reaktionsmedium um ein Medium handelt, welches Folgendes ermöglicht: das Nachweisen und/oder Identifizieren und/oder Zählen von mindestens einem Zielmikroorganismus, vorzugsweise mindestens einer Zielbakterie, in einer Probe, die ihn halten kann, wie eine Probe industriellen Ursprungs oder klinischen Ursprungs, wobei es sich bei der mindestens einen antimikrobiellen Verbindung um mindestens ein selektives Mittel handelt, welches Folgendes ermöglicht: das Hemmen des Überlebens und/oder des Wachstums des Nichtzielmikroorganismus/der Nichtzielmikroorganismen, um das Überleben und/oder Wachstum des mindestens einen Zielmikroorganismus zu begünstigen.

13. Reaktionsmedium gemäß Anspruch 12, wobei es sich bei dem Reaktionsmedium um ein Kulturmedium handelt, das mindestens einen Nährstoff beinhaltet, um das Wachstum des mindestens einen Zielmikroorganismus zu ermöglichen, wobei das mindestens eine selektive Mittel das Hemmen des Wachstums des Nichtzielmikroorganismus/der Nichtzielmikroorganismen ermöglicht, um das Wachstum des mindestens einen Zielmikroorganismus zu begünstigen.

14. Reaktionsmedium gemäß Anspruch 12 oder 13, wobei das Reaktionsmedium ferner ein Enzymsubstrat beinhaltet, das für eine enzymatische Aktivität des mindestens einen Zielmikroorganismus spezifisch ist.

15. Reaktionsmedium gemäß einem der Ansprüche 12 bis 14, wobei das mindestens eine selektive Mittel aus Folgendem besteht:
- β-Chlor-L-alanyl-L-1-aminoethylphosphonsäure, vorzugsweise in einer Endkonzentration von zwischen 0,002 und 1024,0 mg/l, vorzugsweise von zwischen 0,003 und 32,0 mg/l, vorteilhafterweise von zwischen 0,2 und 8,0 mg/l, auf bevorzugte Weise von zwischen 0,2 und 2,0 mg/l, oder
- β-Chlor-L-alanyl-β-chlor-L-alanyl-L-1-aminoethylphosphonsäure, vorzugsweise in einer Endkonzentration von zwischen 0,002 und 1024,0 mg/l, vorzugsweise von zwischen 0,003 und 32,0 mg/l, vorteilhafterweise von zwischen 0,2 und 8,0 mg/l, auf bevorzugte Weise von zwischen 0,2 und 2,0 mg/l, oder
- einer Mischung aus beidem.

16. Reaktionsmedium gemäß einem der Ansprüche 12 bis 15, wobei es sich bei dem mindestens einen Zielmikroorganismus um Folgendes handelt:
- mindestens einen gramnegativen Zielmikroorganismus, der zur Gattung *Salmonella,* zum Beispiel zur Spezies *Salmonella enterica,* dem Serotyp *Salmonella Typhimurium* oder dem Serotyp *Salmonella Enteridis,* der Gattung *Acinetobacter,* zum Beispiel der Spezies *Acinetobacter baumannii,* der Gattung *Burkholderia,* zum Beispiel der Spezies *Burkholderia cepacia,* der Gattung *Pseudomonas,* zum Beispiel der Spezies *Pseudomonas aeruginosa,* gehört; wobei der mindestens eine gramnegative Zielmikroorganismus vorteilhafterweise zur Gattung *Salmonella* gehört; oder
- mindestens einen grampositiven Zielmikroorganismus, der zur Gattung *Listeria,* zum Beispiel zur Spezies *Listeria monocytogenes,* oder zur Gattung *Streptococcus,* zum Beispiel *Streptococcus agalactiae* und/oder *Streptococcus pneumoniae* und/oder *Streptococcus pyogenes, gehört;* wobei der mindestens eine grampositive Zielmikroorganismus vorteilhafterweise zur Gattung *Listeria* gehört.

17. Reaktionsmedium gemäß Anspruch 16, wobei der mindestens eine Zielmikroorganismus zur Gattung *Salmonella* gehört und das Medium Folgendes beinhaltet:
- mindestens ein Nährmittel, wie etwa Peptone, zum Beispiel von Schweinen oder Rindern, in einer Konzentration von zwischen 0,2 und 30,0 g/l,
- gegebenenfalls einen Puffer,
- mindestens einen chromogenen Marker, wie etwa ein Esterase-Enzymsubstrat und/oder ein alpha-Galactosidase-Enzymsubstrat, in einer Konzentration von zwischen 0,05 und 15,0 g/l,
- Agar in einer Konzentration von zwischen 9,0 und 28,0 g/l und
- mindestens ein selektives Mittel, wie in einem der Ansprüche 12 bis 16 definiert, vorzugsweise in einer Konzentration von zwischen 0,002 und 1024,0 mg/l, vorzugsweise von zwischen 0,003 und 32,0 mg/l, vorteilhafterweise von zwischen 0,2 und 8,0 mg/l, auf bevorzugte Weise von zwischen 0,2 und 2,0 mg/l.

18. Reaktionsmedium gemäß einem der Ansprüche 12 bis 17, wobei der Nichtzielmikroorganismus/die Nichtzielmikroorganismen enthalten sind in der Gruppe, die aus Folgendem zusammengesetzt ist:
- Gattungen der Familie *Enterobacteriaceae,* wie der Gattung *Enterobacter* und/oder der Gattung *Escherichia* und/oder der Gattung *Klebsiella* und/oder der Gattung *Serratia* und/oder der Gattung *Yersinia,* und/oder
- der Gattung *Enterococcus* und/oder
- der Gattung *Staphylococcus;*
wobei der Nichtzielmikroorganismus/die Nichtzielmikroorganismen vorzugsweise gegenüber mindestens einem herkömmlichen antibakteriellen Mittel resistent ist/sind.

19. Eine In-vitro-Verwendung eines Reaktionsmediums gemäß einem der Ansprüche 11 bis 18 zum Nachweisen und/oder Identifizieren und/oder Zählen von mindestens einem Zielmikroorganismus, vorzugsweise mindestens einer Zielbakterie, in einer Probe, die ihn enthalten kann, wie einer Probe industriellen Ursprungs oder klinischen Ursprungs.

20. Ein Verfahren zum Nachweisen und/oder Identifizieren und/oder Zählen von mindestens einem Zielmikroorganismus, vorzugsweise mindestens einer Zielbakterie, in einer Probe, die ihn enthalten kann, wie etwa einer Probe industriellen Ursprungs oder klinischen Ursprungs, wobei das Verfahren die folgenden Schritte beinhaltet:
a) Beimpfen des Reaktionsmediums gemäß einem der Ansprüche 11 bis 18 mit der Probe,
b) falls nötig, Inkubieren des Ganzen über einen Zeitraum, der ausreicht, um das Nachweisen und/oder Identifizieren und/oder Zählen von mindestens einem Zielmikroorganismus zu ermöglichen,
c) Identifizieren der durch den mindestens einen Zielmikroorganismus gebildeten Kolonien.

21. Antimikrobielle Verbindung gemäß einem der Ansprüche 1 bis 10 zur Verwendung als Medikament zur menschlichen oder tiermedizinischen Anwendung.

22. Antimikrobielle Verbindung gemäß einem der Ansprüche 1 bis 10 zur Verwendung als Medikament bei der Behandlung von mikrobiellen Infektionen, vorzugsweise bakteriellen Infektionen, bei Menschen oder Tieren.

## Claims

1. An antimicrobial compound, as well as the salts thereof, said antimicrobial compound being represented by the general formula (I): 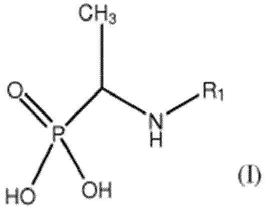 wherein R₁ represents:
- a peptide part P1 consisting of a linear sequence of one to five amino acid residues; said peptide part P1 comprising at least one residue of β-chloroalanine, preferably of β-chloro-L-alanine, or
- a peptide part P2, said peptide part P2 being represented by the following general formula (II): 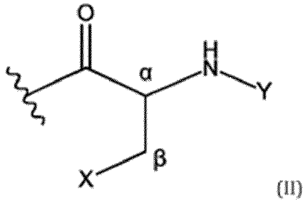
wherein X represents a hydrogen or chlorine atom, and
Y represents a hydrogen atom or a linear sequence of one to four amino acid residues, advantageously comprising at least one residue of β-chloroalanine, preferably of β-chloro-L-alanine, and/or at least one residue of norvaline, preferably of L-norvaline, and/or at least one residue of methionine, preferably of L-methionine, and wherein if X represents a hydrogen atom, Y is as defined previously with the exception of a hydrogen atom or an alanine residue in the N-terminal position or bound to another amino acid residue via a peptide bond between the α amino function of said alanine residue and the α carboxylic acid function of the other amino acid residue.

2. The antimicrobial compound according to claim 1, wherein R₁ is represented by the general formula (III): 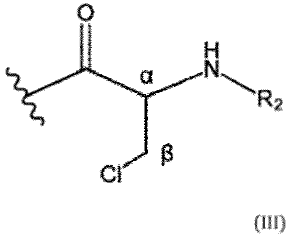 wherein R₂ is a hydrogen atom or a linear sequence of one to four amino acid residues, advantageously comprising at least one residue of β-chloroalanine, preferably of β-chloro-L-alanine, and/or at least one residue of norvaline, preferably of L-norvaline, and/or at least one residue of methionine, preferably of L-methionine.

3. The antimicrobial compound according to claim 1 or 2, wherein R₁ is represented by the general formula (IV): 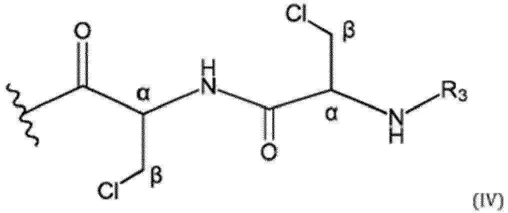 wherein R₃ is a hydrogen atom or a linear sequence of one to three amino acid residues, advantageously comprising at least one residue of β-chloroalanine, preferably of β-chloro-L-alanine.

4. The antimicrobial compound according to one of claims 1 to 3, wherein R₁ consists of a linear chain of two or three, preferably two, amino acid residues.

5. The antimicrobial compound according to one of claims 1 to 4, wherein said amino acid residue(s) is/are selected from glycine, sarcosine and the L and D forms, preferably L, of the residues of β-chloroalanine, alanine, arginine, asparagine, aspartic acid, cysteine, glutamic acid, gamma-glutamic acid, glutamine, histidine, isoleucine, leucine, lysine, methionine, norvaline, phenylalanine, proline, pyroglutamic acid, serine, threonine, tryptophan, tyrosine and valine.

6. The antimicrobial compound according to one of claims 1 to 5, wherein said amino acid residue(s) is/are selected from the L and D forms, preferably L, of the residues of β-chloroalanine, alanine, methionine, norvaline and valine.

7. The antimicrobial compound according to claim 1, wherein R₁ represents said peptide part P2 wherein X represents a hydrogen atom.

8. The antimicrobial compound according to claim 7, wherein Y is an amino acid residue selected from glycine, sarcosine, the L and D forms, preferably L, of the residues of β-chloroalanine, arginine, asparagine, aspartic acid, cysteine, glutamic acid, gamma-glutamic acid, glutamine, histidine, isoleucine, leucine, lysine, methionine, norvaline, phenylalanine, proline, pyroglutamic acid, serine, threonine, tryptophan, tyrosine and valine; advantageously Y is an amino acid residue selected from the L and D forms, preferably L, of the residues of methionine and norvaline.

9. The antimicrobial compound according to claim 1, said antimicrobial compound consisting of:
- β-chloro-L-alanyl-D/L-1-aminoethylphosphonic acid, preferably β-chloro-L-alanyl-L-1-aminoethylphosphonic acid, and/or
- β-chloro-L-alanyl-β-chloro-L-alanyl-D/L-1-aminoethylphosphonic acid, preferably β-chloro-L-alanyl-β-chloro-L-alanyl-L-1-aminoethylphosphonic acid, and/or
- L-norvalinyl-β-chloro-L-alanyl-D/L-1-aminoethylphosphonic acid, preferably L-norvalinyl-β-chloro-L-alanyl-L-1-aminoethylphosphonic acid, and/or
- L-methionyl-β-chloro-L-alanyl-D/L-1-aminoethylphosphonic acid, preferably L-methionyl-β-chloro-L-alanyl-L-1-aminoethylphosphonic acid, and/or
- L-norvalinyl-L-alanyl-D/L-1-aminoethylphosphonic acid, preferably L-norvalinyl-L-alanyl-L-1-aminoethylphosphonic acid, and/or
- L-methionyl-L-alanyl-D/L-1-aminoethylphosphonic acid, preferably L-methionyl-L-alanyl-L-1-aminoethylphosphonic acid;
advantageously said antimicrobial compound consisting of β-chloro-L-alanyl-D/L-1-aminoethylphosphonic acid, preferably β-chloro-L-alanyl-L-1-aminoethylphosphonic acid, and/or β-chloro-L-alanyl-β-chloro-L-alanyl-D/L-1-aminoethylphosphonic acid, preferably β-chloro-L-alanyl-β-chloro-L-alanyl-L-1-aminoethylphosphonic acid.

10. The antimicrobial compound according to one of claims 1 to 9, wherein the N-terminal amino function of said antimicrobial compound is protected by a protecting group such as a tertiobutylocarbonyl, 9-fluorenylmethoxycarbonyl or benzyloxycarbonyl group.

11. A reaction medium comprising at least one antimicrobial compound according to one of claims 1 to 10, preferably said at least one antimicrobial compound being present in a final concentration of between 0.002 and 1024.0 mg/L, preferably between 0.003 and 32.0 mg/L, advantageously between 0.2 and 8.0 mg/L, in a preferred manner between 0.2 and 2.0 mg/L.

12. The reaction medium according to claim 11, said reaction medium being a medium making it possible to detect and/or identify and/or enumerate at least one target microorganism, preferably at least one target bacterium, in a sample capable of containing it, such as a sample of industrial origin or of clinical origin, wherein said at least one antimicrobial compound is at least one selective agent making it possible to inhibit the survival and/or growth of non-target microorganism(s) so as to favour the survival and/or growth of said at least one target microorganism.

13. The reaction medium according to claim 12, said reaction medium being a culture medium comprising at least one nutrient enabling the growth of said at least one target microorganism, wherein said at least one selective agent makes it possible to inhibit the growth of non-target microorganism(s) so as to favour the growth of said at least one target microorganism.

14. The reaction medium according to claim 12 or 13, said reaction medium further comprising an enzyme substrate specific to an enzymatic activity of said at least one target microorganism.

15. The reaction medium according to one of claims 12 to 14, wherein said at least one selective agent consists of:
- β-chloro-L-alanyl-L-1-aminoethylphosphonic acid, preferably at a final concentration of between 0.002 and 1024.0 mg/L, preferably between 0.003 and 32.0 mg/L, advantageously between 0.2 and 8.0 mg/L, in a preferred manner between 0.2 and 2.0 mg/L, or
- β-chloro-L-alanyl-β-chloro-L-alanyl-L-1-aminoethylphosphonic acid, preferably at a final concentration of between 0.002 and 1024.0 mg/L, preferably between 0.003 and 32.0 mg/L, advantageously between 0.2 and 8.0 mg/L, in a preferred manner between 0.2 and 2.0 mg/L, or
- a mixture of the two.

16. The reaction medium according to one of claims 12 to 15, said at least one target microorganism being:
- at least one Gram-negative target microorganism belonging to the genus *Salmonella,* for example belonging to the species *Salmonella enterica,* to the serotype *Salmonella Typhimurium* or to the serotype *Salmonella Enteridis,* to the genus *Acinetobacter,* for example to the species *Acinetobacter baumannii, to the genus Burkholderia,* for example to the species *Burkholderia cepacia,* to the genus *Pseudomonas,* for example to the species *Pseudomonas aeruginosa;* advantageously said at least one Gram-negative target microorganism belonging to the genus *Salmonella; or*
- at least one Gram-positive target microorganism belonging to the genus *Listeria,* for example belonging to the species *Listeria monocytogenes*, or to the genus *Streptococcus,* for example *Streptococcus agalactiae* and/or *Streptococcus pneumoniae* and/or *Streptococcus pyogenes;* advantageously said at least one Gram-positive target microorganism belonging to the genus *Listeria.*

17. The reaction medium according to claim 16, said at least one target microorganism belonging to the genus *Salmonella* and said medium comprising:
- at least one nutrient agent, such as peptones, for example of porcine or bovine origin, at a concentration of between 0.2 and 30.0 g/L,
- possibly a buffer,
- at least one chromogenic marker, such as an esterase enzyme substrate and/or an alpha-galactosidase enzyme substrate, at a concentration of between 0.05 and 15.0 g/L,
- agar at a concentration of between 9.0 and 28.0 g/L, and
- at least one selective agent as defined in one of claims 12 to 16, preferably at a concentration of between 0.002 and 1024.0 mg/L, preferably between 0.003 and 32.0 mg/L, advantageously between 0.2 and 8.0 mg/L, in a preferred manner between 0.2 and 2.0 mg/L.

18. The reaction medium according to one of claims 12 to 17, wherein the non-target microorganism(s) is/are within the group constituted by:
- genera of the *Enterobacteriaceae* family, such as the genus *Enterobacter* and/or the *genus Escherichia* and/or the genus *Klebsiella* and/or the genus *Serratia* and/or the genus *Yersinia,* and/or
- the genus *Enterococcus,* and/or
- the genus *Staphylococcus*;
preferably the non-target microorganism(s) is/are resistant to at least one conventional antibacterial.

19. *In vitro* use of a reaction medium according to one of claims 11 to 18 to detect and/or identify and/or enumerate at least one target microorganism, preferably at least one target bacterium, in a sample capable of containing it, such as a sample of industrial origin or of clinical origin.

20. A method of detecting and/or identifying and/or enumerating at least one target microorganism, preferably at least one target bacterium, in a sample capable of containing it, such as a sample of industrial origin or of clinical origin, said method comprising the following steps:
a) seeding the reaction medium according to one of claims 11 to 18 with said sample,
b) if necessary incubating the assembly for a sufficient period of time to enable detection and/or identification and/or enumeration of at least one target microorganism,
c) identifying the colonies formed by said at least one target microorganism.

21. The antimicrobial compound according to one of claims 1 to 10 for its use as a medicine for human or veterinary use.

22. The antimicrobial compound according to one of claims 1 to 10 for its use as a medicine in the treatment of microbial infections, preferably bacterial infections, in humans or animals.
